# EUROPEAN PATENT SPECIFICATION

(11) **EP 1 381 268 B1**
(45) Date of publication and mention of the grant of the patent: **30.11.2011**
(21) Application number: 02736510.5
(22) Date of filing: 26.03.2002
(51) Int. Cl.: A01H 5/00, C12N 5/04, C07K 14/415, C12N 15/82, C12N 15/29

(54) **METHOD FOR MODIFYING PLANT BIOMASS**
VERFAHREN ZUR MODIFIKATION VON PFLANZLICHER BIOMASSE
PROCEDE DE MODIFICATION DE LA PHYTOMASSE

(30) Priority: 30.03.2001 US 823676
(43) Date of publication of application: 21.01.2004
(73) Proprietor: Mendel Biotechnology, Inc., Hayward, CA 94545 (US)
(72) Inventor: JIANG, Cai-Zhong, Fremont, CA 94555 (US)
(74) Representative: Brasnett, Adrian Hugh
(86) International application number: PCT/US2002/009139
(87) International publication number: WO 2002/079403

(56) References cited:
- EP-A2- 1 033 405
- WO-A-01/36444
- WO-A-02/15675
- DATABASE EMBL [Online] 22 April 1998 (1998-04-22), "Arabidopsis thaliana DNA chromosome 4, BAC clone F23E12 (ESSA project)" XP002304783 retrieved from EBI accession no. EM_PRO:ATF23E12 Database accession no. AL022604
- PATENT ABSTRACTS OF JAPAN vol. 2000, no. 05, 14 September 2000 (2000-09-14) -& JP 2000 041685 A (OJI PAPER CO LTD; KAZUSA DNA KENKYUSHO), 15 February 2000 (2000-02-15)
- ARAVIND L ET AL: "AT-hook motifs identified in a wide variety of DNA-binding proteins" NUCLEIC ACIDS RESEARCH, vol. 26, no. 19, 1 October 1998 (1998-10-01), pages 4413-4421, XP002304781 ISSN: 0305-1048
- MEIJER ANNEMARIE H ET AL: "Novel members of a family of AT hook-containing DNA-binding proteins from rice are identified through their in vitro interaction with consensus target sites of plant and animal homeodomain proteins" PLANT MOLECULAR BIOLOGY, vol. 31, no. 3, 1996, pages 607-618, XP009039546 ISSN: 0167-4412
- GRASSER KLAUS D ET AL: "Chromatin-associated HMG1, HMGI/Y and SSRP1 proteins of higher plants" PHYSIOLOGIA PLANTARUM, vol. 110, no. 4, December 2000 (2000-12), pages 427-435, XP002304782 ISSN: 0031-9317
- DATABASE PIR [Online] April 1990 BEVAN ET AL., XP002962839 Database accession no. (T06118)
- DATABASE GENBANK [Online] XP002962840 Database accession no. (AAG29345) & EP 1 033 405 A2 17 October 2000
- DATABASE GENBANK [Online] XP002962841 Database accession no. (AAC48248) & EP 1 033 405 A2 18 October 2000

## Description

This application claims priority to U.S. Patent Application Serial No. 09/823,676, filed March 30, 2001.

### FIELD OF THE INVENTION

This invention relates to the field of plant biology. More particularly, the present invention pertains to compositions and methods for phenotypically modifying a plant.

### BACKGROUND OF THE INVENTION

Increasing the biomass of a plant has several commercial applications. For example, increasing plant leaf biomass may increase the yield of leafy vegetables for human or animal consumption. Additionally, increasing leaf biomass can be used to increase production of plant-derived pharmaceutical or industrial products. By increasing plant biomass, increased production levels of the products may be obtained from the plants. Tobacco leaves, in particular, have been employed as plant factories to generate such products. Furthermore, it may be desirable to increase crop yields of plants by increasing total plant photosynthesis. An increase in total plant photosynthesis is typically achieved by increasing leaf area of the plant. Additional photosynthetic capacity may be used to increase the yield derived from particular plant tissue, including the leaves, roots, fruits or seed. In addition, the ability to modify the biomass of the leaves may be useful for permitting the growth of a plant under decreased light intensity or under high light intensity. Modification of the biomass of another tissue, such as roots, may be useful to improve a plant's ability to grow under harsh environmental conditions, including drought or nutrient deprivation, because the roots may grow deeper into the ground.

Thus, the present invention provides a method for modifying the plant biomass by modifying the size or number of leaves or seed of a plant.

Database sequence accession number AL022604 is from *Arabidopsis thaliana* chromosome 4. EP-A2-1-33405 discloses sequences that have the database accession numbers AAG29345, AAG29344, AAG10650, AAG10649 and AAG10648. Proteins comprising AT hook domains are referred to in Aravind et al, Nucleic Acids Research, 1998, vol 26; 4413-4421; in Meijer et al, Plant Mol. Biol., 1996, vol 31; 607-618; and in Grasser et al, Pyhsiologia Pantarum, 2000, vol 110; 427-435.

### SUMMARY OF THE INVENTION

The present invention thus provides a method for producing a transgenic plant having increased seed yield compared to a wild-type plant of the same species, the method steps comprising: (a) introducing into a plant an expression vector comprising (i) a sequence having at least 85% amino acid identity to the polypeptide of SEQ ID NO:2 or (ii) a recombinant polynucleotide that encodes an AT-hook family polypeptide comprising a conserved domain that is at least 65% identical to amino acid residues 33 through 50 of SEQ ID NO: 2; and (b) selecting a transgenic plant having increased seed yield so produced by comparing said transgenic plant with a wild-type plant of the same species.

The invention also provides a method for increasing seed yield of a plant relative to a wild-type plant of the same species, comprising introducing into a plant an expression vector comprising a recombinant polynucleotide that encodes (i) a sequence having at least 85% amino acid identity to the polypeptide of SEQ ID NO:2 or (ii) an AT-hook family polypeptide comprising a conserved domain that is at least 65% identical to amino acid residues 33 through 50 of SEQ ID NO: 2.

The invention also provides a method to produce a transgenic seed, the method comprising:
(a) introducing into a plant an expression vector comprising (i) a sequence having at least 85% amino acid identity to the polypeptide of SEQ ID NO:2 or (ii) a recombinant polynucleotide that encodes an AT-hook family polypeptide comprising a conserved domain that is at least 65% identical to amino acid residues 33 through 50 of SEQ ID NO: 2; (b) selecting a transgenic plant having increased seed yield so produced by comparing said transgenic plant with a wild-type plant of the same species; and (c) growing the transgenic plant to produce a transgenic seed, wherein the transgenic seed comprises the recombinant polynucleotide.

A progeny plant grown from the transgenic seed may have greater expression levels of the polypeptide than the wild-type plant.

In the methods of the invention, said AT hook family polypeptide may in one embodiment comprise a conserved domain that is at least 80% identical to amino acid residues 33 through 50 of SEQ ID NO: 2. In a further embodiment of the methods, said AT-hook family polypeptide is SEQ ID NO: 2.

In one aspect of the invention, said seed yield is at least 50% greater than the seed yield of the wild-type plant.

Transgenic plants which may be subject of the methods of the invention include those selected from the group consisting of *Cruciferae* including broccoli, rapeseed and cabbage; wheat; alfalfa; soybean; rice; corn; and *Arabidopsis.*

The recombinant polynucleotide may further comprise a constitutive, inducible, or tissue-specific promoter operably linked to the nucleotide sequence. Also described herein are compositions comprising at least two of the above described polynucleotides.

Also described herein is a transgenic plant comprising one or more of the above described recombinant polynucleotides.

Also described herein is a cloning or expression vector comprising the isolated or recombinant polynucleotide described above or cells comprising the cloning or expression vector.

In yet a further aspect, described herein is a composition produced by incubating a polynucleotide of the invention with a nuclease, a restriction enzyme, a polymerase; a polymerase and a primer; a cloning vector, or with a cell.

Also described herein is a method of identifying a factor that is modulated by or interacts with a polypeptide encoded by a polynucleotide. The method comprises expressing a polypeptide encoded by the polynucleotide in a plant and identifying at least one factor that is modulated by or interacts with the polypeptide. In one embodiment the method for identifying modulating or interacting factors is by detecting binding by the polypeptide to a promoter sequence, or by detecting interactions between an additional protein and the polypeptide in a yeast two hybrid system, or by detecting expression of a factor by hybridization to a microarray, subtractive hybridization or differential display.

Also described herein is a method of identifying a molecule that modulates activity or expression of a polynucleotide or polypeptide of interest. The method comprises placing the molecule in contact with a plant comprising the polynucleotide or polypeptide encoded by the polynucleotide and monitoring one or more of the expression level of the polynucleotide in the plant, the expression level of the polypeptide in the plant, and modulation of an activity of the polypeptide in the plant.

Also described herein is an integrated system, computer or computer readable medium comprising one or more character strings corresponding to a polynucleotide, or to a polypeptide encoded by the polynucleotide. The integrated system, computer or computer readable medium may comprise a link between one or more sequence strings to a modified biomass phenotype.

Also described herein is a method for identifying a sequence similar or homologous to one or more polynucleotides of the invention, or one or more polypeptides encoded by the polynucleotides. The method comprises providing a sequence database and querying the sequence database with one or more target sequences corresponding to the one or more polynucleotides or to the one or more polypeptides to identify one or more sequence members of the database that display sequence similarity or homology to one or more of the target sequences.

### BRIEF DESCRIPTION OF THE SEQUENCE LISTING AND DRAWINGS

The Sequence Listing provides exemplary polynucleotide and polypeptide sequences to which the invention relates. These sequences may be employed to modify the biomass of a plant.

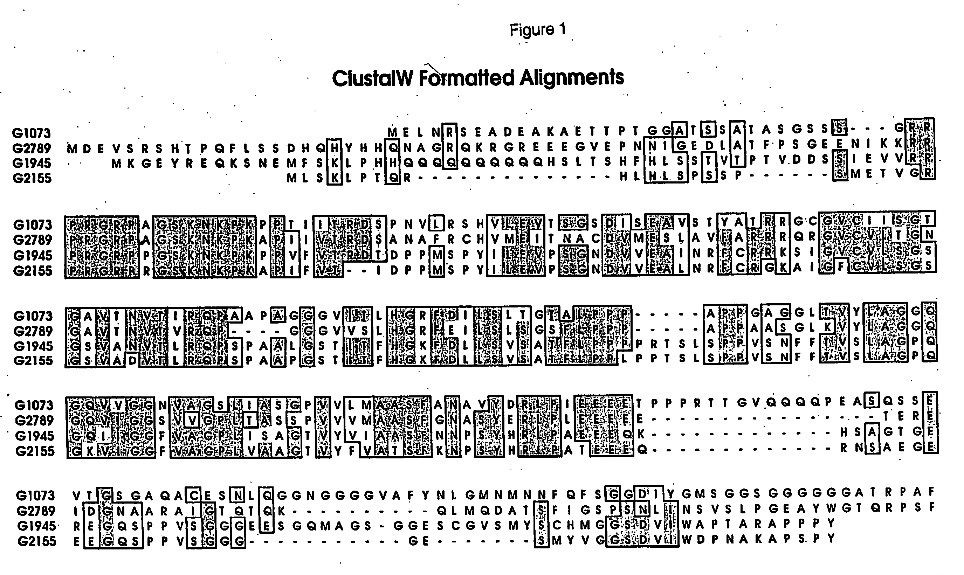

Figure 1 shows the polypeptide alignments for G1073 (SEQ ID Nos. 1 and 2), G2789 (SEQ ID Nos. 3 and 4), G1945 (SEQ ID Nos. 5 and 6) and G2155 (SEQ ID Nos. 7 and 8) showing regions of the polypeptides with sequence identity.

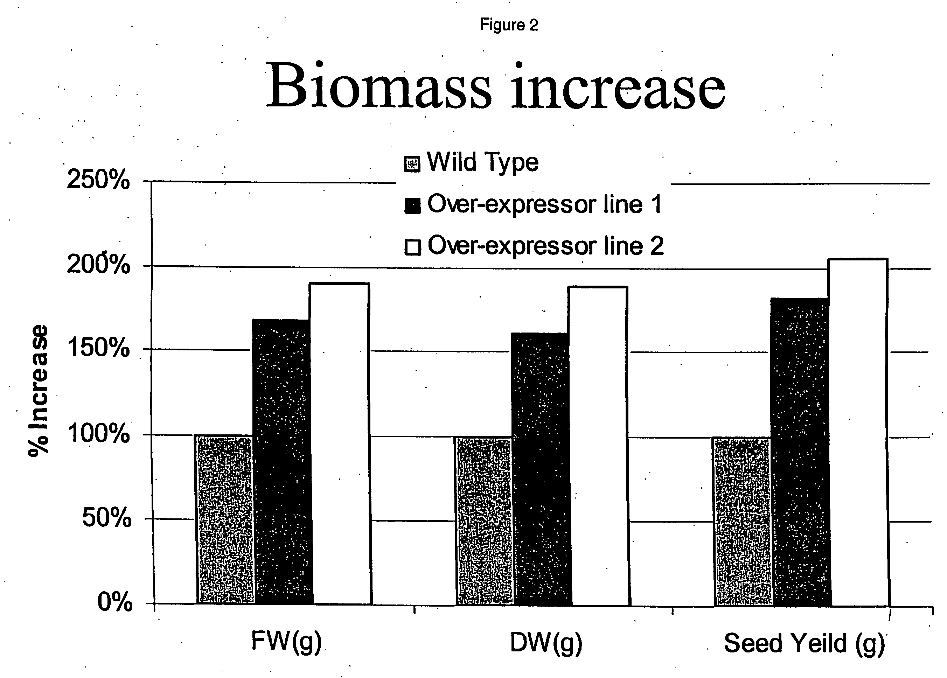

Figure 2 shows that plants overexpressing G1073 have an increased fresh weight, dry weight and seed yield (greater than 150%) when compared with plants that do not overexpress G1073.

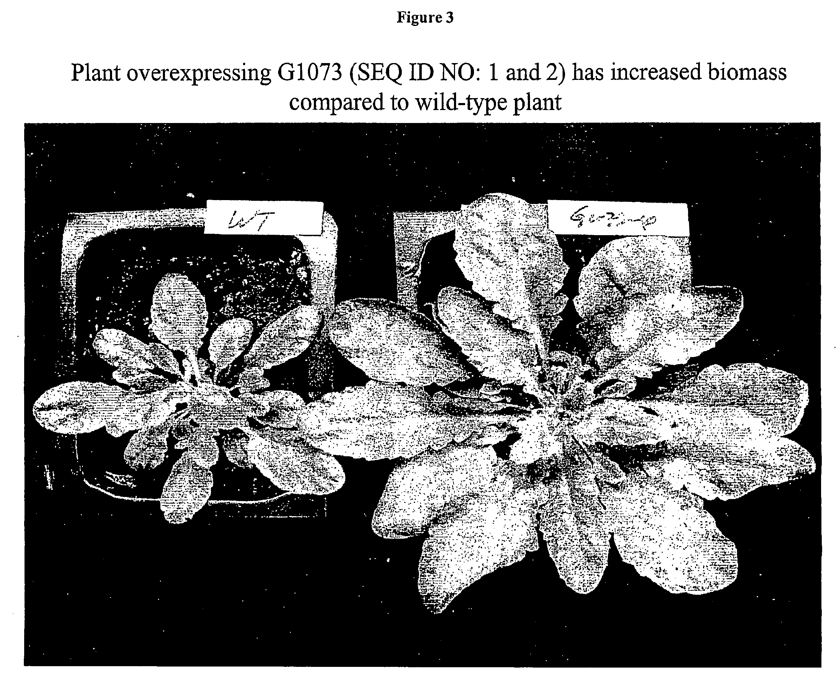

Figure 3 contains photographs of a plant overexpressing G1073 and a wild-type plant (wt) and demonstrates that plants overexpressing G1073 have an increased biomass when compared with control or reference plants.

### DETAILED DESCRIPTION

Each of the references, documents or information sources listed herein can be relied on to make or use the invention.

The present invention relates to polynucleotides and polypeptides, e.g. for modifying phenotypes of plants. In particular, the polynucleotides or polypeptides are useful for modifying plant biomass when the expression levels of the polynucleotides or expression levels or activity levels of the polypeptides are altered compared with those found in a wild type plant. Plant biomass can be either decreased, increased or made inducible under specific conditions using the polynucleotides or polypeptides of this invention.

The polynucleotides encode plant transcription factors. The plant transcription factors are derived, e.g., from *Arabidopsis thaliana* and can belong, e.g., to one or more of the following transcription factor families: the AP2 (APETALA2) domain transcription factor family (Riechmann and Meyerowitz (1998) Biol. Chem. 379:633-646); the MYB transcription factor family (Martin and Paz-Ares (1997) Trends Genet. 13:67-73); the MADS domain transcription factor family (Riechmann and Meyerowitz (1997) Biol. Chem. 378:1079-1101); the WRKY protein family (Ishiguro and Nakamura (1994) Mol. Gen. Genet. 244:563-571); the ankyrin-repeat protein family (Zhang et al. (1992) Plant Cell 4:1575-1588); the miscellaneous protein (MISC) family (Kim et al. (1997) Plant J. 11:1237-1251); the zinc finger protein (Z) family (Klug and Schwabe (1995) FASEB J. 9: 597-604); the homeobox (HB) protein family (Buerglin in Duboule (1994) Guidebook to the Homeobox Genes. Oxford University Press); the CAAT-element binding proteins (Forsburg and Guarente (1989) Genes Dev. 3:1166-1178); the squamosa promoter binding proteins (SPB) (Klein et al. (1996) Mol. Gen. Genet. 1996 250:7-16); the NAM protein family; the IAA/AUX proteins (Rouse et al. (1998) Science 279:1371-1373); the HLH/MYC protein family (Littlewood et al. (1994) Prot. Profile 1:639-709); the DNA-binding protein (DBP) family (Tucker et al. (1994) EMBO J. 13:2994-3002); the bZIP family of transcription factors (Foster et al. (1994) FASEB J. 8:192-200); the BPF-1 protein (Box P-binding factor) family (da Costa e Silva et al. (1993) Plant J. 4:125-135); the golden protein (GLD) family (Hall et al. (1998) Plant Cell 10:925-936); and the AT-hook protein (AT-Hook) family (Aravind et al. (1998) Nucl. Acid Res. 26: 4413-4421). Exemplary transcription factors are listed in the Sequence Listing.

In addition to methods for modifying a plant phenotype by employing one or more polynucleotides and polypeptides described herein, the polynucleotides and polypeptides have a variety of additional uses. These uses include their use in the recombinant production (i.e, expression) of proteins, as regulators of plant gene expression, as diagnostic probes for the presence of complementary or partially complementary nucleic acids (including for detection of natural coding nucleic acids), as substrates for further reactions, e.g., mutation reactions, PCR reactions, or the like, or as substrates for cloning e.g., including digestion or ligation reactions, and for identifying exogenous or endogenous modulators of the transcription factors.

### DEFINITIONS

A "polynucleotide" is a nucleic acid sequence comprising a plurality of polymerized nucleotide residues, e.g., at least about 15 consecutive polymerized nucleotide residues, optionally at least about 30 consecutive nucleotides, or at least about 50 consecutive nucleotides. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, a purification tag, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "recombinant polypeptide" is a polypeptide produced by translation of a recombinant polynucleotide. An "isolated polypeptide," whether a naturally occurring or a recombinant polypeptide, is more enriched in (or out of) a cell than the polypeptide in its natural state in a wild type cell, e.g., more than about 5% enriched, more than about 10% enriched, or more than about 20%, or more than about 50%, or more, enriched, i.e., alternatively denoted: 105%, 110%, 120%, 150%, 300% or more, enriched relative to wild type standardized at 100%. Such an enrichment is not the result of a natural response of a wild type plant. Alternatively, or additionally, the isolated polypeptide is separated from other cellular components with which it is typically associated, e.g., by any of the various protein purification methods herein.

The term "transgenic plant" refers to a plant that contains genetic material, not found in a wild type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

The phrase "ectopically expression or altered expression" in reference to a polynucleotide indicates that the pattern of expression in, e.g., a transgenic plant or plant tissue, is different from the expression pattern in a wild type plant or a reference plant of the same species. For example, the polynucleotide or polypeptide is expressed in a cell or tissue type other than a cell or tissue type in which the sequence is expressed in the wild type plant, or by expression at a time other than at the time the sequence is expressed in the wild type plant, or by a response to different inducible agents, such as hormones or environmental signals, or at different expression levels (either higher or lower) compared with those found in a wild type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" may further relate to altered activity levels resulting from the interactions of the polypeptides with exogenous or endogenous modulators or from interactions with factors or as a result of the chemical modification of the polypeptides.

The term "fragment" or "domain," with respect to a polypeptide, refers to a subsequence of the polypeptide. In some cases, the fragment or domain is a subsequence of the polypeptide, which performs at least one biological function of the intact polypeptide in substantially the same manner, or to a similar extent, as does the intact polypeptide. For example, a polypeptide fragment can comprise a recognizable structural motif or functional domain such as a DNA binding domain that binds to a DNA promoter region, an activation domain or a domain for protein-protein interactions. Fragments can vary in size from as few as 5, 6 or 8 amino acids to the full length of the intact polypeptide, but are preferably at least about 30 amino acids in length and more preferably at least about 60 amino acids in length. In reference to a nucleotide sequence, "a fragment" refers to any subsequence of a polynucleotide, typically, of at least consecutive about 15 nucleotides, encoding 5, 6, 8 or 10 amino acids for example, preferably at least about 30 nucleotides, more preferably at least about 50, of any of the sequences provided herein. A fragment can consist of or comprise nucleotides encoding amino acids outside of a conserved domain known to exist in a particular transcription factor belonging to a transcription factor family, for example.

The term "trait" refers to a physiological, morphological, biochemical or physical characteristic of a plant or particular plant material or cell. In some instances, this characteristic is visible to the human eye, such as seed or plant size, or can be measured by available biochemical techniques, such as the protein, starch or oil content of seed or leaves or by the observation of the expression level of genes, e.g., by employing Northern analysis, RT-PCR, microarray gene expression assays or reporter gene expression systems, or by agricultural observations such as stress tolerance, yield or pathogen tolerance.

"Trait modification" refers to a detectable difference in a characteristic in a plant ectopically expressing a polynucleotide or polypeptide of the present invention relative to a plant not doing so, such as a wild type plant. In some cases, the trait modification can be evaluated quantitatively. For example, the trait modification can entail at least about a 2% increase or decrease in an observed trait (difference), at least about a 5% difference, at least about a 10% difference, at least about a 20% difference, at least about a 30%, at least about a 50%, at least about a 70%, or at least about a 100%, at least a 300% or an even greater difference. It is known that there can be a natural variation in the modified trait. Therefore, the trait modification observed entails a change of the normal distribution of the trait in the plants compared with the distribution observed in wild type plant.

### POLYPEPTIDES AND POLYNUCLEOTIDES OF THE INVENTION

The present invention provides, among other things, transcription factors (TFs), and transcription factor homologue polypeptides, and isolated or recombinant polynucleotides encoding the polypeptides. These polypeptides and polynucleotides may be employed to modify a plant's biomass.

Exemplary polynucleotides encoding the polypeptides of the invention were identified in the *Arabidopsis thaliana* GenBank database using publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides of the invention were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full length cDNA was then recovered by a routine end-to-end polymerase chain reaction (PCR) using primers specific to the isolated 5' and 3' ends. Exemplary sequences are provided in the Sequence Listing.

The polynucleotides of the invention were ectopically expressed in overexpressor or knockout plants and changes in plant biomass were observed. Therefore, the polynucleotides and polypeptides can be employed to improve (increase or decrease) plant biomass.

### Making polynucleotides

The polynucleotides of the invention include sequences that encode transcription factors and transcription factor homologue polypeptides and sequences complementary thereto, as well as unique fragments of coding sequence, or sequence complementary thereto. Such polynucleotides can be, e.g., DNA or RNA, mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homologue polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homologue polypeptide is an endogenous or exogenous gene.

A variety of methods exist for producing the polynucleotides of the invention. Procedures for identifying and isolating DNA clones are well known to those of skill in the art, and are described in, e.g., Berger and Kimmel, Guide to Molecular Cloning Techniques, Methods in Enzymology volume 152 Academic Press, Inc., San Diego, CA ("Berger"); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc., (supplemented through 2000) ("Ausubel").

Alternatively, polynucleotides of the invention can be produced by a variety of in vitro amplification methods adapted to the present invention by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids of the invention are found in Berger, Sambrook, and Ausubel, as well as Mullis et al., (1987) PCR Protocols A Guide to Methods and Applications (Innis et al. eds) Academic Press Inc. San Diego, CA (1990) (Innis). Improved methods for cloning in vitro amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. See, e.g., Ausubel, Sambrook and Berger, all *supra.*

Alternatively, polynucleotides and oligonucleotides can be assembled from fragments produced by solid-phase synthesis methods. Typically, fragments of up to approximately 100 bases are individually synthesized and then enzymatically or chemically ligated to produce a desired sequence, e.g., a polynucleotide encoding all or part of a transcription factor. For example, chemical synthesis using the phosphoramidite method is described, e.g., by Beaucage et al. (1981) Tetrahedron Letters 22:1859-69; and Matthes et al. (1984) EMBO J. 3:801-5. According to such methods, oligonucleotides are synthesized, purified, annealed to their complementary strand, ligated and then optionally cloned into suitable vectors. And if so desired, the polynucleotides and polypeptides of the invention can be custom ordered from any of a number of commercial suppliers.

### HOMOLOGOUS SEQUENCES

Sequences homologous, i.e., that share significant sequence identity or similarity, to those provided in the Sequence Listing, derived from *Arabidopsis thaliana* or from other plants of choice are also an aspect of the invention. Homologous sequences can be derived from any plant including monocots and dicots and in particular agriculturally important plant species including, but not limited to, crops such as soybean, wheat, com, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype can be changed include barley, rye, millet, sorghum, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, and sweet potato, and beans. The homologous sequences may also be derived from woody species, such pine, poplar and eucalyptus.

Transcription factors that are homologous to the listed sequences will typically share at least about 35% amino acid sequence identity. More closely related transcription factors can share at least about 50%, about 60%, about 65%, about 70%, about 75% or about 80% or about 90% or about 95% or about 98% or more sequence identity with the listed sequences. Factors that are most closely related to the listed sequences share, e.g., at least about 85%, about 90% or about 95% or more sequence identity to the listed sequences. At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences. The degeneracy of the genetic code enables major variations in the nucleotide sequence of a polynucleotide while maintaining the amino acid sequence of the encoded protein. Conserved domains within a transcription factor family may exhibit a higher degree of sequence homology, such as at least 65% sequence identity including conservative substitutions, and preferably at least 80% sequence identity. Exemplary conserved domains of the present invention include for example, for G1073 (SEQ ID NO: 1 and 2) amino acid residues 35 through 40 or 42 through 48 which are conserved in each of the sequences G2789 (SEQ ID NO: 3 and 4), G1945 (SEQ ID NO: 5 and 6) and G2155 (SEQ ID NO: 7 and 8). Transcription factors of the invention can also contain 5, 6, 8, 10 or 12 consecutive amino acids from the sequences of SEQ ID NO: 2, 4, 6 or 8 where the consecutive amino acids are taken from a region outside of the conserved domain. Polynucleotides having at least 90%, or at least 85%, or at least 75%, or at least 60%, or at least 50%, or at least 40% sequence identity to those encoding the above transcription factors are also included in this invention.

### Orthologs and Paralogs

Several different methods are known by those of skill in the art for identifying and defining these functionally homologous sequences. Three general methods for defining paralogs and orthologs are described; a paralog or ortholog may be identified by only one or more of the methods described below.

Orthologs and paralogs are evolutionarily related genes that have similar sequences and similar functions. Paralogs are related genes within a single species and are most likely a result of gene duplication, whereas orthologs are related genes in different species derived from a common ancestral molecule prior to speciation.

Within a single plant species, gene duplication may cause two copies of a particular gene, giving rise to two or more genes with similar sequence and similar function known as paralogs. A paralog is therefore a similar gene with a similar function within the same species. Paralogs typically cluster together or in the same clade (a group of similar genes), as is shown when a gene family phylogeny is analyzed using programs such as CLUSTAL (Thompson et al. (1994) Nucleic Acids Res. 22:4673-4680; Higgins et al. (1996) Methods Enzymol. 266 383-402). Groups of similar genes can also be identified using by pair-wise BLAST analysis (Feng and Doolittle (1987) J. Mol. Evol. 25:351-360). For example, a clade of very similar MADS domain transcription factors from Arabidopsis all share a common function in flowering time (Ratcliffe et al. (2001) Plant Physiol. 126:122-132), and a group of very similar AP2 domain transcription factors from Arabidopsis are involved in tolerance of plants to freezing (Gilmour et al. (1998) Plant J. 16:433-442). Analysis of groups of similar genes with similar function that fall within one clade can yield subsequences that are particular to the clade. These subsequences, known as consensus sequences, can not only be used to define the sequences within each clade, but define the functions of these genes, since genes within each clade typically share the same function. (See also, for example, Mount, D.W. (2001) Bioinformatics: Sequence and Genome Analysis Cold Spring Harbor Laboratory Press, Cold Spring Harbor, New York page 543.)

Speciation, the production of new species from a parental species, can also give rise to two or more genes with similar sequence and similar function. These genes, termed orthologs, often have an identical function within their host plants and are often interchangeable between species without losing function. Because plants have common ancestors, many genes in any plant species will have a corresponding orthologous gene in another plant species. Once a phylogenic tree for a gene family of one species has been constructed using a program such as CLUSTAL (Thompson et al. (1994) Nucleic Acids Res. 22:4673-4680; Higgins et al. (1996) Methods Enzymol. 266:383-402), potential orthologous sequences can placed into the phylogenetic tree and their relationship to genes from the species of interest can be determined. Once the ortholog pair has been identified, the function of the test ortholog can be determined by determining the function of the reference ortholog.

Orthologs can also be identified by pair-wise BLAST analysis by aligning a set of reference sequences against a set of test sequences. Test sequences with the closest match to a particular reference sequence, as determined by the P-value of the BLAST analysis, can be taken and individually aligned against the reference set of sequences. The individual test sequence will either best match the particular reference sequence, in which case it is likely to be an ortholog, or not, in which case it may not be an ortholog.

A further way of identifying an ortholog is by identifying a consensus sequence within the candidate ortholog. Using pair-wise BLAST analysis, or programs such as CLUSTAL alignment program, sets of similar genes, or clades, can be identified. The particular subsequences that define commonalities within a particular clade can be derived from an alignment of those sequences. Orthologs would have the consensus sequence, or a sequence similar to the consensus sequence. Orthologs might also have a consensus sequence outside a conserved domain, which could be particular to that family of orthologous sequences.

Corresponding orthologs may bridge the monocot/dicot division of the plant kingdom and orthologous pairs of genes can be identified in rice and Arabidopsis, corn and Arabidopsis and Antirhinnum and corn. For example Peng et al showed that a mutant of the *Arabidopsis* gene termed *Gibberellin Insensitive* (*GAI;* mutant termed *gai*) encoded a transcription factor and which conferred a reduction in gibberellin responsiveness in the native plant (Peng et al. 1997 Genes and Development 11:3194-3205). In addition, Peng et al. subsequently showed that the *Arabidopsis* GAI protein has 62 % amino acid residue identity with the wheat Rht-D1a protein and 62 % amino acid residue identity with the maize d8. Peng et al. showed that transgenic rice plants containing a mutant GAI allele give reduced response to gibberellin and are dwarfed, mimicking the dwarfed wheat variety from which the mutant *Rht-D1a* gene was isolated. Peng et al. taught that Arabidopsis GAI protein is an ortholog of the wheat Rht-D1a and maize d8 proteins. (Peng et al. 1999 Nature 400:256-261.)

In addition Fu et al. (2001 Plant Cell 13:1791-1802), Nandi et al. (2000 Curr. Biol. 10:215-218), Coupland (1995 Nature 377:482-483), and Weigel and Nilsson (1995 Nature 377:482-500) show that an Arabidopsis transcription factor expressed in an exogenous plant species elicits the same or very similar phenotypic response. Furthermore, Kater et al. (1998 Plant Cell 10:171-182), Mandel et al. (1992 Cell 71-133-143), and Suzuki et al. (2001 Plant J. 28:409-418) showed that a transcription factor expressed in another plant species elicits the same or very similar phenotypic response of the endogenous sequence, as often predicted in earlier studies of Arabidopsis transcription factors in Arabidopsis.

Table 1 (appended to this application) lists a summary of orthologous and homologous sequences identified using BLAST (tblastx program) and the standard BLAST result data generated from a search. The first column shows the polynucleotide sequence identifier (SEQ ID NO), the second column shows the transcription factor cDNA identifier (Gene ID), the third column shows the GenBank Accession Number of the orthologous or homologous polynucleotide sequence identified in a BLAST search (Test Sequence ID), the fourth column shows the calculated probability value that the sequence identity is due to chance (Smallest Sum Probability), the fifth column identifies the plant species of the Test Sequence (Test Sequence Species), and the sixth column shows the GenBank annotation for the sequence identified in a BLAST search (Test Sequence GenBank Annotation).

Table 2 (appended to this application) lists orthologous and homologous sequences identified using BLAST (tblastx program) and the standard BLAST result data generated from a search. The first column shows the polynucleotide sequence identifier (SEQ ID NO), the second column shows the transcription factor cDNA identifier (Gene ID), the third column shows the GenBank Accession Number of the orthologous or homologous polynucleotide (Test Sequence ID), the fourth column shows the GenBank annotation for the sequence identified in a BLAST search (Test Sequence GenBank Annotation), the fifth column shows the reading frame of the Test sequence encoding the orthologous or homologous sequence (Reading Frame), the sixth column shows the calculated score value of the aligned sequences (High Score), the seventh column shows the calculated probability value that the sequence identity is due to chance (Smallest Sum Probability), and the eighth column shows the number of regions in the Test Sequence that align with a sequence from the SEQ ID NO. (N).

### Identifying Nucleic Acids by Hybridization

Polynucleotides homologous to the sequences illustrated in the Sequence Listing can be identified in a variety of ways known to one skilled in the art, e.g., by hybridization to each other under stringent or under highly stringent conditions. Single stranded polynucleotides hybridize when they associate based on a variety of well characterized physico-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number), as described in more detail in the references cited above.

An example of stringent hybridization conditions for hybridization of complementary nucleic acids which have more than 100 complementary residues on a filter in a Southern or northern blot is about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Nucleic acid molecules that hybridize under stringent conditions will typically hybridize to a probe based on either the entire cDNA or selected portions, e.g., to a unique subsequence, of the cDNA under wash conditions of 0.2x SSC to 2.0 x SSC, 0.1% SDS at 50-65° C, for example 0.2 x SSC, 0.1% SDS at 65° C. For identification of less closely related homologues washes can be performed at a lower temperature, e.g., 50° C. In general, stringency is increased by raising the wash temperature and/or decreasing the concentration of SSC.

As another example, stringent conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. Conditions can be selected such that a higher signal to noise ratio is observed in the particular assay which is used, e.g., about 15x, 25x, 35x, 50x or more. Accordingly, the subject nucleic acid hybridizes to the unique coding oligonucleotide with at least a 2x higher signal to noise ratio as compared to hybridization of the coding oligonucleotide to a nucleic acid encoding known polypeptide. Again, higher signal to noise ratios can be selected, e.g., about 5x, 10x, 25x, 35x, 50x or more. The particular signal will depend on the label used in the relevant assay, e.g., a fluorescent label, a colorimetric label, a radioactive label, or the like.

Alternatively, transcription factor homologue polypeptides can be obtained by screening an expression library using antibodies specific for one or more transcription factors. With the disclosed transcription factor and transcription factor homologue nucleic acid sequences, the encoded polypeptide(s) can be expressed and purified in a heterologous expression system (e.g., *E. coli*) and used to raise antibodies (monoclonal or polyclonal) specific for the polypeptide(s) in question. Antibodies can also be raised against synthetic peptides derived from transcription factor, or transcription factor homologue, amino acid sequences. Methods of raising antibodies are well known in the art and are described in Harlow and Lane (1988) Antibodies: A Laboratory Manual, Cold Spring Harbor Laboratory, New York. Such antibodies can then be used to screen an expression library produced from the plant from which it is desired to clone additional transcription factor homologues, using the methods described above. The selected cDNAs can be confirmed by sequencing and enzymatic activity.

### SEQUENCE VARIATIONS

It will readily be appreciated by those of skill in the art, that any of a variety of polynucleotide sequences are capable of encoding the transcription factors and transcription factor homologue polypeptides Due to the degeneracy of the genetic code, many different polynucleotides can encode identical and/or substantially similar polypeptides in addition to those sequences illustrated in the Sequence Listing.

For example, Table 3 illustrates, e.g., that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

**Table 3**

| Amino acids | | | Codon | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art. Accordingly, any and all such variations of a sequence selected from the above table are a feature of the invention.

In addition to silent variations, other conservative variations that alter one, or a few amino acids in the encoded polypeptide, can be made without altering the function of the polypeptide, these conservative variants are, likewise, a feature of the invention.

For example, substitutions, deletions and insertions introduced into the sequences provided in the Sequence Listing are also envisioned by the invention. Such sequence modifications can be engineered into a sequence by site-directed mutagenesis (Wu (ed.) Meth. Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 4 when it is desired to maintain the activity of the protein. Table 4 shows amino acids which can be substituted for an amino acid in a protein and which are typically regarded as conservative substitutions.

**Table 4**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser; Val |
| Trp | Tyr |
| Tyr | Trp; Phe |
| Val | Ile; Leu |

Substitutions that are less conservative than those in Table 4 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

### FURTHER MODIFYING SEQUENCES OF THE INVENTION-MUTATION/FORCED EVOLUTION

In addition to generating silent or conservative substitutions as noted, above, methods of modifying the sequences of the Sequence Listing may be employed. In the methods, nucleic acid or protein modification methods are used to alter the given sequences to produce new sequences and/or to chemically or enzymatically modify given sequences to change the properties of the nucleic acids or proteins.

Thus, in one embodiment, given nucleic acid sequences are modified, e.g., according to standard mutagenesis or artificial evolution methods to produce modified sequences. For example, Ausubel, *supra,* provides additional details on mutagenesis methods. Artificial forced evolution methods are described, e.g., by Stemmer (1994) Nature 370:389-391, and Stemmer (1994) Proc. Natl. Acad. Sci. USA 91:10747-10751. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, the invention provides for modification of any given nucleic acid by mutation, evolution, chemical or enzymatic modification, or other available methods, as well as for the products produced by practicing such methods, e.g., using the sequences herein as a starting substrate for the various modification approaches.

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for *S. cerevisiae* and mammals are TAA and TGA, respectively. The preferred stop codon for monocotyledonous plants is TGA, whereas insects and *E. coli* prefer to use TAA as the stop codon.

The polynucleotide sequences of the present invention can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides can be combined with domains derived from other transcription factors or synthetic domains to modify the biological activity of a transcription factor. For instance, a DNA binding domain derived from a transcription factor can be combined with the activation domain of another transcription factor or with a synthetic activation domain. A transcription activation domain assists in initiating transcription from a DNA binding site. Examples include the transcription activation region of VP16 or GAL4 (Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, (1987) Nature 330:670-672).

### EXPRESSION AND MODFFICATION OF POLYPEPTIDES

Typically, polynucleotide sequences are incorporated into recombinant DNA (or RNA) molecules that direct expression of polypeptides of the invention in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homologue.

### Vectors, Promoters and Expression Systems

The present invention makes use of recombinant constructs comprising one or more of the nucleic acid sequences herein. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available.

General texts that describe molecular biological techniques useful herein, including the use and production of vectors, promoters and many other relevant topics, include Berger, Sambrook and Ausubel, *supra.* Any of the identified sequences can be incorporated into a cassette or vector, e.g., for expression in plants. A number of expression vectors suitable for stable transformation of plant cells or for the establishment of transgenic plants have been described including those described in Weissbach and Weissbach, (1989) Methods for Plant Molecular Biology, Academic Press, and Gelvin et al., (1990) Plant Molecular Biology Manual, Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucl Acid Res. 12: 8711-8721, Klee (1985) Bio/Technology 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/Technology 10:667-674; Wan and Lemeaux (1994) Plant Physiol 104: 37-48, and for Agrobacterium-mediated DNA transfer (Ishida et al. (1996) Nature Biotech 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

Examples of constitutive plant promoters which can be useful for expressing the TF sequence include: the cauliflower mosaic virus (CaMV) 35S promoter, which confers constitutive, high-level expression in most plant tissues (*see,* e.g., Odel et al. (1985) Nature 313:810); the nopaline synthase promoter (An et al. (1988) Plant Physiol 88:547); and the octopine synthase promoter (Fromm et al. (1989) Plant Cell 1: 977).

A variety of plant gene promoters that regulate gene expression in response to environmental, hormonal, chemical, developmental signals, and in a tissue-specific or preferential manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorable be employed to promote expression of a polynucleotide of the invention in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol Biol 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol Biol 37:977-988), flower-specific (Kaiser et al, (1995) Plant Mol Biol 28:231-243), pollen (Baerson et al. (1994) Plant Mol Biol 26:1947-1959), carpels (Ohl et al. (1990) Plant Cell 2:837-848), pollen and ovules (Baerson et al. (1993) Plant Mol Biol 22:255-267), auxin-inducible promoters (such as that described in van der Kop et al. (1999) Plant Mol Biol 39:979-990 or Baumann et al. (1999) Plant Cell 11:323-334), cytokinin-inducible promoter (Guevara-Garcia (1998) Plant Mol Biol 38:743-753), promoters responsive to gibberellin (Shi et al. (1998) Plant Mol Biol 38:1053-1060, Willmott et al. (1998) 38:817-825) and the like. Additional promoters are those that elicit expression in response to heat (Ainley et al. (1993) Plant Mol Biol 22: 13-23), light (e.g., the pea rbcS-3A promoter, Kuhlemeier et al. (1989) Plant Cell 1:471, and the maize rbcS promoter, Schaffner and Sheen (1991) Plant Cell 3: 997); wounding (e.g., *wunI*, Siebertz et al. (1989) Plant Cell 1: 961); pathogens (such as the PR-1 promoter described in Buchel et al. (1999) Plant Mol. Biol. 40:387-396, and the PDF1.2 promoter described in Manners et al. (1998) Plant Mol. Biol. 38:1071-80), and chemicals such as methyl jasmonate or salicylic acid (Gatz et al. (1997) Ann. Rev. Plant Physiol. Plant Mol Biol 48: 89-108). In addition, the timing of the expression can be controlled by using promoters such as those acting at senescence (Gan and Amasino (1995) Science 270: 1986-1988); or late seed development (Odell et al. (1994) Plant Physiol 106:447-458).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

### Additional Expression Elements

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed. However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

### Expression Hosts

The present invention also relates to host cells which are transduced with vectors, and the production of polypeptides of the invention (including fragments thereof) by recombinant techniques. Host cells are genetically engineered (i.e., nucleic acids are introduced, e.g., transduced, transformed or transfected) with the vectors, which may be, for example, a cloning vector or an expression vector comprising the relevant nucleic acids herein. The vector is optionally a plasmid, a viral particle, a phage, a naked nucleic acids, *etc*. The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook and Ausubel.

The host cell can be a eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al., (1985) Proc. Natl. Acad. Sci. USA 82, 5824, infection by viral vectors such as cauliflower mosaic virus (CaMV) (Hohn et al., (1982) Molecular Biology of Plant Tumors, (Academic Press, New York) pp. 549-560; US 4,407,956), high velocity ballistic penetration by small particles with the nucleic acid either within the matrix of small beads or particles, or on the surface (Klein et al., (1987) Nature 327, 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens*, and a portion is stably integrated into the plant genome (Horsch et al. (1984) Science 233:496-498; Fraley et al. (1983) Proc. Natl. Acad. Sci. USA 80,4803).

The cell can include a nucleic acid which encodes a polypeptide, wherein the cells expresses a polypeptide. The cell can also include vector sequences, or the like. Furthermore, cells and transgenic plants which include any polypeptide or nucleic acid above or throughout this specification, e.g., produced by transduction of a vector of the invention, are an additional feature of the invention.

For long-term, high-yield production of recombinant proteins, stable expression can be used. Host cells transformed with a nucleotide sequence encoding a polypeptide of the invention are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins of the invention can be designed with signal sequences which direct secretion of the mature polypeptides through a prokaryotic or eukaryotic cell membrane.

### PRODUCTION OF TRANSGENIC PLANTS

### Modification of Traits

The polynucleotides described above are favorably employed to produce transgenic plants with various traits, or characteristics, that have been modified in a desirable manner, e.g., to improve plant biomass. For example, alteration of expression levels or patterns (e.g., spatial or temporal expression patterns) of one or more of the transcription factors (or transcription factor homologues) of the invention, as compared with the levels of the same protein found in a wild type plant, can be used to modify a plant's traits. An illustrative example of trait modification, improved plant biomass, by altering expression levels of a particular transcription factor is described further in the Examples and the Sequence Listing.

### Antisense and Cosuppression Approaches

In addition to expression of the nucleic acids as gene replacement or plant phenotype modification nucleic acids, the nucleic acids are also useful for sense and anti-sense suppression of expression, e.g., to down-regulate expression of a nucleic acid of the invention, e.g., as a further mechanism for modulating plant phenotype. That is, the nucleic acids, or subsequences or anti-sense sequences thereof, can be used to block expression of naturally occurring homologous nucleic acids. A variety of sense and anti-sense technologies are known in the art, e.g., as set forth in Lichtenstein and Nellen (1997) Antisense Technology: A Practical Approach IRL Press at Oxford University, Oxford, England. In general, sense or anti-sense sequences are introduced into a cell, where they are optionally amplified, e.g., by transcription. Such sequences include both simple oligonucleotide sequences and catalytic sequences such as ribozymes.

For example, a reduction or elimination of expression (i.e., a "knockout") of a transcription factor or transcription factor homologue polypeptide in a transgenic plant, e.g., to modify a plant trait, can be obtained by introducing an antisense construct corresponding to the polypeptide of interest as a cDNA. For antisense suppression, the transcription factor or homologue cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous transcription factor gene in the plant cell.

Suppression of endogenous transcription factor gene expression can also be achieved using a ribozyme. Ribozymes are RNA molecules that possess highly specific endoribonuclease activity. The production and use of ribozymes are disclosed in U.S. Patent No. 4,987,071 and U.S. Patent No. 5,543,508. Synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a transcription factor or transcription factor homologue cDNA is over-expressed can also be used to obtain co-suppression of a corresponding endogenous gene, e.g., in the manner described in U.S. Patent No. 5,231,020 to Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, e.g., as the introduced sequence is lengthened, and/or as the sequence similarity between the introduced sequence and the endogenous transcription factor gene is increased.

Vectors expressing an untranslatable form of the transcription factor mRNA, e.g., sequences comprising one or more stop codon, or nonsense mutation) can also be used to suppress expression of an endogenous transcription factor, thereby reducing or eliminating it's activity and modifying one or more traits. Methods for producing such constructs are described in U.S. Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141).

Another method for abolishing the expression of a gene is by insertion mutagenesis using the T-DNA of *Agrobacterium tumefaciens.* After generating the insertion mutants, the mutants can be screened to identify those containing the insertion in a transcription factor or transcription factor homologue gene. Plants containing a single transgene insertion event at the desired gene can be crossed to generate homozygous plants for the mutation (Koncz et al. (1992) Methods in Arabidopsis Research, World Scientific).

Alternatively, a plant phenotype can be altered by eliminating an endogenous gene, such as a transcription factor or transcription factor homologue, e.g., by homologous recombination (Kempin et al. (1997) Nature 389:802).

A plant trait can also be modified by using the cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides can also be expressed in a plant in the absence of an expression cassette by manipulating the activity or expression level of the endogenous gene by other means. For example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide of the invention *(See*, e.g., PCT Publications WO 96/06166 and WO 98/53057 which describe the modification of the DNA binding specificity of zinc finger proteins by changing particular amino acids in the DNA binding motif).

The transgenic plant can also include the cellular machinery or mechanisms necessary for expressing or altering the activity of a polypeptide encoded by an endogenous gene, for example by altering the phosphorylation state of the polypeptide to maintain it in an activated state.

Transgenic plants (or plant cells, or plant explants, or plant tissues) incorporating the polynucleotides and/or expressing the polypeptides can be produced by a variety of well established techniques as described above. Following construction of a vector, most typically an expression cassette, including a polynucleotide, e.g., encoding a transcription factor or transcription factor homologue, standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, com, rice, barley, millet, etc.), *Solanaceae* (potato, tomato, tobacco, peppers, etc.), and various other crops. See protocols described in Ammirato et al. (1984) Handbook of Plant Cell Culture -Crop Species. Macmillan Publ. Co. Shimamoto et al. (1989) Nature 338:274-276; Fromm et al. (1990) Bio/Technology 8:833-839; and Vasil et al. (1990) Bio/Technology 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumeficiens* mediated transformation. Transformation means introducing a nucleotide sequence in a plant in a manner to cause stable or transient expression of the sequence.

Successful examples of the modification of plant characteristics by transformation with cloned sequences which serve to illustrate the current knowledge in this field of technology, and which are herein incorporated by reference, include: U.S. Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modified trait can be any of those traits described above. Additionally, to confirm that the modified trait is due to changes in expression levels or activity of the polypeptide or polynucleotide of the invention can be determined by analyzing mRNA expression using Northern blots, RT-PCR or microarrays, or protein expression using immunoblots or Western blots or gel shift assays.

### INTEGRATED SYSTEMS-SEQUENCE IDENTITY

One aspect of this disclosure relates to an integrated system, computer or computer readable medium that comprises an instruction set for determining the identity of one or more sequences in a database. In addition, the instruction set can be used to generate or identify sequences that meet any specified criteria. Furthermore, the instruction set may be used to associate or link certain functional benefits, such as an improved plant biomass, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madison, WI). Public sequence databases such as GenBank, EMBL, Swiss-Prot and PIR or private sequence databases such as PhytoSeq (Incyte Pharmaceuticals, Palo Alto, CA) can be searched.

Alignment of sequences for comparison can be conducted by the local homology algorithm of Smith and Waterman (1981) Adv. Appl. Math. 2:482, by the homology alignment algorithm of Needleman and Wunsch (1970) J. Mol. Biol. 48:443, by the search for similarity method of Pearson and Lipman (1988) Proc. Natl. Acad. Sci. U.S.A. 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al., *supra.*

A variety of methods of determining sequence relationships can be used, including manual alignment and computer assisted sequence alignment and analysis. This later approach is a preferred approach in the present invention, due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et al. J. Mol. Biol 215:403-410 (1990). Software for performing BLAST analyses is publicly available, e.g., through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff (1989) Proc. Natl. Acad. Sci. USA 89:10915).

In addition to calculating percent sequence identity, the BLAST algorithm also performs a statistical analysis of the similarity between two sequences (*see,* e.g., Karlin & Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element which displays an alignment produced by the alignment instruction set.

The methods can be implemented in a localized or distributed computing environment. In a distributed environment, the methods may implemented on a single computer comprising multiple processors or on a multiplicity of computers. The computers can be linked, e.g. through a common bus, but more preferably the computer(s) are nodes on a network. The network can be a generalized or a dedicated local or wide-area network and, in certain preferred embodiments, the computers may be components of an intra-net or an internet.

Thus, there are provided herein methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

### EXAMPLES

The following examples are intended to illustrate but not limit the present invention.

### EXAMPLE I. FULL LENGTH GENE IDENTIFICATION AND CLONING

Putative transcription factor sequences (genomic or ESTs) related to known transcription factors were identified in the *Arabidopsis thaliana* GenBank database using the tblastn sequence analysis program using default parameters and a P-value cutoff threshold of-4 or-5 or lower, depending on the length of the query sequence. Putative transcription factor sequence hits were then screened to identify those containing particular sequence strings. If the sequence hits contained such sequence strings, the sequences were confirmed as transcription factors.

Alternatively, Arabidopsis *thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60° C) and labeled with ³²P dCTP using the High Prime DNA Labeling Kit (Boehringer Mannheim). Purified radiolabelled probes were added to filters immersed in Church hybridization medium (0.5 M NaPO₄ pH 7.0, 7% SDS, 1 % w/v bovine serum albumin) and hybridized overnight at 60 °C with shaking. Filters were washed two times for 45 to 60 minutes with 1xSCC, 1% SDS at 60°C.

To identify additional sequence 5' or 3' of a partial cDNA sequence in a cDNA library, 5' and 3' rapid amplification of cDNA ends (RACE) was performed using the Marathon^{™} cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the Marathon^{™} Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

### EXAMPLE II. CONSTRUCTION OF EXPRESSION VECTORS

The sequence was amplified from a genomic or cDNA library using primers specific to sequences upstream and downstream of the coding region. The expression vector was pMEN20 or pMEN65, which are both derived from pMON316 (Sanders et al, (1987) Nucleic Acids Research 15:1543-58) and contain the CaMV 35S promoter to express transgenes. To clone the sequence into the vector, both pMEN20 and the amplified DNA fragment were digested separately with SalI and NotI restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the *E*. *coli* strain DH5alpha by using the heat shock method. The transformations were plated on LB plates containing 50 mg/l kanamycin (Sigma).

Individual colonies were grown overnight in five milliliters of LB broth containing 50 mg/l kanamycin at 37° C. Plasmid DNA was purified by using Qiaquick Mini Prep kits (Qiagen, CA).

### EXAMPLE III. TRANSFORMATION OF AGROBACTERIUM WITH THE EXPRESSION VECTOR

After the plasmid vector containing the gene was constructed, the vector was used to transform *Agrobacterium tumefaciens* cells expressing the gene products. The stock of *Agrobacterium tumefaciens* cells for transformation was made as described by Nagel et al. (1990) FEMS Microbiol Letts. 67: 325-328. *Agrobacterium* strain ABI was grown in 250 ml LB medium (Sigma) overnight at 28°C with shaking until an absorbance (A₆₀₀) of 0.5 - 1.0 was reached. Cells were harvested by centrifugation at 4,000 x g for 15 min at 4° C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. For each DNA construct to be transformed, 50 -100 ng DNA (generally resuspended in 10 mM Tris-HCl, 1 mM EDTA, pH 8.0) was mixed with 40 µl of *Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 µg/ml spectinomycin (Sigma) and incubated for 24-48 hours at 28° C. Single colonies were then picked and inoculated in fresh medium. The presence of the plasmid construct was verified by PCR amplification and sequence analysis.

### EXAMPLE IV. TRANSFORMATION OF ARABIDOPSIS PLANTS WITH AGROBACTERIUM TUMEFACIENS WITH EXPRESSION VECTOR

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 µM benzylamino purine (Sigma), 200 µl/L Silwet L-77 (Lehle Seeds) until an absorbance (A₆₀₀) of 0.8 was reached.

Prior to transformation, *Arabidopsis thaliana* seeds (ecotype Columbia) were sown at a density of ~10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### EXAMPLE V. IDENTIFICATION OF ARABIDOPSIS PRIMARY TRANSFORMANTS

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1% (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4° C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).

Primary transformants were crossed and progeny seeds (T₂) collected; kanamycin resistant seedlings were selected and analyzed. The expression levels of the recombinant polynucleotides in the transformants vary from about a 5% expression level increase to a least a 100% expression level increase. Similar observations are made with respect to polypeptide level expression.

### EXAMPLE VI. IDENTIFICATION OF ARABIDOPSIS PLANTS WITH TRANSCRIPTION FACTOR GENE KNOCKOUTS

The screening of insertion mutagenized *Arabidopsis* collections for null mutants in a known target gene was essentially as described in Krysan et al (1999) Plant Cell 11:2283-2290. Briefly, gene-specific primers, nested by 5-250 base pairs to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23, 1998.

### EXAMPLE VII. IDENTIFICATION OF OVEREXPRESSOR OR GENE KNOCKOUT PLANTS WITH MODIFIED PLANT BIOMASS

Experiments were performed to identify those transformants or knockouts that exhibited a modified biomass phenotype. The plants were grown under continuous light conditions at 20-25° C. For such studies, the transformants' leaves and seeds were observed for a modified phenotype. For plant dry weight determination, a plant was placed in an oven for 3 days at 65 to 70 °C.

We observed that plants overexpressing G1073 (SEQ ID NO: 1 and 2) constitutively (three independent T2 populations having 6 plants in one population and 16 plants in each of the other two) had increased biomass as measured by an increase in the plant fresh weight, the plant's dry weight or the seed yield compared with control plants transformed with an empty transformation vector under the control of the 35S promoter. Typically, the plant fresh weight, dry weight or seed yield were increased by at least 150%.

We identified additional genes that are related to G1073 based on sequence identity and therefore are suitable for increasing plant biomass. The genes were G2789 (SEQ ID NO: 3 and 4), G1945 (SEQ ID NO: 5 and 6), and G2155 (SEQ ID NO: 7 and 8). G2789 shares 89% sequence identity over a conserved domain of G1073 (amino acid residues 33 through 50 of SEQ ID NO: 2), whereas G1945 shares about an 89% sequence identity over the same domain and G2155 shares a 78% sequence identity over that domain. G2155 and G1945 share an 83% sequence identity over that region. To confirm that these related transcription factors could be used to modify plant biomass, we measured changes in plant biomass for G2155 or G2789 overexpressors. We observed that when either G2155 or G2789 were overexpressed in plants the transformed plants were substantially larger than the wild type plant.

Genes or sequences selected from Table 1 or Table 2 can also be overexpressed or knocked out in a plant to produce a plant with modified biomass. Preferably, the sequence selected (Test Sequence) from Table 1 or Table 2 is overexpressed in the same species listed for the selected sequences, however, another species may be used.

### EXAMPLE VIII. IDENTIFICATION OF HOMOLOGOUS SEQUENCES

Homologous sequences from Arabidopsis and plant species other than Arabidopsis are identified using database sequence search tools, such as the Basic Local Alignment Search Tool (BLAST) (Altschul et al. (1990) J. Mol. Biol. 215:403-410; and Altschul et al. (1997) Nucl. Acid Res. 25: 3389-3402). The tblastx sequence analysis programs are employed using the BLOSUM-62 scoring matrix (Henikoff, S. and Henikoff, J. G. (1992) Proc. Natl. Acad. Sci. USA 89: 10915-10919).

Identified Arabidopsis homologous sequences are provided in Tables 1 and 2, appended to this application.. The percent sequence identity among these sequences can be as low as 47%, or even lower sequence identity. The entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaliana*). These sequences were compared to sequences representing genes of SEQ ID NO: 1-8 using the Washington University TBLASTX algorithm (version 2.0a19MP) at the default settings using gapped alignments with the filter "off For each gene of SEQ ID NO: 1-8, individual comparisons are ordered by probability score (P-value), where the score reflects the probability that a particular alignment occurred by chance. For example, a score of 3.6e-40 is 3.6 x 10-40. In addition to P-values, comparisons are also scored by percentage identity. Percentage identity reflects the degree to which two segments of DNA or protein are identical over a particular length.

Table 1 (appended to this application) lists a summary of orthologous and homologous sequences identified using BLAST (tblastx program) and the standard BLAST result data generated from a search. The first column shows the polynucleotide sequence identifier (SEQ ID NO), the second column shows the transcription factor cDNA identifier (Gene ID), the third column shows the GenBank Accession Number of the orthologous or homologous polynucleotide sequence identified in a BLAST search (Test Sequence ID), the fourth column shows the calculated probability value that the sequence identity is due to chance (Smallest Sum Probability), the fifth column identifies the plant species of the Test Sequence (Test Sequence Species), and the sixth column shows the GenBank annotation for the sequence identified in a BLAST search (Test Sequence GenBank Annotation).

Table 2 (appended to this application) lists orthologous and homologous sequences identified using BLAST (tblastx program) and the standard BLAST result data generated from a search. The first column shows the polynucleotide sequence identifier (SEQ ID NO), the second column shows the transcription factor cDNA identifier (Gene ID), the third column shows the GenBank Accession Number of the orthologous or homologous polynucleotide (Test Sequence ID), the fourth column shows the GenBank annotation for the sequence identified in a BLAST search (Test Sequence GenBank Annotation), the fifth column shows the reading frame of the Test sequence encoding the orthologous or homologous sequence (Reading Frame), the sixth column shows the calculated score value of the aligned sequences (High Score), the seventh column shows the calculated probability value that the sequence identity is due to chance (Smallest Sum Probability), and the eighth column shows the number of regions in the Test Sequence that align with a sequence from the SEQ ID NO. (N).

**Table 1**

| SEQ ID NO | Gene ID | Test Sequence ID | Smallest Sum Probability | Test Sequence Species | Test Sequence GenBank Annotation |
|---|---|---|---|---|---|
| 1 | G1073 | AP004165 | 4.10E-68 | [Oryza sativa] | chromosome 2 clone OJ1479_B12, *** SEQUENCING |
| 1 | G1073 | BH730050 | 1.10E-57 | [Brassica oleracea] | BOMEB22TF BO_2_3_KB Brassica oleracea gen |
| 1 | G1073 | BG134451 | 2.80E-57 | [Lycopersicon esculentum] | EST467343 tomato crown gall Lycoper |
| 1 | G1073 | BG581882 | 4.10E-57 | [Medicago truncatula] | EST483618 GVN Medicago truncatula cDNA |
| 1 | G1073 | BM110212 | 2.40E-54 | [Solanum tuberosum] | EST557748 potato roots Solanum tuberosum |
| 1 | G1073 | BI321563 | 7.00E-50 | [Glycine max] | saf12g07.y3 Gm-c1076 Glycine max cDNA clone GEN |
| 1 | G1073 | BF254863 | 3.70E-47 | [Hordeum vulgare] | HVSMEf0005E05f Hordeum vulgare seedling roo |
| 1 | G1073 | AW720668 | 5.50E-42 | [Lotus japonicus] | LjNEST14h9rc Lotus japonicus nodule library |
| 1 | G1073 | BF588135 | 6.10E-30 | [Sorghum propinquum] | FM1_38_B09.b1_A003 Floral-Induced Merist |
| 1 | G1073 | BH173434 | 4.10E-28 | [Zea mays] | 0104B13 maize genomic ORF library F Zea mays genom |
| 1 | G1073 | gi15528814 | 2.60E-37 | [Oryza sativa] | hypothetical protein-similar to Arabidopsis |
| 1 | G1073 | gi2213536 | 5.50E-21 | [Pisum sativum] | DNA-binding protein PD1. |
| 1 | G1073 | gi4165183 | 2.70E-19 | [Antirrhinum majus] | SAP1 protein. |
| 1 | G1073 | gi1276971 | 1.80E-05 | [Daucus carota] | glycine-rich protein. |
| 1 | G1073 | gi19867 | 0.0002 | [Nicotiana tabacum] | extensin (AA 1-620). |
| 1 | G1073 | gi1155068 | 0.00031 | [Brassica napus] | cell wall-plasma membrane linker protein. |
| 1 | G1073 | gi168457 | 0.00078 | [Zea mays] | cell wall protein (put.); putative. |
| 1 | G1073 | gi6523547 | 0.0018 | [Volvox carteri f. nagariensis] | hydroxyprofine-rich glycopr |
| 1 | G1073 | gi1166450 | 0.0028 | [Lycopersicon esculentum] | Tfm5. |
| 1 | G1073 | gi11545668 | 0.004 | [Chlamydomonas reinhardtii] | ClA5. |

**Table 2**

| SEQ ID NO | Gene ID | Test Sequence ID | Test Sequence GenBank Annotation | Reading Frame | High Score | Smallest Sum Probability | N |
|---|---|---|---|---|---|---|---|
| 1 | G1073 | AP004165 | AP004165 Oryza sativa chromosome 2 clone OJ1... | -3 | 721 | 4.10E-68 | 1 |
| 1 | G1073 | OSJN00182 | AL662981 Oryza sativa chromosome 4 clone OSJ... | 1 | 684 | 3.40E-64 | 1 |
| 1 | G1073 | AP004757 | AP004757 Oryza sativa chromosome 6 clone P06... | -3 | 659 | 1.50E-61 | 1 |
| 1 | G1073 | BH730050 | BH730050 BOMEB22TF BO_2_3_KB Brassica olerac... | -1 | 614 | 1.10E-57 | 1 |
| 1 | G1073 | BG134451 | BG134451 EST467343 tomato crown gall Lycoper... | 1 | 612 | 2.80E-57 | 1 |
| 1 | G1073 | BG581882 | BG581882 EST483618 GVN Medicago truncatula c... | 3 | 609 | 4.10E-57 | 1 |
| 1 | G1073 | AP004020 | AP004020 Oryza sativa chromosome 2 clone OJ1... | 2 | 616 | 5.40E-57 | 1 |
| 1 | G1073 | BH660108 | BH660108 BOHVI48TF BO_2_3_KB Brassica olerac... | -1 | 595 | 1.10E-55 | 1 |
| 1 | G1073 | BH550632 | BH550632 BOGUC18TR BOGU Brassica oleracea ge... | -2 | 594 | 1.40E-55 | 1 |
| 1 | G1073 | BH419879 | BH419879 BOGGX69TF BOGG Brassica oleracea ge... | -2 | 588 | 6.60E-55 | 1 |
| 1 | G1073 | AW774484 | AW774484 EST333635 KV3 Medicago truncatula c... | 1 | 588 | 7.50E-55 | 1 |
| 1 | G1073 | AP004635 | AP004635 Oryza sativa chromosome 8 clone P06... | -2 | 594 | 1.20E-54 | 1 |
| 1 | G1073 | AW574000 | AW574000 EST316591 GVN Medicago truncatula c... | 1 | 586 | 1.20E-54 | 1 |
| 1 | G1073 | BG583651 | BG583651 EST485403 GVN Medicago truncatula c... | 3 | 307 | 1.20E-54 | 3 |
| 1 | G1073 | BG647144 | BG647144 EST508763 HOGA Medicago truncatula ... | 1 | 585 | 1.40E-54 | 1 |
| 1 | G1073 | AP003526 | AP003526 Oryza sativa chromosome 6 clone P05... | 1 | 593 | 1.50E-54 | 1 |
| 1 | G1073 | AP004698 | AP004698 Oryza sativa chromosome 8 clone P04... | 2 | 591 | 2.40E-54 | 1 |
| 1 | G1073 | BM110212 | BM110212 EST557748 potato roots Solanum tube... | 1 | 583 | 2.40E-54 | 1 |
| 1 | G1073 | BH685875 | BH685875 BOMBO90TR BO_2_3_KB Brassica olerac... | -1 | 578 | 9.60E-54 | 1 |
| 1 | G1073 | BH566718 | BH566718 BOHCV23TR BOHC Brassica oleracea ge... | -2 | 575 | 1.70E-53 | 1 |
| 1 | G1073 | AL366947 | AL366947 MtBA11B10F1 MtBA Medicago truncatul... | 1 | 568 | 1.40E-52 | 1 |
| 1 | G1073 | AP003891 | AP003891 Oryza sativa chromosome 8 clone OJ1... | 2 | 573 | 2.00E-52 | 1 |
| 1 | G1073 | AP004587 | AP004587 Oryza sativa chromosome 8 clone P05... | 3 | 573 | 2.00E-52 | 1 |
| 1 | G1073 | BH729110 | BH729110 BOHVN82TF BO_2_3_KB Brassica olerac... | -2 | 557 | 1.40E-51 | 1 |
| 1 | G1073 | BH459056 | BH459056 BOGKX90TR BOGK Brassica oleracea ge... | -2 | 548 | 1.20E-50 | 1 |
| 1 | G1073 | BH717588 | BH717588 BOMDB19TF BO_2_3_KB Brassica olerac... | 2 | 547 | 1.80E-50 | 1 |
| 1 | G1073 | B1321563 | BI321563 saf12g07.y3 Gm-c1076 Glycine max cD... | 1 | 542 | 7.00E-50 | 1 |
| 1 | G1073 | BH481983 | BH481983 BOGYI73TR BOGY Brassica oleracea ge... | 2 | 536 | 2.20E-49 | 1 |
| 1 | G1073 | AW980581 | AW980581 EST391734 GVN Medicago truncatula c... | 3 | 526 | 3.10E-48 | 1 |
| 1 | G1073 | BE203784 | BE203784 EST396460 KV0 Medicago truncatula c... | 2 | 526 | 4.30E-48 | 1 |
| 1 | G1073 | AW776082 | A776082 EST335147 DSIL Medicago truncatula ... | 3 | 522 | 8.80E-48 | 1 |
| 1 | G1073 | BF254863 | BF254863 HVSMEf0005E05f Hordeum vulgare seed... | 2 | 516 | 3.70E-47 | 1 |
| 1 | G1073 | BH664974 | BH664974 BOMBC11TF BO_2_3_KB Brassica olerac... | 2 | 514 | 4.50E-47 | 1 |
| 1 | G1073 | BG583687 | BG583687 EST485440 GVN Medicago truncatula c... | 2 | 513 | 6.10E-47 | 1 |
| 1 | G1073 | BH596283 | BH596283 BOGBL42TR BOGB Brassica oleracea ge... | 2 | 506 | 3.40E-46 | 1 |
| 1 | G1073 | BE999313 | BE999313 EST431036 GVSN Medicago truncatula ... | 1 | 395 | 7.20E-45 | 2 |
| 1 | G1073 | AW349284 | AW349284 GM210004B21H7 Gm-r1021 Glycine max ... | -2 | 411 | 1.20E-44 | 2 |
| 1 | G1073 | BF067277 | BF067277 st37f11.y1 Gm-c1067 Glycine max cDN... | 3 | 489 | 4.10E-44 | 1 |
| 1 | G1073 | BF004270 | BF004270 EST432768 KV1 Medicago truncatula c... | 3 | 481 | 2.50E-43 | 1 |
| 1 | G1073 | BI701170 | BI701170 sag55e11.y1 Gm-c1082 Glycine max cD... | 2 | 478 | 4.00E-43 | 1 |
| 1 | G1073 | AP004680 | AP004680 Oryza sativa chromosome 6 clone OSJ... | -1 | 266 | 5.50E-43 | 2 |
| 1 | G1073 | BH669958 | BH669958 BOMEJ12TF BO_2_3_KB Brassica olerac... | 1 | 478 | 5.70E-43 | 1 |
| 1 | G1073 | AW720668 | AW720668 LjNEST14h9rc Lotus japonicus nodule... | 1 | 467 | 5.50E-42 | 1 |
| 1 | G1073 | BH513264 | BH513264 BOHQC93TR BOHQ Brassica oleracea ge... | -3 | 466 | 5.50E-42 | 1 |
| 1 | G1073 | AW350603 | AW350603 GM210008B10B12 Gm-r1021 Glycine max... | -1 | 463 | 1.20E-41 | 1 |
| 1 | G1073 | BM779718 | BM779718 EST590294 KV2 Medicago truncatula c... | -3 | 461 | 1.90E-41 | 1 |
| 1 | G1073 | BI419871 | BI419871 LjNEST17a9r Lotus japonicus nodule ... | 2 | 461 | 3.60E-41 | 1 |
| 1 | G1073 | BH550963 | BH550963 BOHQE85TR BOHQ Brassica oleracea ge... | -3 | 458 | 3.80E-41 | 1 |
| 1 | G1073 | BG881387 | BG881387 sae81e03.y1 Gm-c1065 Glycine max cD... | 3 | 457 | 7.60E-41 | 1 |
| 1 | G1073 | BM526396 | BM526396 sal40g01.yl Gm-c1059 Glycine max cD... | 3 | 456 | 9.50E-41 | 1 |
| 1 | G1073 | AW560824 | AW560824 EST315872 DSIR Medicago truncatula ... | 2 | 455 | 1.20E-40 | 1 |
| 1 | G1073 | BE998262 | BE998262 EST429985 GVSN Medicago truncatula ... | 2 | 426 | 1.40E-37 | 1 |
| 1 | G1073 | BM523502 | BM523502 sam84f06.y2 Gm-c1087 Glycine max cD... | 1 | 425 | 1.70E-37 | 1 |
| 1 | G1073 | BG646893 | BG646893 EST508512 HOGA Medicago truncatula ... | 2 | 422 | 2.80E-37 | 1 |
| 1 | G1073 | BH419964 | BH419964 BOGQI28TF BOGQ Brassica oleracea ge... | -1 | 420 | 7.30E-37 | 1 |
| 1 | G1073 | AW774872 | AW774872 EST334023 KV3 Medicago truncatula c... | 3 | 413 | 4.60E-36 | 1 |
| 1 | G1073 | BH688757 | BH688757 BOMDL66TF BO_2_3_KB Brassica olerac ... | 2 | 410 | 6.00E-36 | 1 |
| 1 | G1073 | AW780525 | AW780525 sl72a07.y1 Gm-c1027 Glycine max cDN... | 1 | 410 | 1.10E-35 | 1 |
| 1 | G1073 | AV422634 | AV422634 AV422634 Lotus japonicus young plan... | 3 | 406 | 2.20E-35 | 1 |
| 1 | G1073 | AP003938 | AP003938 Oryza sativa chromosome 6 clone OJ1... | -2 | 405 | 1.30E-34 | 1 |
| 1 | G1073 | AP003683 | AP003683 Oryza sativa genomic DNA, chromosom... | 3 | 402 | 2.60E-34 | 1 |
| 1 | G1073 | BG647027 | BG647027 EST508646 HOGA Medicago truncatula ... | 3 | 393 | 2.90E-34 | 1 |
| 1 | G1073 | AW349908 | AW349908 GM210006A20F9 Gm-r1021 Glycine max ... | -3 | 392 | 4.90E-34 | 1 |
| 1 | G1073 | AW621455 | AW621455 EST312253 tomato root during/after ... | 2 | 389 | 9.20E-34 | 1 |
| 1 | G1073 | BG599840 | BG599840 EST504735 cSTS Solanum tuberosum cD... | 3 | 205 | 2.20E-33 | 2 |
| 1 | G1073 | AP004319 | AP004319 Oryza sativa chromosome 1 clone B10... | 1 | 217 | 6.30E-32 | 2 |
| 1 | G1073 | A1736668 | AI736668 sb32a03.y1 Gm-c1012 Glycine max cDN... | 2 | 370 | 1.10E-31 | 1 |
| 1 | G1073 | AI494847 | AI494847 sb06b09.y1 Gm-c1004 Glycine max cDN... | 1 | 368 | 2.30E-31 | 1 |
| 1 | G1073 | BI426899 | BI426899 sag08g12.y1 Gm-c1080 Glycine max cD... | 1 | 367 | 2.40E-31 | 1 |
| 1 | G1073 | BF650426 | BF650426 NF096G12EC1 F1098 Elicited cell cult... | 3 | 365 | 3.40E-31 | 1 |
| 1 | G1073 | BH430946 | BH430946 BOHSM94TF BOHS Brassica oleracea ge... | -1 | 362 | 6.80E-31 | 1 |
| 1 | G1073 | BF588135 | BF588135 FM1_38_B09.b1_A003 Floral-Induced M... | 3 | 354 | 6.10E-30 | 1 |
| 1 | G1073 | AW348874 | AW348874 GM210010A10G1 Gm-r1021 Glycine max ... | -2 | 352 | 7.20E-30 | 1 |
| 1 | G1073 | BM525692 | BM525692 sak62d03.y1 Gm-c1036 Glycine max cD... | 1 | 351 | 1.20E-29 | 1 |
| 1 | G1073 | BI970749 | BI970749 GM830011A20H08 Gm-r1083 Glycine max... | -1 | 350 | 1.20E-29 | 1 |
| 1 | G1073 | BH679922 | BH679922 BOMNO92TR BO_2_3_KB Brassica olerac... | 3 | 348 | 1.80E-29 | 1 |
| 1 | G1073 | BG644865 | BG644865 EST506484 KV3 Medicago truncatula c... | 2 | 347 | 2.30E-29 | 1 |
| 1 | G1073 | BE020549 | BE020549 sm45a11.y1 Gm-c1028 Glycine max cDN... | 3 | 349 | 3.30E-29 | 1 |
| 1 | G1073 | AW648634 | AW648634 EST327184 tomato germinating seedli... | 3 | 343 | 8.50E-29 | 1 |
| 1 | G1073 | BH491363 | BH491363 BOGPS71TR BOGP Brassica oleracea ge... | -1 | 338 | 2.10E-28 | 1 |
| 1 | G1073 | AV415346 | AV415346 AV415346 Lotus japonicus young plan... | 3 | 338 | 4.00E-28 | 1 |
| 1 | G1073 | BH173434 | BH173434 0104B13 maize genomic ORF library F... | -1 | 339 | 4.10E-28 | 1 |
| 1 | G1073 | BI308126 | BI308126 EST529536 GPOD Medicago truncatula ... | 3 | 336 | 4.30E-28 | 1 |
| 1 | G1073 | BG600318 | BG600318 EST505213 cSTS Solanum tuberosum cD... | 1 | 333 | 1.80E-27 | 1 |
| 1 | G1073 | BE496294 | BE496294 NXCI_022 C11_F NXCI (Nsf Xylem Comp... | 1 | 330 | 2.40E-27 | 1 |
| 1 | G1073 | BI699513 | BI699513 sag37g08.y1 Gm-c1081 Glycine max cD... | 2 | 320 | 2.70E-26 | 1 |
| 1 | G1073 | BH480897 | BH480897 BOGRA01TF BOGR Brassica oleracea ge... | -1 | 319 | 2.90E-26 | 1 |
| 1 | G1073 | AP004303 | AP004303 Oryza sativa chromosome 7 clone P04... | 2 | 323 | 6.20E-26 | 1 |
| 1 | G1073 | AI522924 | AI522924 sa92b03.y1 Gm-c1004 Glycine max cDN... | 2 | 312 | 2.10E-25 | 1 |
| 1 | G1073 | BH588550 | BH588550 BOHOI48TR BOHO Brassica oleracea ge... | -2 | 310 | 2.30E-25 | 1 |
| 1 | G1073 | AW596625 | AW596625 sj14f10.y1 Gm-c1032 Glycine max cDN... | 2 | 310 | 2.80E-25 | 1 |
| 1 | G1073 | B1971972 | B1971972 sag84f09.y1 Gm-c1084 Glycine max cD... | 1 | 310 | 2.90E-25 | 1 |
| 1 | G1073 | BE432188 | BE432188 EST398717 tomato breaker fruit, TIG... | 3 | 309 | 3.70E-25 | 1 |
| 1 | G1073 | BE445433 | BE445433 WHE1136_A09_B18ZS Wheat etiolated s... | 2 | 304 | 1.20E-24 | 1 |
| 1 | G1073 | BI924382 | BI924382 EST544271 tomato flower, buds 0-3 m... | 3 | 304 | 1.40E-24 | 1 |
| 1 | G1073 | AP003917 | AP003917 Oryza sativa chromosome 8 clone OJ1... | 3 | 224 | 2.80E-24 | 2 |
| 1 | G1073 | BE442709 | BE442709 WHE1105_B09_C17ZS Wheat etiolated s... | 3 | 299 | 4.00E-24 | 1 |
| 1 | G1073 | BG593477 | BG593477 EST492155 cSTS Solanum tuberosum cD... | 3 | 298 | 5.10E-24 | 1 |
| 1 | G1073 | BJ189956 | BJ189956 BJ189956 normalized full length cDN... | 3 | 297 | 5.80E-24 | 1 |
| 1 | G1073 | BH732637 | BH732637 BOMBI03TF BO_2_3_KB Brassica olerac... | 2 | 296 | 6.50E-24 | 1 |
| 1 | G1073 | BE342838 | BE342838 EST395682 potato stolon, Cornell Un... | 1 | 296 | 7.60E-24 | 1 |
| 1 | G1073 | BI786439 | BI786439 sai49b05.y1 Gm-c1065 Glycine max cD... | 1 | 295 | 1.10E-23 | 1 |
| 1 | G1073 | BM407572 | BM407572 EST581899 potato roots Solanum tube... | 1 | 219 | 1.70E-23 | 2 |
| 1 | G1073 | AW596434 | AW596434 sj12d05.y1 Gm-c1032 Glycine max cDN... | 2 | 293 | 2.50E-23 | 1 |
| 1 | G1073 | BE442615 | BE442615 WHE1101_G09_M17ZS Wheat etiolated s... | 2 | 289 | 4.70E-23 | 1 |
| 1 | G1073 | BG045781 | BG045781 saa06a11.y1 Gm-c1058 Glycine max cD... | 3 | 290 | 6.00E-23 | 1 |
| 1 | G1073 | AW776953 | AW776953 EST336018 DSIL Medicago truncatula ... | 2 | 279 | 4.90E-22 | 1 |
| 1 | G1073 | BM371214 | BM371214 EBro04_SQ003_M20_R IGF Barley EBro0... | 2 | 279 | 7.50E-22 | 1 |
| 1 | G1073 | BI972076 | BI972076 sag85g11.y1 Gm-c1084 Glycine max cD... | 2 | 277 | 8.50E-22 | 1 |
| 1 | G1073 | BI266587 | BI266587 NF099D091N1F1078 Insect herbivory M... | 2 | 271 | 3.20E-21 | 1 |
| 1 | G1073 | BI406306 | BI406306 159E11 Mature tuber lambda ZAP Sola... | 1 | 270 | 4.50E-21 | 1 |
| 1 | G1073 | AV422959 | AV422959 AV422959 Lotus japonicus young plan... | 2 | 268 | 9.40E-21 | 1 |
| 1 | G1073 | BI699401 | BI699401 sag36e12.y1 Gm-c1081 Glycine max cD... | -2 | 162 | 9.60E-21 | 2 |
| 1 | G1073 | BG465540 | BG465540 RHIZ2_45_G09.b1 A003 Rhizome2 (RHIZ... | 3 | 267 | 1.00E-20 | 1 |
| 1 | G1073 | AI522913 | AI522913 sa91h08.y1 Gm-c1004 Glycine max cDN... | 1 | 266 | 1.50E-20 | 1 |
| 1 | G1073 | AP003074 | AP003074 Oryza sativa genomic DNA, chromosom... | -1 | 272 | 1.60E-20 | 1 |
| 1 | G1073 | AC007789 | AC007789 Oryza sativa BAC OSJNBa0049B20 geno... | -1 | 272 | 1.60E-20 | 1 |
| 1 | G1073 | BI321153 | BI321153 saf48b01.y3 Gm-c1077 Glycine max cD... | 1 | 263 | 2.40E-20 | 1 |
| 1 | G1073 | BG596340 | BG596340 EST495018 cSTS Solanum tuberosum cD... | 3 | 262 | 2.80E-20 | 1 |
| 1 | G1073 | AW099294 | AW099294 sd37h01.y1 Gm-c1016 Glycine max cDN... | 2 | 264 | 4.10E-20 | 1 |
| 1 | G1073 | AV933535 | AV933535 AV933535 K. Sato unpublished CDNA I... | 1 | 257 | 9.80E-20 | 1 |
| 1 | G1073 | BF587149 | BF587149 FM1_32_G07.b1_A003 Floral-Induced M... | 3 | 258 | 1.10E-19 | 1 |
| 1 | G1073 | AW906401 | AW906401 EST342523 potato stolon, Cornell Un... | 2 | 256 | 1.60E-19 | 1 |
| 1 | G1073 | BI074657 | BI074657 IP1_14_F04.b1_A002 Immature pannicl... | 1 | 255 | 1.70E-19 | 1 |
| 1 | G1073 | AW672482 | AW672482 LG1_360_E08.b1_A002 Light Grown 1 (... | 3 | 255 | 1.80E-19 | 1 |
| 1 | G1073 | AL373204 | AL373204 MtBA56C11 F1 MtBA Medicago truncatul... | 3 | 253 | 3.60E-19 | 1 |
| 1 | G1073 | AV424890 | AV424890 AV424890 Lotus japonicus young plan... | 3 | 253 | 3.80E-19 | 1 |
| 1 | G1073 | BF324870 | BF324870 su17c07.y1 Gm-c1066 Glycine max cDN... | 1 | 252 | 4.50E-19 | 1 |
| 1 | G1073 | BM322182 | BM322182 PIC1_1_A05.b1_A002 Pathogen-infecte... | 2 | 250 | 5.60E-19 | 1 |
| 1 | G1073 | BM322317 | BM322317 PIC1_3_A06.b1_A002 Pathogen-infecte... | 2 | 250 | 5.60E-19 | 1 |
| 1 | G1073 | BM322320 | BM322320 PIC1_3_A09.b1_A002 Pathogen-infecte... | 2 | 250 | 5.90E-19 | 1 |
| 1 | G1073 | BE496149 | BE496149 WHE1262_H03_P06ZS Secale cereale an... | 2 | 250 | 7.50E-19 | 1 |
| 1 | G1073 | BG583795 | BG583795 EST485550 GVN Medicago truncatula c... | 1 | 248 | 7.50E-19 | 1 |
| 1 | G1073 | BI974724 | BI974724 sai72b06.y1 Gm-c1068 Glycine max cD... | 1 | 245 | 2.00E-18 | 1 |
| 1 | G1073 | BM322319 | BM322319 PIC1_3_A08.b1_A002 Pathogen-infecte... | 2 | 245 | 2.10E-18 | 1 |
| 1 | G1073 | PSDBPPD1 | X98738 P.sativum mRNA encoding DNA-binding p... | 1 | 248 | 2.50E-18 | 1 |
| 1 | G1073 | BM322185 | BM322185 PIC1_1_A08.b1_A002 Pathogen-infecte... | 2 | 245 | 2.60E-18 | 1 |
| 1 | G1073 | BM322192 | BM322192 PIC1_1_B08.b1_A002 Pathogen-infecte... | 2 | 245 | 2.60E-18 | 1 |
| 1 | G1073 | BM322318 | BM322318 PIC1_3_A07.b1_A002 Pathogen-infecte... | 2 | 245 | 2.60E-18 | 1 |
| 1 | G1073 | AI965992 | AI965992 sc25a12.y1 Gm-c1013 Glycine max cDN... | 3 | 244 | 2.60E-18 | 1 |
| 1 | G1073 | BH596271 | BH596271 BOGBL42TF BOGB Brassica oleracea ge... | -3 | 242 | 3.10E-18 | 1 |
| 1 | G1073 | AW309814 | AW309814 sf25b03.x1 Gm-c1028 Glycine max cDN... | -1 | 242 | 3.50E-18 | 1 |
| 1 | G1073 | AL372048 | AL372048 MtBA48C06F1 MtBA Medicago truncatul... | 1 | 244 | 4.00E-18 | 1 |
| 1 | G1073 | BM079070 | BM079070 MEST88-H11.T3 ISUM4-TN Zea mays cDN... | -1 | 241 | 4.20E-18 | 1 |
| 1 | G1073 | PSDBLPPD1 | X98739 P.sativum mRNA encoding DNA-binding P... | 1 | 244 | 4.40E-18 | 1 |
| 1 | G1073 | BG909764 | BG909764 TaLr1108d02R TaLr1 Triticum aestivu... | 3 | 242 | 4.70E-18 | 1 |
| 1 | G1073 | AW755604 | AW755604 sl05h07.y1 Gm-c1036 Glycine max cDN... | 3 | 241 | 5.30E-18 | 1 |
| 1 | G1073 | BF429428 | BF429428 WHE1803_A09_B17ZS Secale cereale an... | 1 | 242 | 5.60E-18 | 1 |
| 1 | G1073 | BE444180 | BE444180 WHE1128_B06_D12ZS Wheat etiolated s... | 2 | 241 | 6.20E-18 | 1 |
| 1 | G1073 | BG441060 | BG441060 GA_Ea0011119fGossypium arboreum 7... | 3 | 237 | 9.50E-18 | 1 |
| 1 | G1073 | BE587763 | BE587763 WHE0652_C06_E12ZM Secale cereale ro... | 1 | 239 | 1.00E-17 | 1 |
| 1 | G1073 | AW979681 | AW979681 EST341290 tomato root deficiency, C... | 1 | 236 | 1.80E-17 | 1 |
| 1 | G1073 | BE822274 | BE822274 GM700017A10A4 Gm-r1070 Glycine max ... | -1 | 233 | 2.80E-17 | 1 |
| 1 | G1073 | BI139442 | BI139442 F131P74Y Populus flower cDNA librar... | -1 | 233 | 4.10E-17 | 1 |
| 1 | G1073 | AMA132349 | AJ132349 Antirrhinum majus mRNA for SAP1 pro... | 3 | 232 | 4.60E-17 | 1 |
| 1 | G1073 | AW310124 | AW310124 sf31d10.x1 Gm-c1028 Glycine max cDN... | -1 | 231 | 5.60E-17 | 1 |
| 1 | G1073 | BM322184 | BM322184 PIC1_1 A07.b1_A002 Pathogen-infecte... | 2 | 233 | 5.80E-17 | 1 |
| 1 | G1073 | AW622329 | AW622329 EST313127 tomato root during/after ... | 2 | 231 | 7.20E-17 | 1 |
| 1 | G1073 | BE600816 | BE600816 PI1_90_E07.b1_A002 Pathogen induced... | 3 | 229 | 1.00E-16 | 1 |
| 1 | G1073 | BG350206 | BG350206 084D10 Mature tuber lambda ZAP Sola... | 3 | 227 | 1.30E-16 | 1 |
| 1 | G1073 | BE357932 | BE357932 DG1_23_D12.b1_A002 Dark Grown 1 (DG... | 1 | 226 | 2.10E-16 | 1 |
| 1 | G1073 | AV917319 | AV917319 AV917319 K. Sato unpublished CDNA I... | 2 | 225 | 2.40E-16 | 1 |
| 1 | G1073 | BF637181 | BF637181 NF050F02LF1F1016 Developing leaf Me... | 3 | 225 | 2.60E-16 | 1 |
| 1 | G1073 | AV411735 | AV411735 AV411735 Lotus japonicus young plan... | 1 | 226 | 2.80E-16 | 1 |
| 1 | G1073 | BE591277 | BE591277 WHE1655-1658_J17_J17ZS Wheat heat s... | 1 | 224 | 3.40E-16 | 1 |
| 1 | G1073 | BE125776 | BE125776 DG1_57_B07.b1_A002 Dark Grown 1 (DG... | 1 | 225 | 3.70E-16 | 1 |
| 1 | G1073 | BJ203193 | BJ203193 BJ203193 normalized full length cDN... | 1 | 224 | 3.80E-16 | 1 |
| 1 | G1073 | AW455702 | AW455702 707090A08.x1 707 - Mixed adult tiss... | 3 | 223 | 4.20E-16 | 1 |
| 1 | G1073 | BG241750 | BG241750 RHIZ2_50_G05.b1_A003 Rhizome2 (RHIZ... | 3 | 224 | 4.30E-16 | 1 |
| 1 | G1073 | BG589060 | BG589060 EST490869 MHRP- Medicago truncatula... | 1 | 222 | 4.40E-16 | 1 |
| 1 | G1073 | BI929403 | BI929403 EST549292 tomato flower, 3 - 8 mm b... | 1 | 220 | 6.30E-16 | 1 |
| 1 | G1073 | AW278127 | AW278127 sf40a09.y1 Gm-c1009 Glycine max cDN... | 1 | 222 | 6.60E-16 | 1 |
| 1 | G1073 | BI946361 | BI946361 01104 leafy spurge Lambda HybriZAP ... | 1 | 221 | 6.70E-16 | 1 |
| 1 | G1073 | BF625927 | BF625927 HVSMEa0014H11f Hordeum vulgare seed... | 1 | 221 | 8.40E-16 | 1 |
| 1 | G1073 | AI960613 | AI960613 sc86h10.y1 Gm-c1018 Glycine max cDN... | 1 | 221 | 8.60E-16 | 1 |
| 1 | G1073 | BE609898 | BE609898 sq46e12.y1 Gm-c1019 Glycine max cDN... | 3 | 219 | 1.20E-15 | 1 |
| 1 | G1073 | BE643892 | BE643892 NXCI_048_F12_F NXCI (Nsf Xylem Comp... | 3 | 218 | 2.00E-15 | 1 |
| 1 | G1073 | AL508367 | AL508367 AL508367 Hordeum vulgare Barke deve... | 3 | 214 | 3.50E-15 | 1 |
| 1 | G1073 | AW755831 | AW755831 sl09h02.y1 Gm-c1036 Glycine max cDN... | 3 | 214 | 5.50E-15 | 1 |
| 1 | G1073 | D42950 | D42950 D42950 Rice callus cDNA (H.Uchimiya) ... | 2 | 214 | 5.50E-15 | 1 |
| 1 | G1073 | BH677454 | BH677454 BOHZJ50TR BO_2_3_KB Brassica olerac... | -3 | 210 | 7.10E-15 | 1 |
| 1 | G1073 | BM094106 | BM094106 sah25e06.y1 Gm-c1036 Glycine max cD... | 1 | 212 | 7.20E-15 | 1 |
| 1 | G1073 | BM358644 | BM358644 GA_Ea0011119r Gossypium arboreum 7... | 3 | 211 | 7.40E-15 | 1 |
| 1 | G1073 | AW773741 | AW773741 EST332727 KV3 Medicago truncatula c... | 3 | 211 | 8.10E-15 | 1 |
| 1 | G1073 | BE807468 | BE807468 ss22h03.y1 Gm-c1047 Glycine max cDN... | 2 | 212 | 9.10E-15 | 1 |
| 1 | G1073 | BH650598 | BH650598 BOHZR58TR BO_2_3_KB Brassica olerac... | 1 | 210 | 9.60E-15 | 1 |
| 1 | G1073 | BM307331 | BM307331 sak27h05.y1 Gm-c1075 Glycine max cD... | 1 | 209 | 1.40E-14 | 1 |
| 1 | G1073 | BH510717 | BH510717 BOGGW07TF BOGG Brassica oleracea ge... | 1 | 208 | 1.50E-14 | 1 |
| 1 | G1073 | AW773742 | AW773742 EST332728 KV3 Medicago truncatula c... | 3 | 207 | 2.10E-14 | 1 |
| 1 | G1073 | BI267267 | BI267267 NF100C06IN1F1070 Insect herbivory M... | 2 | 137 | 2.20E-14 | 2 |
| 1 | G1073 | BE600623 | BE600623 PI1_89_D08.b1_A002 Pathogen induced... | 3 | 207 | 2.50E-14 | 1 |
| 1 | G1073 | AI881841 | AI881841 606074A03.y1 606-Ear tissue cDNA... | 3 | 206 | 2.60E-14 | 1 |
| 1 | G1073 | AW448258 | AW448258 BRY_1522 BRY Triticum aestivum cDNA... | 3 | 203 | 4.00E-14 | 1 |
| 1 | G1073 | BG132224 | BG132224 EST465116 tomato crown gall Lycoper... | 2 | 203 | 6.70E-14 | 1 |
| 1 | G1073 | BM086350 | BM086350 sah38h08.y1 Gm-c1036 Glycine max cD... | 3 | 202 | 7.40E-14 | 1 |
| 1 | G1073 | AI443215 | AI443215 sa45h07.y1 Gm-c1004 Glycine max cDN... | 1 | 203 | 8.20E-14 | 1 |
| 1 | G1073 | BE345655 | BE345655 946021H10.y2 946 - tassel primordiu... | 3 | 201 | 9.60E-14 | 1 |
| 1 | G1073 | BH544467 | BH544467 BOHKE60TF BOHK Brassica oleracea ge... | 1 | 199 | 1.10E-13 | 1 |
| 1 | G1073 | BH572435 | BH572435 BOHBY93TF BOHB Brassica oleracea ge... | 1 | 199 | 1.20E-13 | 1 |
| 1 | G1073 | BI096714 | BI096714 949019H11.y1 949 - Juvenile leaf an... | 2 | 200 | 1.30E-13 | 1 |
| 1 | G1073 | BH439306 | BH439306 BOHGL38TR BOHG Brassica oleracea ge... | 1 | 199 | 1.60E-13 | 1 |
| 1 | G1073 | BE202989 | BE202989 EST403011 KV1 Medicago truncatula c... | -3 | 199 | 1.60E-13 | 1 |
| 1 | G1073 | BE555817 | BE555817 sp94c01.y1 Gm-c1045 Glycine max cDN... | 3 | 199 | 1.70E-13 | 1 |
| 1 | G1073 | BG888793 | BG888793 EST514644 cSTD Solanum tuberosum cD... | 3 | 197 | 1.80E-13 | 1 |
| 1 | G1073 | BI788210 | BI788210 sag68a10.y1 Gm-c1082 Glycine max cD... | 3 | 196 | 3.10E-13 | 1 |
| 1 | G1073 | BE330349 | BE330349 so77e09.y1 Gm-c1040 Glycine max cDN... | 3 | 196 | 3.50E-13 | 1 |
| 1 | G1073 | BG352828 | BG352828 sab91g04.y1 Gm-c1040 Glycine max cD... | 3 | 196 | 3.60E-13 | 1 |
| 1 | G1073 | BG651836 | BG651836 sad61f05.y1 Gm-c1051 Glycine max cD... | 3 | 196 | 3.70E-13 | 1 |
| 1 | G1073 | BM094108 | BM094108 sah25e08.y1 Gm-c1036 Glycine max cD... | 1 | 195 | 4.70E-13 | 1 |
| 1 | G1073 | AW066510 | AW066510 660015F03.y1 660 - Mixed stages of ... | 1 | 194 | 6.00E-13 | 1 |
| 1 | G1073 | BG599554 | BG599554 EST504449 cSTS Solanum tuberosum cD... | 2 | 192 | 6.30E-13 | 1 |
| 1 | G1073 | AW928863 | AW928863 EST337651 tomato flower buds 8 mm t... | 3 | 193 | 6.90E-13 | 1 |
| 1 | G1073 | BM136003 | BM136003 WHE2604_D04_H08ZS Wheat Fusarium gr... | -1 | 191 | 1.00E-12 | 1 |
| 1 | G1073 | BM521838 | BM521838 sak76f11.y1 Gm-c1036 Glycine max cD... | 2 | 191 | 1.10E-12 | 1 |
| 1 | G1073 | BE586257 | BE586257 WHE501_B07_C13ZR Secale cereale alu... | 2 | 190 | 1.30E-12 | 1 |
| 1 | G1073 | BF257835 | BF257835 HVSMEf0014A01f Hordeum vulgare seed... | 2 | 190 | 1.40E-12 | 1 |
| 1 | G1073 | BJ158045 | BJ158045 BJ158045 full length cDNA library, ... | -3 | 190 | 1.40E-12 | 1 |
| 1 | G1073 | BG582457 | BG582457 EST484202 GVN Medicago truncatula c... | 3 | 190 | 2.10E-12 | 1 |
| 1 | G1073 | AI988292 | AI988292 sc98f12.y1 Gm-c1020 Glycine max cDN... | 2 | 188 | 2.20E-12 | 1 |
| 1 | G1073 | BG043009 | BG043009 st91a08.y1 Gm-c1054 Glycine max cDN... | 3 | 190 | 2.30E-12 | 1 |
| 1 | G1073 | AV410107 | AV410107 AV410107 Lotus japonicus young plan... | 2 | 189 | 2.80E-12 | 1 |
| 1 | G1073 | BG130143 | BG130143 EST475789 tomato shoot/meristem Lyc... | 1 | 186 | 3.00E-12 | 1 |
| 1 | G1073 | BF258208 | BF258208 HVSMEf0015B16f Hordeum vulgare seed... | 2 | 185 | 3.50E-12 | 1 |
| 1 | G1073 | BI435896 | BI435896 EST538657 P. infestans-challenged I... | 3 | 188 | 3.60E-12 | 1 |
| 1 | G1073 | BI700385 | BI700385 sag60b11.y1 Gm-c1082 Glycine max cD... | 2 | 187 | 3.70E-12 | 1 |
| 1 | G1073 | BF112609 | BF112609 EST440199 tomato breaker fruit Lyco... | 3 | 186 | 4.10E-12 | 1 |
| 1 | G1073 | BI469296 | BI469296 sai10a11.y1 Gm-c1053 Glycine max cD... | 3 | 186 | 4.50E-12 | 1 |
| 1 | G1073 | BJ186205 | BJ186205 BJ186205 normalized full length cDN... | 1 | 185 | 4.50E-12 | 1 |
| 1 | G1073 | BE805340 | BE805340 ss41d08.y1 Gm-c1061 Glycine max cDN... | 1 | 186 | 5.30E-12 | 1 |
| 1 | G1073 | BG240061 | BG240061 OV1_17_D10.b1 A002 Ovary 1 (OV1) So... | 1 | 180 | 1.60E-11 | 1 |
| 1 | G1073 | BG240043 | BG240043 OV1_17_B12.b1_A002 Ovary 1 (OV1) So... | 1 | 180 | 1.70E-11 | 1 |
| 1 | G1073 | BM427899 | BM427899 NXRV_006_A04_FNXRV (Nsf Xylem Root... | 1 | 180 | 1.80E-11 | 1 |
| 1 | G1073 | BG240220 | BG240220 OV1_19_D02.b1_A002 Ovary 1 (OV1) So... | 1 | 180 | 2.10E-11 | 1 |
| 1 | G1073 | BG662369 | BG662369 Ljirnpest43-456-c2 Ljirnp Lambda Hy... | 1 | 184 | 2.10E-11 | 1 |
| 1 | G1073 | AW132605 | AW132605 se06d10.y1 Gm-c1013 Glycine max cDN... | 3 | 178 | 2.80E-11 | 1 |
| 1 | G1073 | BI932139 | BI932139 EST552028 tomato flower, 8 mm to pr... | 1 | 182 | 3.10E-11 | 1 |
| 1 | G1073 | HVU234403 | AJ234403 Hordeum vulgare partial mRNA; clone... | 1 | 175 | 5.10E-11 | 1 |
| 1 | G1073 | AW720136 | AW720136 LjNEST14h9r Lotus japonicus nodule... | 1 | 175 | 7.60E-11 | 1 |
| 1 | G1073 | BG594217 | BG594217 EST492895 cSTS Solanum tuberosum cD... | 2 | 173 | 8.60E-11 | 1 |
| 1 | G1073 | BI972355 | BI972355 sag90d06.y1 Gm-c1084 Glycine max cD... | 2 | 173 | 9.90E-11 | 1 |
| 1 | G1073 | BM309583 | BM309583 sak64c09.y1 Gm-c1036 Glycine max cD... | 1 | 170 | 2.10E-10 | 1 |
| 1 | G1073 | BE639390 | BE639390 946024E11.y1 946 -tassel primordiu... | 1 | 169 | 2.60E-10 | 1 |
| 1 | G1073 | BM412174 | BM412174 EST586501 tomato breaker fruit Lyco... | 1 | 163 | 1.30E-09 | 1 |
| 1 | G1073 | BF636643 | BF636643 NF091G07DT1F1055 DroughtMedicagot... | 1 | 164 | 1.30E-09 | 1 |
| 1 | G1073 | AW394835 | AW394835 sh36a01.y1 Gm-c1017 Glycine max cDN... | 2 | 162 | 2.10E-09 | 1 |
| 1 | G1073 | A1507693 | A1507693 sb10d09.y1 Gm-c1004 Glycine max cDN... | 2 | 161 | 2.70E-09 | 1 |
| 1 | G1073 | AW760534 | AW760534 sl51e09.y1 Gm-c1027 Glycine max cDN... | 1 | 157 | 7.30E-09 | 1 |
| 1 | G1073 | AV411140 | AV411140 AV411140 Lotus japonicus young plan... | 2 | 158 | 7.50E-09 | 1 |
| 1 | G1073 | AW223229 | AW223229 EST300040 tomato fruit red ripe, TA... | 1 | 156 | 9.10E-09 | 1 |
| 1 | G1073 | BG592977 | BG592977 EST491655 cSTS Solanum tuberosum cD... | -2 | 155 | 1.20E-08 | 1 |
| 1 | G1073 | AI897688 | AI897688 EST267131 tomato ovary, TAMU Lycope... | 3 | 155 | 1.40E-08 | 1 |
| 1 | G1073 | BE923621 | BE923621 EST427390 potato leaves and petiole... | 1 | 155 | 1.50E-08 | 1 |
| 1 | G1073 | BM524672 | BM524672 sal18d07.y1 Gm-c1059 Glycine max cD... | 2 | 154 | 1.70E-08 | 1 |
| 1 | G1073 | BM524654 | BM524654 sal18b12.y1 Gm-c1059 Glycine max cD... | 2 | 154 | 1.80E-08 | 1 |
| 1 | G1073 | BF187248 | BF187248 EST443535 potato stolon, Cornell Un... | 2 | 154 | 1.90E-08 | 1 |
| 1 | G1073 | BE996884 | BE996884 NXCl_103_F12_F NXCI (Nsf Xylem Comp... | 1 | 155 | 2.20E-08 | 1 |
| 1 | G1073 | BE587194 | BE587194 WHE0519_B07_D13ZR Secale cereale al... | 1 | 155 | 2.30E-08 | 1 |
| 1 | G1073 | D15459 | D15459 RICC0669A Rice callus Oryza sativa cD... | 1 | 154 | 2.70E-08 | 1 |
| 1 | G1073 | AW776126 | AW776126 EST335191 DSIL Medicago truncatula ... | 2 | 163 | 3.00E-08 | 1 |
| 1 | G1073 | AW225998 | AW225998 ST76B07 Pine Tripe shoot tip libr... | 2 | 152 | 3.30E-08 | 1 |
| 1 | G1073 | BE446692 | BE446692 WHE1139_D09_H17ZS Wheat etiolated s... | 2 | 152 | 3.70E-08 | 1 |
| 1 | G1073 | BM341562 | BM341562 MEST336-F03.T3 ISUM5-RN Zea mays cD... | -1 | 151 | 3.80E-08 | 1 |
| 1 | G1073 | AF474373 | AF474373 Hordeum vulgare BAC 259116, complet... | 2 | 170 | 4.00E-08 | 1 |
| 1 | G1073 | BJ196157 | BJ196157 BJ196157 normalized full length cDN... | 1 | 153 | 4.20E-08 | 1 |
| 1 | G1073 | AV423934 | AV423934 AV423934 Lotus japonicus young plan... | 3 | 151 | 5.20E-08 | 1 |
| 1 | G1073 | BG604965 | BG604965 WHE2325_E05_l09ZS Wheat pre-anthesi... | 3 | 150 | 5.90E-08 | 1 |
| 1 | G1073 | AU198263 | AU198263 AU198263 Rice green shoot Oryza sat... | 1 | 150 | 5.90E-08 | 1 |
| 1 | G1073 | BG582452 | BG582452 EST484197 GVN Medicago truncatula c... | 3 | 161 | 6.40E-08 | 1 |
| 1 | G1073 | BM405827 | BM405827 EST580154 potato roots Solanum tube... | 2 | 150 | 6.50E-08 | 1 |
| 1 | G1073 | AI487942 | AI487942 EST246264 tomato ovary, TAMU Lycope... | 1 | 151 | 7.90E-08 | 1 |
| 1 | G1073 | BI931107 | BI931107 EST550996 tomato flower, 8 mm to pr... | 1 | 154 | 1.20E-07 | 1 |
| 1 | G1073 | BE923864 | BE923864 EST427633 potato leaves and petiole... | 1 | 147 | 1.40E-07 | 1 |
| 1 | G1073 | AW620872 | AW620872 sj47g06.y1 Gm-c1033 Glycine max cDN... | 3 | 147 | 1.40E-07 | 1 |
| 1 | G1073 | AW647786 | AW647786 EST326240 tomato germinating seedli... | 3 | 145 | 1.90E-07 | 1 |
| 1 | G1073 | BG098673 | BG098673 EST463192 sprouting eyes/shoots Sol... | 2 | 146 | 2.90E-07 | 1 |
| 1 | G1073 | AW761430 | AW761430 s167d09.y1 Gm-c1027 Glycine max cDN... | 2 | 144 | 3.10E-07 | 1 |
| 1 | G1073 | AV421337 | AV421337 AV421337 Lotus japonicus young plan... | 2 | 145 | 3.60E-07 | 1 |
| 1 | G1073 | BF639276 | BF639276 NF011F111N1F1092 Insect herbivory M... | 2 | 151 | 5.00E-07 | 1 |
| 1 | G1073 | AV425818 | AV425818 AV425818 Lotus japonicus young plan... | 3 | 142 | 5.40E-07 | 1 |
| 1 | G1073 | AI897113 | AI897113 EST266556 tomato ovary, TAMU Lycope... | 2 | 141 | 5.80E-07 | 1 |
| 1 | G1073 | BI928278 | BI928278 EST548167 tomato flower, 3 - 8 mm b... | 1 | 153 | 7.50E-07 | 1 |
| 1 | G1073 | BI929489 | BI929489 EST549378 tomato flower, 3 - 8 mm b... | 1 | 153 | 8.50E-07 | 1 |
| 1 | G1073 | BG601044 | BG601044 EST505939 cSTS Solanum tuberosum cD... | 1 | 141 | 9.80E-07 | 1 |
| 1 | G1073 | BG040551 | BG040551 NXSI_112_D08_F NXSI (Nsf Xylem Side... | 1 | 139 | 1.10E-06 | 1 |
| 1 | G1073 | BI927526 | BI927526 EST547415 tomato flower, 3 - 8 mm b... | 3 | 148 | 1.90E-06 | 1 |
| 1 | G1073 | BI788421 | BI788421 sag70e10.y1 Gm-c1082 Glycine max cD... | 3 | 135 | 3.00E-06 | 1 |
| 1 | G1073 | AW761107 | AW761107 sl63c09.y1 Gm-c1027 Glycine max cDN... | 1 | 135 | 3.90E-06 | 1 |
| 1 | G1073 | BG643949 | BG643949 EST512143 tomato shoot/meristem Lyc... | 2 | 146 | 5.10E-06 | 1 |
| 1 | G1073 | BG239709 | BG239709 sab74e11.y1 Gm-c1032 Glycine max cD... | 3 | 139 | 5.70E-06 | 1 |
| 1 | G1073 | BG149109 | BG149109 48 LIN01 Lupinus luteus CDNA, mRNA ... | 3 | 129 | 1.50E-05 | 1 |
| 1 | G1073 | BI308494 | BI308494 EST529904 GPOD Medicago truncatula ... | 2 | 143 | 1.60E-05 | 1 |
| 1 | G1073 | BG645337 | BG645337 EST506956 KV3 Medicago truncatula c... | 3 | 143 | 1.60E-05 | 1 |
| 1 | G1073 | AW031261 | AW031261 EST274636 tomato callus, TAMU Lycop... | 1 | 139 | 1.70E-05 | 1 |
| 1 | G1073 | AW164140 | AW164140 Ljirnpest20-626-g4 Ljimp Lambda Hy... | 1 | 128 | 2.00E-05 | 1 |
| 1 | G1073 | AL383224 | AL383224 MtBC12G03F1 MtBC Medicago truncatul... | 2 | 129 | 2.10E-05 | 1 |
| 1 | G1073 | BG275315 | BG275315 NXSI_142_D09_F NXSI (Nsf Xylem Side... | 3 | 129 | 2.30E-05 | 1 |
| 1 | G1073 | BH594393 | BH594393 BOGKJ24TR BOGK Brassica oleracea ge... | -2 | 137 | 4.90E-05 | 1 |
| 1 | G1073 | D43486 | D43486 D43486 Rice callus CDNA (H.Uchimiya) ... | 3 | 125 | 7.80E-05 | 1 |
| 1 | G1073 | BM299690 | BM299690 MCR053G12_23908 Ice plant Lambda Un... | 2 | 134 | 9.80E-05 | 1 |
| 1 | G1073 | BG580062 | BG580062 EST481784 GVN Medicago truncatula c... | 2 | 129 | 0.00022 | 1 |
| 1 | G1073 | BI943944 | BI943944 sa62b04.y1 Gm-c1004 Glycine max cDN... | 2 | 118 | 0.00026 | 1 |
| 1 | G1073 | AC060755 | AC060755 Oryza sativa chromosome 10 BAC OSJN... | -2 | 108 | 0.00028 | 2 |
| 1 | G1073 | AP004563 | AP004563 Oryza sativa chromosome 8 clone P04... | -3 | 107 | 0.00037 | 2 |
| 1 | G1073 | BM112694 | BM112694 EST560230 potato roots Solanum tube... | 1 | 130 | 0.00046 | 1 |
| 1 | G1073 | AP004765 | AP004765 Oryza sativa chromosome 8 clone P07... | -3 | 107 | 0.00049 | 2 |
| 1 | G1073 | BH467892 | BH467892 BOGWZ38TF BOGW Brassica oleracea ge... | -3 | 115 | 0.00055 | 1 |
| 1 | G1073 | AI779587 | AI779587 EST260466 tomato susceptible, Come... | 1 | 126 | 0.00064 | 1 |
| 1 | G1073 | BG856170 | BG856170 1024044H01.y1 C. reinhardtii CC-169... | -2 | 128 | 0.00069 | 1 |
| 1 | G1073 | BG418060 | BG418060 HVSMEk0021C01f Hordeum vulgare test... | 3 | 129 | 0.00078 | 1 |
| 1 | G1073 | BE611882 | BE611882 sr01b07.y1 Gm-c1049 Glycine max cDN... | 1 | 111 | 0.0015 | 1 |
| 1 | G1073 | BM308752 | BM308752 sak50a05.y1 Gm-c1036 Glycine max cD... | 1 | 121 | 0.0018 | 1 |
| 1 | G1073 | AI691406 | AI691406 606015E08.x1 606 - Ear tissue cDNA ... | -1 | 123 | 0.0021 | 1 |
| 1 | G1073 | AW698129 | AW698129 NXNV_073_B01_F Nsf Xylem Normal woo... | 1 | 111 | 0.0027 | 1 |
| 1 | G1073 | BH586480 | BH586480 BOGPF83TR BOGP Brassica oleracea ge... | -1 | 123 | 0.004 | 1 |
| 1 | G1073 | BH423260 | BH423260 BOGGZ42TF BOGG Brassica oleracea ge... | -2 | 120 | 0.0053 | 1 |
| 1 | G1073 | BH571929 | BH571929 BOGTJ45TF BOGT Brassica oleracea ge... | -1 | 121 | 0.0062 | 1 |
| 1 | G1073 | AW216305 | AW216305 687047G05.x1 687 - Early embryo fro... | -2 | 117 | 0.0083 | 1 |
| 1 | G1073 | AI939164 | AI939164 sc67f03.y1 Gm-c1016 Glycine max cDN... | 1 | 104 | 0.0099 | 1 |
| 1 | G1073 | BE454419 | BE454419 HVSMEh0094G09f Hordeum vulgare 5-45... | 1 | 119 | 0.013 | 1 |
| 1 | G1073 | BM112790 | BM112790 EST560326 potato roots Solanum tube... | 3 | 114 | 0.015 | 1 |
| 1 | G1073 | BG049445 | BG049445 OV1 20_A09.g1 A002 Ovary 1 (OV1) So... | 1 | 114 | 0.016 | 1 |
| 1 | G1073 | AP003925 | AP003925 Oryza sativa chromosome 8 clone OJ1... | -2 | 121 | 0.017 | 1 |
| 1 | G1073 | AP004562 | AP004562 Oryza sativa chromosome 8 clone P04... | -1 | 121 | 0.017 | 1 |
| 1 | G1073 | AW720219 | AW720219 LjNEST17b3r Lotus japonicus nodule ... | 1 | 113 | 0.021 | 1 |
| 1 | G1073 | BH218181 | BH218181 1006077H06.x1 1006 - RescueMu Grid ... | 1 | 106 | 0.026 | 1 |
| 1 | G1073 | BG647551 | BG647551 EST509170 HOGA Medicago truncatula ... | 2 | 112 | 0.027 | 1 |
| 1 | G1073 | BM078207 | BM078207 MEST116-E03.T3 ISUM4-TN Zea mays cD... | -3 | 114 | 0.027 | 1 |
| 1 | G1073 | AW929149 | AW929149 EST337937 tomato flower buds 8 mm t... | 2 | 114 | 0.03 | 1 |
| 1 | G1073 | BI310579 | BI310579 EST5312329 GESD Medicago truncatula... | 3 | 113 | 0.03 | 1 |
| 1 | G1073 | AW506972 | AW506972 660056A02.x1 660 - Mixed stages of ... | -1 | 103 | 0.033 | 1 |
| 1 | G1073 | BI643921 | BI643921 NXPV_129_D06_F NXPV (Nsf Xylem Plan... | 1 | 101 | 0.038 | 1 |
| 1 | G1073 | AI441147 | AI441147 sa59d03.y1 Gm-c1004 Glycine max cDN... | 3 | 98 | 0.046 | 1 |
| 1 | G1073 | AQ577522 | AQ577522 nbxb0091 M13r CUGI Rice BAC Library ... | -1 | 111 | 0.054 | 1 |
| 1 | G1073 | BH723529 | BH723529 BOMET92TR BO_2_3_KB Brassica olerac... | 2 | 110 | 0.085 | 1 |
| 1 | G1073 | AL375620 | AL375620 MtBB15H05F1 MtBB Medicago truncatul... | 1 | 106 | 0.087 | 1 |
| 1 | G1073 | BF274574 | BF274574 GA_Eb0021 G20f Gossypium arboreum 7... | 3 | 108 | 0.095 | 1 |
| 1 | G1073 | BF177556 | BF177556 Ljirnpest31-487-f3 Ljimp Lambda Hy... | 3 | 95 | 0.12 | 1 |
| 1 | G1073 | AU067947 | AU067947 AU067947 Rice callus Oryza sativa c... | -3 | 94 | 0.14 | 1 |
| 1 | G1073 | BM368289 | BM368289 EBed01 SQ002_L11_R IGF Barley EBed0... | 1 | 93 | 0.17 | 1 |
| 1 | G1073 | AP004116 | AP004116 Oryza sativa chromosome 2 clone OJ1... | 1 | 111 | 0.2 | 1 |
| 1 | G1073 | BI595695 | BI595695 949022E11.x1 949 - Juvenile leaf an... | -2 | 91 | 0.26 | 1 |
| 1 | G1073 | BM417802 | BM417802 952005G09.x11 952 - BMS tissue from... | 1 | 91 | 0.28 | 1 |
| 1 | G1073 | BG649667 | BG649667 RHIZ2_82_C04.b1_A003 Rhizome2 (RHIZ... | 1 | 103 | 0.29 | 1 |
| 1 | G1073 | AC108762 | AC108762 Oryza sativa chromosome 9 clone OSJ... | -3 | 109 | 0.3 | 1 |
| 1 | G1073 | BH698099 | BH698099 BOMHI48TR BO_2_3_KB Brassica olerac... | -2 | 106 | 0.31 | 1 |
| 1 | G1073 | BI642324 | BI642324 TZS924 TZS (Sapwood-heartwood trans... | -3 | 103 | 0.37 | 1 |
| 1 | G1073 | AW455681 | AW455681 707089C04.x1 707 - Mixed adult tiss... | 2 | 89 | 0.4 | 1 |
| 1 | G1073 | BE601230 | BE601230 PI1_90_E07.g1_A002 Pathogen induced... | 1 | 103 | 0.42 | 1 |
| 1 | G1073 | BE509974 | BE509974 946069E12.y1 946 - tassel primordiu... | 3 | 89 | 0.44 | 1 |
| 1 | G1073 | BH707416 | BH707416 BOHTM48TR BO_2_3_KB Brassica olerac... | 1 | 104 | 0.44 | 1 |
| 1 | G1073 | BM136703 | BM136703WHE0476_C06_E12ZS Wheat Fusarium gr... | 3 | 88 | 0.46 | 1 |
| 1 | G1073 | BM093021 | BM093021 saj04a05.y1 Gm-c1065 Glycine max cD... | 3 | 100 | 0.48 | 1 |
| 1 | G1073 | BF274040 | BF274040 GA_Eb0019P06f Gossypium arboreum 7... | 3 | 103 | 0.49 | 1 |
| 1 | G1073 | BM404166 | BM404166 EST578493 potato roots Solanum tube... | 3 | 101 | 0.5 | 1 |
| 1 | G1073 | BH550347 | BH550347 BOGPS25TR BOGP Brassica oleracea ge... | 1 | 103 | 0.5 | 1 |
| 1 | G1073 | BF587998 | BF587998 FM1_35_D12.g1_A003 Floral-Induced M... | 2 | 101 | 0.51 | 1 |
| 1 | G1073 | AU097090 | AU097090 AU097090 Rice shoot Oryza sativa cD... | 2 | 100 | 0.54 | 1 |
| 1 | G1073 | BE643053 | BE643053 Cri2_7_M09_SP6 Ceratopteris Spore L... | -3 | 103 | 0.56 | 1 |
| 1 | G1073 | BG051900 | BG051900 RHIZ2_6_C11.g1_A003 Rhizome2 (RHIZ2... | 1 | 101 | 0.56 | 1 |
| 1 | G1073 | BE455222 | BE455222 HVSMEh0096K08f Hordeum vulgare 5-45... | -3 | 104 | 0.56 | 1 |
| 1 | G1073 | BM377857 | BM377857 EBem04_SQ004_G09_R IGF Barley EBem0... | 2 | 97 | 0.56 | 1 |
| 1 | G1073 | AU181026 | AU181026 AU181026 Rice green shoot Oryza sat... | 3 | 87 | 0.63 | 1 |
| 1 | G1073 | AU197357 | AU197357 AU197357 Rice panicle (between 3cm ... | -3 | 90 | 0.76 | 1 |
| 1 | G1073 | AI857154 | AI857154 603007B08.x1 603 - stressed root cD... | 1 | 99 | 0.76 | 1 |
| 1 | G1073 | AQ912903 | AQ912903 nbeb0038P12r CUGI Rice BAC Library ... | 2 | 92 | 0.77 | 1 |
| 1 | G1073 | BG859658 | BG859658 1024064G02.y1 C. reinhardtii CC-169... | 1 | 100 | 0.77 | 1 |
| 1 | G1073 | BI642507 | BI642507 TZS128 TZS (Sapwood-heartwood trans... | 1 | 100 | 0.8 | 1 |
| 1 | G1073 | BM377499 | BM377499 EBem04_SQ003_F04_R IGF Barley EBem0... | 2 | 95 | 0.81 | 1 |
| 1 | G1073 | Arc091776 | AC091776 Chlamydomonas reinhardtii clone cr-... | 2 | 103 | 0.81 | 1 |
| 1 | G1073 | CNS07EFP | AL513405 Oryza sativa chromosome 12 clone OS... | 3 | 103 | 0.81 | 1 |
| 1 | G1073 | AC087726 | AC087726 Chlamydomonas reinhardtii clone cr-... | -2 | 103 | 0.81 | 1 |
| 1 | G1073 | BF256498 | BF256498 HVSMEf0010C11f Hordeum vulgare seed... | -2 | 101 | 0.85 | 1 |
| 1 | G1073 | AI441317 | AI441317 sa55c06.y1 Gm-c1004 Glycine max cDN... | 3 | 84 | 0.86 | 1 |
| 1 | G1073 | BM377468 | BM377468 EBem04_SQ003_D16_R IGF Barley EBem0... | 3 | 93 | 0.86 | 1 |
| 1 | G1073 | AZ132443 | AZ132443 OSJNBb0065J03r CUGI Rice BAC Librar... | 1 | 99 | 0.87 | 1 |
| 1 | G1073 | AU181516 | AU181516 AU181516 Rice callus (2001) Oryza s... | -2 | 94 | 0.89 | 1 |
| 1 | G1073 | BI722855 | BI722855 1031064E12.x1 C. reinhardtii CC-169... | 3 | 84 | 0.9 | 1 |
| 1 | G1073 | BF256757 | BF256757 HVSMEf0010O19f Hordeum vulgare seed... | -1 | 100 | 0.9 | 1 |
| 1 | G1073 | B1779670 | BI779670 EBem05_SQ001_A06_R IGF Barley EBem0... | 2 | 83 | 0.91 | 1 |
| 1 | G1073 | BF256541 | BF256541 HVSMEf0010E13f Hordeum vulgare seed... | -2 | 100 | 0.92 | 1 |
| 1 | G1073 | AU166731 | AU166731 AU166731 Rice callus (2001) Oryza s... | -1 | 93 | 0.92 | 1 |
| 1 | G1073 | AU197773 | AU197773 AU197773 Rice mature leaf Oryza sat... | 2 | 83 | 0.93 | 1 |
| 1 | G1073 | BI478832 | BI478832 952002B08.x10 952 - BMS tissue from... | 2 | 91 | 0.94 | 1 |
| 1 | G1073 | C19731 | C19731 C19731 Rice panicle at ripening stage... | 3 | 83 | 0.95 | 1 |
| 1 | G1073 | AU181350 | AU181350 AU181350 Rice callus (2001) Oryza s... | -1 | 93 | 0.95 | 1 |
| 1 | G1073 | BI779665 | BI779665 EBem05_SQ001_A01_R IGF Barley EBem0... | 3 | 83 | 0.96 | 1 |
| 1 | G1073 | BE421615 | BE421615 HWM011cH.01r ITEC HWM Barley Leaf L... | -3 | 99 | 0.96 | 1 |
| 1 | G1073 | BM779307 | BM779307 EST589882 KV2 Medicago truncatula c... | -1 | 94 | 0.97 | 1 |
| 1 | G1073 | AZ046505 | AZ046505 nbeb0092105f CUGI Rice BAC Library ... | -3 | 91 | 0.97 | 1 |
| 1 | G1073 | BI359024 | BI359024 949054G08.x2 949 - Juvenile leaf an... | -3 | 94 | 0.97 | 1 |
| 1 | G1073 | BE577148 | BE577148 L0-1863T3 Ice plant Lambda Uni-Zap ... | -2 | 94 | 0.98 | 1 |
| 1 | G1073 | BG343934 | BG343934 HVSMEg0007C15f Hordeum vulgare pre-... | 2 | 97 | 0.98 | 1 |
| 1 | G1073 | BI974612 | BI974612 sai70g08.y1 Gm-c1068 Glycine max cD... | 3 | 95 | 0.98 | 1 |
| 1 | G1073 | BI948976 | BI948976 HVSME10011010f Hordeum vulgare spik... | 1 | 98 | 0.98 | 1 |
| 1 | G1073 | BM417696 | BM417696 952005B04.x10 952 - BMS tissue from... | 1 | 91 | 0.98 | 1 |
| 1 | G1073 | BH740357 | BH740357 qj75a08.b1 TO1000 Brassica oleracea... | -3 | 89 | 0.98 | 1 |
| 1 | G1073 | BE025350 | BE025350 945025C05.Y1 945 - Mixed adult tiss... | -1 | 94 | 0.99 | 1 |
| 1 | G1073 | AQ689540 | AQ689540 nbxb0079H20f CUGI Rice BAC Library ... | 3 | 93 | 0.99 | 1 |
| 1 | G1073 | BE405965 | BE405965 WHE0402_c07_c07zB Wheat etiolated s... | 2 | 90 | 0.991 | 1 |
| 1 | G1073. | BE512087 | BE512087 946066G03.x1 946 -tassel primordiu... | -1 | 88 | 0.991 | 1 |
| 1 | G1073 | BH716346 | BH716346 BOMLG50TR BO_2_3_KB Brassica olerac... | 3 | 82 | 0.993 | 1 |
| 1 | G1073 | BG442856 | BG442856 GA_Ea0018I24f Gossypium arboreum 7... | -2 | 95 | 0.995 | 1 |
| 1 | G1073 | AI855566 | AI855566 sc20h05.y1 Gm-c1013 Glycine max cDN... | 2 | 91 | 0.995 | 1 |
| 1 | G1073 | BF256624 | BF256624 HVSMEf0010113f Hordeum vulgare seed... | -2 | 97 | 0.995 | 1 |
| 1 | G1073 | BG361509 | BG361509 gb60d11.y1 Moss EST library PPG Phy... | -1 | 87 | 0.996 | 1 |
| 1 | G1073 | BM417834 | BM417834 952005B04.x9 952 - BMS tissue from ... | 2 | 87 | 0.996 | 1 |
| 1 | G1073 | BH739760 | BH739760 gf19c08.b2 TO1000 Brassica oleracea... | -3 | 82 | 0.997 | 1 |
| 1 | G1073 | AQ867623 | AQ867623 nbeb0032F15f CUGI Rice BAC Library ... | 1 | 94 | 0.998 | 1 |
| 1 | G1073 | BF257444 | BF257444 HVSMEf0012O23f Hordeum vulgare seed... | -1 | 96 | 0.9992 | 1 |
| 1 | G1073 | DCU47097 | U47097 Daucus carota glycine-rich protein mR... | 2 | 88 | 0.9994 | 1 |
| 1 | G1073 | AV933536 | AV933536 AV933536 K. Sato unpublished CDNA I ... | 2 | 82 | 0.9994 | 1 |
| 1 | G1073 | BM737310 | BM737310 952055A09.x1 952 - BMS tissue from ... | 2 | 91 | 0.9996 | 1 |
| 1 | G1073 | BG309092 | BG309092 HVSMEc0002B12f Hordeum vulgare seed... | 1 | 94 | 0.9997 | 1 |
| 1 | G1073 | BE036544 | BE036544 MP01B10 MP Mesembryanthemum crystal... | -1 | 95 | 0.9997 | 1 |
| 1 | G1073 | BE455291 | BE455291 HVSMEh0096O24f Hordeum vulgare 5-45... | -2 | 95 | 0.9997 | 1 |
| 1 | G1073 | BF256580 | BF256580 HVSMEf0010G11f Hordeum vulgare seed... | 3 | 95 | 0.9997 | 1 |
| 1 | G1073 | C19642 | C19642 C19642 Rice panicle at ripening stage... | 1 | 90 | 0.9997 | 1 |
| 1 | G1073 | AI460865 | AI460865 sa70c03.y1 Gm-c1004 Glycine max cDN... | 3 | 90 | 0.9997 | 1 |
| 1 | G1073 | AQ257581 | AQ257581 nbxb0018B13r CUGI Rice BAC Library ... | -1 | 94 | 0.9998 | 1 |
| 1 | G1073 | BE455156 | BE455156 HVSMEh0096G12f Hordeum vulgare 5-45... | -1 | 95 | 0.9998 | 1 |
| 1 | G1073 | AW457868 | AW457868 sh88c12.y1 Gm-c1016 Glycine max cDN... | 1 | 87 | 0.9999 | 1 |
| 1 | G1073 | BE455227 | BE455227 HVSMEh0096K18f Hordeum vulgare 5-45... | -2 | 95 | 0.9999 | 1 |
| 1 | G1073 | BI952821 | BI952821 HVSMEm0007N22f Hordeum vulgare gree... | 1 | 94 | 0.9999 | 1 |
| 1 | G1073 | BI95785 | BI957854 HVSMEn0011 M04f Hordeum vulgare rach... | -2 | 94 | 0.9999 | 1 |
| 1 | G1073 | AP004359 | AP004359 Oryza sativa chromosome 1 clone B11... | -3 | 96 | 0.99994 | 1 |
| 1 | G1073 | AP003431 | AP003431 Oryza sativa chromosome 1 clone B10... | 1 | 96 | 0.99994 | 1 |
| 1 | G1073 | AP004813 | AP004813 Oryza sativa (japonica cultivar-gro... | 1 | 96 | 0.99995 | 1 |
| 1 | G1073 | GI-15528814 | hypothetical protein-similar to Arabidopsi... | -1 | 402 | 2.60E-37 | 1 |
| 1 | G1073 | GI-12643044 | AC060755_3 putative AT-Hook DNA-binding pr... | -1 | 278 | 1.20E-27 | 2 |
| 1 | G1073 | GI-5042445 | AC007789_8 hypothetical protein | -1 | 271 | 4.80E-24 | 2 |
| 1 | G1073 | GI-2213536 | DNA-binding protein PD1 | -1 | 248 | 5.50E-21 | 1 |
| 1 | G1073 | GI-7488825 | T06584 probable DNA-binding protein - gard... | -1 | 248 | 5.50E-21 | 1 |
| 1 | G1073 | GI-2213534 | DNA-binding PD1-like protein | -1 | 244 | 1.50E-20 | 1 |
| 1 | G1073 | GI-7488826 | T06582 probable DNA-binding protein - gard... | -1 | 244 | 1.50E-20 | 1 |
| 1 | G1073 | GI-4165183 | SAP1 protein | -1 | 232 | 2.70E-19 | 1 |
| 1 | G1073 | GI-1276971 | glycine-rich protein | -3 | 107 | 1.80E-05 | 1 |
| 1 | G1073 | GI-7488920 | T14306 glycine-rich protein - carrot (frag... | -3 | 107 | 1.80E-05 | 1 |
| 1 | G1073 | GI-19867 | extensin (AA 1-620) | 2 | 82 | 0.0002 | 3 |
| 1 | G1073 | GI-82169 | S06733 hydroxyproline-rich glycoprotein pr... | 2 | 82 | 0.0002 | 3 |
| 1 | G1073 | GI-119714 | EXTN_TOBAC EXTENSIN PRECURSOR (CELL WALL H... | 2 | 82 | 0.0002 | 3 |
| 1 | G1073 | GI-1155068 | cell wall-plasma membrane linker protein | 2 | 93 | 0.00031 | 3 |
| 1 | G1073 | GI-7446482 | S71558 probable cell wall-plasma membrane ... | 2 | 93 | 0.00031 | 3 |
| 1 | G1073 | GI-168457 | cell wall protein (put.); putative | 2 | 70 | 0.00078 | 3 |
| 1 | G1073 | GI-16924043 | AC079179_10 Unknown protein [Oryza sativa] | 2 | 94 | 0.0011 | 3 |
| 1 | G1073 | GI-6523547 | hydroxyproline-rich glycoprotein DZ-HRGP | - 2 | 85 | 0.0018 | 3 |
| 1 | G1073 | GI-1166450 | Tfm5 | -1 | 82 | 0.0028 | 2 |
| 1 | G1073 | GI-7489011 | T07381 glycine-rich protein Tfm5-tomato | -1 | 82 | 0.0028 | 2 |
| 1 | G1073 | GI-20245 | Glycine-rich protein | -1 | 82 | 0.0036 | 3 |
| 1 | G1073 | GI-72322 | KNRZG2 glycine-rich cell wall structural p... | -1 | 82 | 0.0036 | 3 |
| 1 | G1073 | GI-232183 | GRP2_ORYSA GLYCINE-RICH CELL WALL STRUCTUR... | -1 | 82 | 0.0036 | 3 |
| 1 | G1073 | GI-1167557 | glycine-rich protein | -1 | 82 | 0.0036 | 3 |
| 1 | G1073 | GI-5917666 | AF159297_1 extensin-like protein | 2 | 104 | 0.0039 | 3 |
| 1 | G1073 | GI-11545668 | AF317732_1 CIA5 [Chlamydomonas reinhardtii] | -1 | 78 | 0.004 | 3 |
| 1 | G1073 | GI-11875321 | CCM1-B [Chlamydomonas reinhardtii] | -1 | 78 | 0.004 | 3 |
| 1 | G1073 | GI-11875325 | CCM1-B [Chlamydomonas reinhardtii] | -1 | 78 | 0.004 | 3 |
| 1 | G1073 | GI-11875320 | CCM1-A [Chlamydomonas reinhardtii] | -1 | 78 | 0.004 | 3 |
| 1 | G1073 | GI-11875323 | CCM1-A [Chlamydomonas reinhardtii] | -1 | 78 | 0.004 | 3 |
| 1 | G1073 | GI-7008009 | PsAD1 | -1 | 85 | 0.0048 | 1 |
| 1 | G1073 | GI-22333 | hydroxyproline-rich glycoprotein | 2 | 70 | 0.0049 | 3 |
| 1 | G1073 | GI-228936 | 1814452A Hyp-rich glycoprotein [Zea mays] | 2 | 70 | 0.0049 | 3 |
| 1 | G1073 | GI-283045 | S28264 hydroxyproline-rich glycoprotein - ... | 2 | 70 | 0.0049 | 3 |
| 1 | G1073 | GI-22269 | cell wall protein (108 AA) | 2 | 68 | 0.0053 | 2 |
| 1 | G1073 | GI-100864 | S08315 cell wall protein - maize (fragment) | 2 | 68 | 0.0053 | 2 |
| 1 | G1073 | GI-168459 | cell wall protein (put.); putative | 2 | 68 | 0.0053 | 2 |
| 1 | G1073 | GI-227614 | 1707318A Thr rich extensin [Zea mays] | 2 | 70 | 0.0068 | 3 |
| 1 | G1073 | GI-13277222 | ZF-HD homeobox protein [Flaveria bidentis] | -1 | 87 | 0.0069 | 2 |
| 1 | G1073 | GI-228938 | 1814452C Hyp-rich glycoprotein [Zea diplop... | 2 | 70 | 0.0084 | 3 |
| 1 | G1073 | GI-22508 | cell wall protein (AA 1-267) | 2 | 70 | 0.0088 | 3 |
| 1 | G1073 | GI-100863 | S08314 cell wall glycoprotein - maize | 2 | 70 | 0.0088 | 3 |
| 1 | G1073 | GI-119712 | EXTN_MAIZE EXTENSIN PRECURSOR (PROLINE-RIC... | 2 | 70 | 0.0088 | 3 |
| 1 | G1073 | GI-168455 | cell wall protein (put.); putative | 2 | 70 | 0.0088 | 3 |
| 1 | G1073 | GI-226756 | 1604465A cell wall protein [Zea mays] | 2 | 70 | 0.0088 | 3 |
| 1 | G1073 | GI-228937 | 1814452B Hyp-rich glycoprotein [Zea mays] | 2 | 70 | 0.01 | 3 |
| 1 | G1073 | GI-14549150 | glycine-rich protein LeGRP1 [Lycopersicon ... | -1 | 85 | 0.012 | 3 |
| 1 | G1073 | GI-19929 | pistil extensin like protein | 2 | 78 | 0.012 | 2 |
| 1 | G1073 | GI-322761 | JQ1696 pistil extensin-like protein precur... | 2 | 78 | 0.012 | 2 |
| 1 | G1073 | GI-544262 | EXlP_TOBAC PISTIL-SPECIFIC EXTENSIN-LIKE P... | 2 | 78 | 0.012 | 2 |
| 1 | G1073 | GI-12083536 | AC084218_15 hypothetical protein [Oryza sa... | -1 | 97 | 0.012 | 2 |
| 1 | G1073 | GI-82091 | A25494 hydroxyproline-rich glycoprotein - ... | 2 | 66 | 0.013 | 2 |
| 1 | G1073 | GI-170454 | cell wall hydroxyproline-rich glycoprotein | 2 | 66 | 0.013 | 2 |
| 1 | G1073 | GI-224726 | 1111324A glycoprotein,Hyp rich [Lycopersic... | 2 | 66 | 0.013 | 2 |
| 1 | G1073 | GI-22092 | hydroxyproline-rich glycoprotein | 2 | 70 | 0.013 | 3 |
| 1 | G1073 | GI-283032 | S22456 hydroxyproline-rich glycoprotein - ... | 2 | 70 | 0.013 | 3 |
| 1 | G1073 | GI-395147 | glycine-rich protein | -1 | 80 | 0.015 | 3 |
| 1 | G1073 | GI-421936 | S34666 glycine-rich protein - common tobacco | -1 | 80 | 0.015 | 3 |
| 1 | G1073 | GI-226743 | 1604369A sulfated surface glycoprotein SSG... | 2 | 74 | 0.015 | 3 |
| 1 | G1073 | GI-82698 | JQ0985 hydroxyproline-rich glycoprotein pr... | 2 | 70 | 0.016 | 3 |
| 1 | G1073 | GI-257041 | hydroxyproline-rich glycoprotein, HRGP [ma... | 2 | 70 | 0.016 | 3 |
| 1 | G1073 | GI-4007865 | Hydroxyproline-rich Glycoprotein (HRGP) | 2 | 70 | 0.016 | 3 |
| 1 | G1073 | GI-4755087 | aluminum-induced protein; AI-induced protein | -1 | 95 | 0.025 | 1 |
| 1 | G1073 | GI-1184100 | pistil extensin-like protein | 2 | 71 | 0.027 | 3 |
| 1 | G1073 | GI-21992 | extensin | 2 | 72 | 0.027 | 3 |
| 1 | G1073 | GI-81286 | S22697 extensin - Volvox carteri (fragment) | 2 | 72 | 0.027 | 3 |
| 1 | G1073 | GI-347455 | hydroxyproline-rich glycoprotein | 2 | 58 | 0.034 | 3 |
| 1 | G1073 | GI-7488674 | T07623 extensin homolog HRGP2 - soybean (f... | 2 | 58 | 0.034 | 3 |
| 1 | G1073 | GI-2668742 | glycine-rich RNA binding protein | -1 | 89 | 0.041 | 1 |
| 1 | G1073 | GI-7439972 | T01356 glycine-rich RNA binding protein - ... | -1 | 89 | 0.041 | 1 |
| 1 | G1073 | GI-81870 | A29356 hydroxyproline-rich glycoprotein (c... | 2 | 60 | 0.047 | 3 |
| 1 | G1073 | GI-7671199 | AF246990_1 flagellar autotomy protein Fa1p... | -1 | 105 | 0.048 | 1 |
| 1 | G1073 | GI-2108256 | extensin | 2 | 55 | 0.05 | 2 |
| 1 | G1073 | GI-600118 | extensin-like protein | 2 | 103 | 0.051 | 1 |
| 1 | G1073 | GI-1076802 | S49915 extensin-like protein - maize | 2 | 103 | 0.051 | 1 |
| 1 | G1073 | GI-1096557 | 2111476A extensin-like domain [Zea mays] | 2 | 103 | 0.051 | 1 |
| 1 | G1073 | GI-14253159 | VMP3 protein [Volvox carteri f. nagariensis] | 2 | 73 | 0.055 | 3 |
| 1 | G1073 | GI-5917664 | AF159296_1 extensin-like protein | 2 | 66 | 0.056 | 3 |
| 1 | G1073 | GI-12018147 | AF309494_1 vegetative cell wall protein gp... | 2 | 67 | 0.057 | 3 |
| 1 | G1073 | GI-19322 | glycine-rich protein | -1 | 75 | 0.058 | 1 |
| 1 | G1073 | GI-82087 | S19774 glycine-rich protein - tomato (frag... | -1 | 75 | 0.058 | 1 |
| 1 | G1073 | GI-1486263 | extensin | 2 | 75 | 0.064 | 2 |
| 1 | G1073 | GI-7442094 | T09964 extensin CYC15 precursor- Madagasc... | 2 | 75 | 0.064 | 2 |
| 1 | G1073 | GI-451544 | proline-rich cell wall protein | 2 | 74 | 0.065 | 2 |
| 1 | G1073 | GI-7441828 | T10737 extensin-like cell wall protein - s... | 2 | 74 | 0.065 | 2 |
| 1 | G1073 | GI-435039 | proline-rich cell wall protein | 2 | 70 | 0.065 | 2 |
| 1 | G1073 | GI-7441829 | T09854 praline-rich cell wall protein - up... | 2 | 70 | 0.065 | 2 |
| 1 | G1073 | GI-496237 | homology with RNA-binding proteins in meri... | -1 | 89 | 0.08 | 1 |
| 1 | G1073 | GI-1346181 | GRP2_SINAL GLYCINE-RICH RNA-BINDING PROTEIN... | -1 | 89 | 0.08 | 1 |
| 1 | G1073 | GI-7440001 | T10465 glycine-rich protein 2a -white mus... | -1 | 89 | 0.08 | 1 |
| 1 | G1073 | GI-100687 | S20500 hydroxyproline-rich glycoprotein - ... | 3 | 60 | 0.082 | 3 |
| 1 | G1073 | GI-433816 | hydroxyproline-rich glycoprotein | 3 | 60 | 0.082 | 3 |
| 1 | G1073 | GI-100211 | S14984 glycine-rich protein (clone uA-3) -... | -1 | 84 | 0.089 | 1 |
| 1 | G1073 | GI-388258 | glycine-rich protein | -1 | 84 | 0.089 | 1 |
| 1 | G1073 | GI-347457 | hydroxyproline-rich glycoprotein | 2 | 60 | 0.09 | 3 |
| 1 | G1073 | GI-2331131 | glycine-rich protein | -1 | 83 | 0.1 | 2 |
| 1 | G1073 | GI-20553 | precursor polypeptide (aa -27 to 357) | -1 | 77 | 0.1 | 3 |
| 1 | G1073 | GI-82244 | A26099 glycine-rich cell wall structural p... | -1 | 77 | 0.1 | 3 |
| 1 | G1073 | GI-121627 | GRP1_PETHY GLYCINE-RICH CELL WALL STRUCTUR... | -1 | 77 | 0.1 | 3 |
| 1 | G1073 | GI-225181 | 1210313A Gly rich structural protein [Petu... | -1 | 77 | 0.1 | 3 |
| 1 | G1073 | GI-3204132 | extensin | 2 | 59 | 0.11 | 3 |
| 1 | G1073 | GI-17298137 | cold shock protein-1 [Triticum aestivum] | -1 | 85 | 0.12 | 1 |
| 1 | G1073 | GI-81869 | C29356 hydroxyproline-rich glycoprotein (c... | 2 | 68 | 0.12 | 3 |
| 1 | G1073 | GI-169349 | hydroxyproline-rich glycoprotein | 2 | 68 | 0.12 | 3 |
| 1 | G1073 | GI-12018149 | AF309495_1 gamete-specific hydroxyproline-... | 2 | 59 | 0.12 | 3 |
| 1 | G1073 | GI-15866589 | hypothetical protein [Capsella rubella] | 2 | 89 | 0.13 | 1 |
| 1 | G1073 | GI-20247 | glycine-rich cell wall protein | -1 | 87 | 0.13 | 1 |
| 1 | G1073 | GI-72321 | KNRZG1 glycine-rich cell wall structural p... | -1 | 87 | 0.13 | 1 |
| 1 | G1073 | GI-121626 | GRP1_ORYSA GLYCINE-RICH CELL WALL STRUCTUR... | -1 | 87 | 0.13 | 1 |
| 1 | G1073 | GI-19927 | pistil extensin like protein | 2 | 69 | 0.14 | 3 |
| 1 | G1073 | GI-322759 | PQ0479 pistil extensin-like protein (clone... | 2 | 69 | 0.14 | 3 |
| 1 | G1073 | GI-13486631 | hypothetical protein [Oryza sativa] | 2 | 68 | 0.14 | 2 |
| 1 | G1073 | GI-728594 | glycine rich protein, RNA binding protein | -1 | 81 | 0.16 | 2 |
| 1 | G1073 | GI-1076731 | S53050 RNA binding protein - barley | -1 | 81 | 0.16 | 2 |
| 1 | G1073 | GI-19917 | Pistil extensin like protein, partial CDS... | -2 | 68 | 0.16 | 2 |
| 1 | G1073 | GI-22293 | glycine-rich protein | -1 | 85 | 0.17 | 1 |
| 1 | G1073 | GI-82696 | S20846 glycine-rich protein - maize | -1 | 85 | 0.17 | 1 |
| 1 | G1073 | GI-13122418 | putative glycin-rich protein [Oryza sativa] | -1 | 79 | 0.17 | 3 |
| 1 | G1073 | GI-13161451 | putative glycin-rich protein [Oryza sativa] | -1 | 79 | 0.17 | 3 |
| 1 | G1073 | GI-488731 | chitinase | 2 | 60 | 0.17 | 3 |
| 1 | G1073 | GI-829258 | Chitinase | 2 | 60 | 0.17 | 3 |
| 1 | G1073 | GI-904359 | chitinase 1 | 2 | 60 | 0.17 | 3 |
| 1 | G1073 | GI-2129478 | S51939 chitinase (EC 3.2.1.14) precursor-... | 2 | 60 | 0.17 | 3 |
| 1 | G1073 | GI-7488999 | S14981 extensin class I (clone w1-8 L) - t... | 2 | 69 | 0.18 | 3 |
| 1 | G1073 | GI-2147979 | S66275 proline-rich protein - Solanum brev... | 2 | 66 | 0.18 | 3 |
| 1 | G1073 | GI-100213 | S14972 extensin class I (clone w6) - tomato | 2 | 59 | 0.21 | 2 |
| 1 | G1073 | GI-1345536 | extensin (class I) | 2 | 59 | 0.21 | 2 |
| 1 | G1073 | GI-974605 | single-stranded nucleic acid binding protein | -1 | 84 | 0.28 | 1 |
| 1 | G1073 | GI-7439981 | S71779 glycine-rich RNA-binding protein GR... | -1 | 84 | 0.28 | 1 |
| 1 | G1073 | GI-2267593 | glycine-rich RNA-binding protein | -1 | 73 | 0.28 | 2 |
| 1 | G1073 | GI-7439985 | T03583 glycine-rich RNA-binding protein - ... | -1 | 73 | 0.28 | 2 |
| 1 | G1073 | GI-20420 | extensin | 2 | 73 | 0.29 | 2 |
| 1 | G1073 | GI-99861 | S20790 extensin - almond | 2 | 73 | 0.29 | 2 |
| 1 | G1073 | GI-445616 | 1909363A extensin [Prunus dulcis] | 2 | 73 | 0.29 | 2 |
| 1 | G1073 | GI-2624326 | OsGRP1 | -1 | 78 | 0.3 | 2 |
| 1 | G1073 | GI-7439987 | T04346 glycine-rich RNA-binding protein - ... | -1 | 78 | 0.3 | 2 |
| 1 | G1073 | GI-791148 | extensin-like protein | 2 | 65 | 0.32 | 3 |
| 1 | G1073 | GI-1076556 | S54156 extensin-like protein - cowpea (fra... | 2 | 65 | 0.32 | 3 |
| 1 | G1073 | GI-18076086 | glycine-rich RNA-binding protein [Ricinus ... | -1 | 83 | 0.34 | 1 |
| 1 | G1073 | GI-100753 | S13383 hydroxyproline-rich glycoprotein - ... | 2 | 81 | 0.35 | 3 |
| 1 | G1073 | GI-228939 | 1814452D Hyp-rich glycoprotein [Sorghum bi... | 2 | 81 | 0.35 | 3 |
| 1 | G1073 | GI-21627 | hydroxyproline-rich glycoprotein | 2 | 81 | 0.35 | 3 |
| 1 | G1073 | GI-119713 | EXTN_SORVU EXTENSIN PRECURSOR (PROLINE-RIC... | 2 | 81 | 0.35 | 3 |
| 1 | G1073 | GI-15624033 | hypothetical protein [Oryza sativa] | -2 | 66 | 0.36 | 2 |
| 1 | G1073 | GI-100219 | S14985 glycine-rich protein (clone uK-4) -... | -1 | 67 | 0.36 | 1 |
| 1 | G1073 | GI-1345534 | glycine-rich protein | -1 | 67 | 0.36 | 1 |
| 1 | G1073 | GI-13122430 | hypothetical protein [Oryza sativa] | 2 | 68 | 0.39 | 2 |
| 1 | G1073 | GI-5726567 | AF169205_1 glycine-rich RNA-binding protein | -1 | 82 | 0.39 | 1 |
| 1 | G1073 | GI-21001 | GRP 1.0 protein (AA 1 - 252) | -1 | 81 | 0.39 | 2 |
| 1 | G1073 | GI-81867 | S01821 glycine-rich protein 1.0 precursor ... | -1 | 81 | 0.39 | 2 |
| 1 | G1073 | GI-121628 | GRP1_PHAVU GLYCINE-RICH CELL WALL STRUCTUR... | -1 | 81 | 0.39 | 2 |
| 1 | G1073 | GI-13958149 | AF363369_1 acetolactate synthase [Amaranth... | -1 | 91 | 0.44 | 1 |
| 1 | G1073 | GI-13958151 | AF363370_1 acetolactate synthase [Amaranth... | -1 | 91 | 0.44 | 1 |
| 1 | G1073 | GI-100216 | S14975 extensin class II (clone uJ-2) - to... | 2 | 66 | 0.44 | 1 |
| 1 | G1073 | GI-1345538 | extensin (class II) | 2 | 66 | 0.44 | 1 |
| 1 | G1073 | GI-2108258 | extensin | -2 | 43 | 0.44 | 2 |
| 1 | G1073 | GI-17819 | glycine-rich RNA binding protein | -1 | 73 | 0.45 | 2 |
| 1 | G1073 | GI-541908 | S38331 glycine-rich RNA-binding protein - ... | -1 | 73 | 0.45 | 2 |
| 1 | G1073 | GI-544416 | GR10_BRANA GLYCINE-RICH RNA-BINDING PROTEI... | -1 | 73 | 0.45 | 2 |
| 1 | G1073 | GI-4704605 | AF109917_1 glycine-rich RNA-binding protein | -1 | 81 | 0.45 | 1 |
| 1 | G1073 | GI-18844875 | hypothetical protein [Oryza sativa (japoni... | -1 | 73 | 0.45 | 1 |
| 1 | G1073 | GI-19743 | nsGRP-2 | -1 | 84 | 0.46 | 1 |
| 1 | G1073 | GI-72323 | KNNT2S glycine-rich protein 2 - wood tobacco | -1 | 84 | 0.46 | 1 |
| 1 | G1073 | GI-121631 | GRP2_NICSY GLYCINE-RICH CELL WALL STRUCTUR... | -1 | 84 | 0.46 | 1 |
| 1 | G1073 | GI-322760 | PQ0474 pistil extensin-like protein precur... | -2 | 68 | 0.47 | 2 |
| 1 | G1073 | GI-3810890 | glycine-rich protein-2 | -1 | 72 | 0.49 | 3 |
| 1 | G1073 | GI-19923 | pistil extensin like protein, partial CDS | 2 | 67 | 0.5 | 2 |
| 1 | G1073 | GI-322758 | PQ0477 pistil extensin-like protein (clone... | -2 | 65 | 0.52 | 1 |
| 1 | G1073 | GI-496233 | homology with RNA-binding proteins in meri... | -1 | 81 | 0.52 | 1 |
| 1 | G1073 | GI-1346180 | GRP1_SINAL GLYCINE-RICH RNA-BINDING PROTEI... | -1 | 81 | 0.52 | 1 |
| 1 | G1073 | GI-7439999 | T10463 glycine-rich protein 1a - white mus... | -1 | 81 | 0.52 | 1 |
| 1 | G1073 | GI-21623 | glycine-rich RNA-binding protein | -1 | 79 | 0.52 | 1 |
| 1 | G1073 | GI-485420 | S12311 glycine-rich RNA-binding protein (c... | -1 | 79 | 0.52 | 1 |
| 1 | G1073 | GI-544421 | GRP1_SORVU GLYCINE-RICH RNA-BINDING PROTEIN 1 | -1 | 79 | 0.52 | 1 |
| 1 | G1073 | GI-15887008 | glycine rich protein [Oryza sativa] | -1 | 82 | 0.53 | 1 |
| 1 | G1073 | GI-169345 | hydroxyproline-rich glycoprotein | 2 | 60 | 0.53 | 2 |
| 1 | G1073 | GI-18343 | put. precursor | 2 | 66 | 0.53 | 3 |
| 1 | G1073 | GI-82047 | A24354 extensin precursor - carrot | 2 | 66 | 0.53 | 3 |
| 1 | G1073 | GI-119711 | EXTN_DAUCA EXTENSIN PRECURSOR | 2 | 66 | 0.53 | 3 |
| 1 | G1073 | GI-224686 | 1111211A extensin [Daucus carota] | 2 | 66 | 0.53 | 3 |
| 1 | G1073 | GI-17821 | glycine-rich_protein_(aa1-291) | -1 | 79 | 0.57 | 2 |
| 1 | G1073 | GI-322647 | S31415 glycine-rich protein GRP22 - rape | -1 | 79 | 0.57 | 2 |
| 1 | G1073 | GI-2317764 | putative cell wall protein | -1 | 71 | 0.58 | 2 |
| 1 | G1073 | GI-7442070 | T07959 probable cell wall protein - loblol... | -1 | 71 | 0.58 | 2 |
| 1 | G1073 | GI-1934994 | SGRP-1 | -1 | 80 | 0.59 | 1 |
| 1 | G1073 | GI-7439998 | T10479 glycine-rich RNA-binding protein GR... | -1 | 80 | 0.59 | 1 |
| 1 | G1073 | GI-1532071 | glycine-rich protein | -1 | 75 | 0.6 | 2 |
| 1 | G1073 | GI-7442081 | T03371 glycine-rich protein grp3 - maize | -1 | 75 | 0.6 | 2 |
| 1 | G1073 | GI-11692772 | AF310674_1 trans-splicing factor Raa3 [Chl... | -1 | 88 | 0.6 | 2 |
| 1 | G1073 | GI-11692774 | trans-splicing factor Raa3 [Chlamydomonas ... | -1 | 88 | 0.6 | 2 |
| 1 | G1073 | GI-2674201 | glycine-rich RNA-binding protein | -1 | 80 | 0.61 | 1 |
| 1 | G1073 | GI-17932712 | putative chitinase [Musa acuminata] | -1 | 85 | 0.62 | 1 |
| 1 | G1073 | GI-791146 | extensin-like protein | 2 | 62 | 0.62 | 3 |
| 1 | G1073 | GI-7488885 | T11671 extensin-like protein 127 - cowpea ... | 2 | 62 | 0.62 | 3 |
| 1 | G1073 | GI-99441 | A33647 sulfated surface glycoprotein 185 -... | 2 | 80 | 0.64 | 2 |
| 1 | G1073 | GI-134920 | SSGP_VOLCA SULFATED SURFACE GLYCOPROTEIN 1... | 2 | 80 | 0.64 | 2 |
| 1 | G1073 | GI-1405821 | SULFATED SURFACE GLYCOPROTEIN 185 | 2 | 80 | 0.64 | 2 |
| 1 | G1073 | GI-2226370 | RNA-binding protein | -1 | 75 | 0.65 | 2 |
| 1 | G1073 | GI-791150 | extensin-like protein | 2 | 57 | 0.65 | 3 |
| 1 | G1073 | GI-1076555 | S54155 extensin-like protein - cowpea (fra... | 2 | 57 | 0.65 | 3 |
| 1 | G1073 | GI-11034630 | hypothetical protein [Oryza sativa] | -1 | 85 | 0.67 | 1 |
| 1 | G1073 | GI-14090230 | hypothetical protein [Oryza sativa] | -1 | 85 | 0.67 | 1 |
| 1 | G1073 | GI-14488305 | AC084884_1 Putative mudrA protein - maize ... | -1 | 90 | 0.68 | 1 |
| 1 | G1073 | GI-15209152 | AC091665_11 Putative mudrA protein - maize... | -1 | 90 | 0.68 | 1 |
| 1 | G1073 | GI-7636182 | glycine-rich protein [Triticum aestivum] | -1 | 74 | 0.68 | 3 |
| 1 | G1073 | GI-388257 | glycine-rich protein | -1 | 63 | 0.71 | 1 |
| 1 | G1073 | GI-399783 | GRW1_LYCES GLYCINE-RICH CELL WALL STRUCTUR... | -1 | 63 | 0.71 | 1 |
| 1 | G1073 | GI-1345533 | glycine-rich protein | -1 | 69 | 0.74 | 1 |
| 1 | G1073 | GI-7489008 | S14980 glycine-rich protein (clone w1-8) -... | -1 | 69 | 0.74 | 1 |
| 1 | G1073 | GI-13486802 | hypothetical protein [Oryza sativa] | 2 | 82 | 0.75 | 2 |
| 1 | G1073 | GI-20249 | gt-2 | -1 | 64 | 0.75 | 3 |
| 1 | G1073 | GI-283004 | S28030 DNA-binding protein Gt-2-rice | -1 | 64 | 0.75 | 3 |
| 1 | G1073 | GI-15022163 | putative proline-rich protein [Solanum bre... | 2 | 66 | 0.75 | 3 |
| 1 | G1073 | GI-18148 | glycine-rich protein (AA 1-144) rpt | -1 | 77 | 0.76 | 1 |
| 1 | G1073 | GI-81450 | S04069 glycine-rich protein - red goosefoot | -1 | 77 | 0.76 | 1 |
| 1 | G1073 | GI-122785 | GRP1_CHERU GLYCINE-RICH PROTEIN HC1 | -1 | 77 | 0.76 | 1 |
| 1 | G1073 | GI-486811 | S35716 glycine-rich protein (clone DC 9.1)... | -1 | 77 | 0.76 | 1 |
| 1 | G1073 | GI-585219 | GRP9_DAUCA GLYCINE-RICH PROTEIN DC9.1 | -1 | 77 | 0.76 | 1 |
| 1 | G1073 | GI-4996642 | Dof zinc finger protein | -1 | 66 | 0.77 | 2 |
| 1 | G1073 | GI-10799202 | glycine-rich RNA-binding protein [Sorghum... | -1 | 68 | 0.78 | 2 |
| 1 | G1073 | GI-13397640 | unnamed protein product [Brassica napus] | 2 | 70 | 0.78 | 3 |
| 1 | G1073 | GI-13447449 | receptor protein kinase PERK1 [Brassica na... | 2 | 70 | 0.78 | 3 |
| 1 | G1073 | GI-19521 | proline rich protein | 2 | 65 | 0.79 | 3 |
| 1 | G1073 | GI-100248 | S16589 proline-rich protein - tomato | 2 | 65 | 0.79 | 3 |
| 1 | G1073 | GI-9711862 | putative extensin-like protein [Oryza sativa] | 2 | 78 | 0.79 | 3 |
| 1 | G1073 | GI-13161443 | putative extensin-like protein [Oryza sativa] | 2 | 78 | 0.79 | 3 |
| 1 | G1073 | GI-4467884 | extensin (class I) | 2 | 62 | 0.79 | 1 |
| 1 | G1073 | GI-7488997 | S14973 extensin class I (clone uG) - tomat... | 2 | 62 | 0.79 | 1 |
| 1 | G1073 | GI-1592792 | auxin induced proline rich protein | -2 | 62 | 0.79 | 1 |
| 1 | G1073 | GI-7488752 | T09540 proline rich protein, auxin-induced... | -2 | 62 | 0.79 | 1 |
| 1 | G1073 | GI-7248461 | root cap-specific protein [Zea mays] | -1 | 62 | 0.79 | 1 |
| 1 | G1073 | GI-8515090 | AF101785_1 putative arabinogalactan protei... | 2 | 66 | 0.79 | 3 |
| 1 | G1073 | GI-2950243 | extensin | 3 | 68 | 0.79 | 2 |
| 1 | G1073 | GI-7446484 | T05717 probable extensin - barley (fragment) | 3 | 68 | 0.79 | 2 |
| 1 | G1073 | GI-21003 | GRP 1.8 protein (AA 1 - 465) | -1 | 85 | 0.8 | 2 |
| 1 | G1073 | GI-81868 | S01820 glycine-rich cell wall protein 1.8 ... | -1 | 85 | 0.8 | 2 |
| 1 | G1073 | GI-121632 | GRP2_PHAVU GLYCINE-RICH CELL WALL STRUCTUR... | -1 | 85 | 0.8 | 2 |
| 1 | G1073 | GI-3893085 | glycine-rich protein 2 | -1 | 80 | 0.81 | 1 |
| 1 | G1073 | GI-18461260 | hypothetical protein [Oryza sativa] | 2 | 58 | 0.83 | 2 |
| 1 | G1073 | GI-18266043 | AF458408_1 glycine-rich protein [Brassica ... | -1 | 74 | 0.83 | 1 |
| 1 | G1073 | GI-886961 | LMW glutenin | -1 | 49 | 0.83 | 2 |
| 1 | G1073 | GI-1362187 | S57645 LMW glutenin precursor - wheat (fra... | -1 | 49 | 0.83 | 2 |
| 1 | G1073 | GI-4115615 | root cap-specific glycine-rich protein | -1 | 79 | 0.84 | 1 |
| 1 | G1073 | GI-347453 | hydroxyproline-rich glycoprotein | 2 | 67 | 0.84 | 2 |
| 1 | G1073 | GI-7488673 | T07622 extensin homolog - soybean (fragment) | 2 | 67 | 0.84 | 2 |
| 1 | G1073 | GI-19034049 | AC092263_11 putative extensin [Oryza sativ... | 2 | 64 | 0.84 | 3 |
| 1 | G1073 | GI-5091527 | Similar to Zea mays PRP gene.(X60432) | 2 | 71 | 0.85 | 2 |
| 1 | G1073 | GI-13365569 | hypothetical protein-similar to Oryza sati... | -1 | 87 | 0.86 | 1 |
| 1 | G1073 | GI-1247386 | PRP2 | 2 | 58 | 0.86 | 2 |
| 1 | G1073 | GI-100582 | S10334 glycine-rich protein precursor-ba... | -1 | 79 | 0.87 | 1 |
| 1 | G1073 | GI-121644 | GRP_HORVU GLYCINE-RICH CELL WALL STRUCTURA... | -1 | 79 | 0.87 | 1 |
| 1 | G1073 | GI-295808 | glycine-rich protein | -1 | 79 | 0.87 | 1 |
| 1 | G1073 | GI-2578444 | ptxA | 2 | 74 | 0.87 | 2 |
| 1 | G1073 | GI-7446483 | T06482 probable cell wall protein - garden... | 2 | 74 | 0.87 | 2 |
| 1 | G1073 | GI-7442088 | T09527 glycine-rich protein 1 - chickpea | -1 | 76 | 0.87 | 1 |
| 1 | G1073 | GI-9988442 | hypothetical protein [Oryza sativa] | 3 | 69 | 0.89 | 2 |
| 1 | G1073 | GI-10934071 | hypothetical protein [Oryza sativa] | 3 | 69 | 0.89 | 2 |
| 1 | G1073 | GI-19390 | proline rich protein | 2 | 65 | 0.89 | 3 |
| 1 | G1073 | GI-100249 | S19129 proline-rich protein TPRP-F1 -tomato | 2 | 65 | 0.89 | 3 |
| 1 | G1073 | GI-1709767 | PRF1_LYCES 36.4 KD PROLINE-RICH PROTEIN | 2 | 65 | 0.89 | 3 |
| 1 | G1073 | GI-7211797 | AF239615_1 CRANTZ hydroxyproline-rich glyc... | 2 | 61 | 0.89 | 3 |
| 1 | G1073 | GI-14140115 | putative protein [Oryza sativa] | -1 | 79 | 0.9 | 1 |
| 1 | G1073 | GI-1276958 | glycine-rich cell wall protein | -1 | 56 | 0.9 | 2 |
| 1 | G1073 | GI-7488919 | T14302 glycine-rich cell wall protein - ca... | -1 | 56 | 0.9 | 2 |
| 1 | G1073 | GI-13443598 | glycine-rich protein 1 [Cicer arietinum] | -1 | 76 | 0.9 | 1 |
| 1 | G1073 | GI-452596 | ORF | -1 | 77 | 0.9 | 1 |
| 1 | G1073 | GI-629868 | JC2213 hypothetical 14.7K protein, LIM14 -... | -1 | 77 | 0.9 | 1 |
| 1 | G1073 | GI-2653671 | 120 kDa style glycoprotein | 2 | 66 | 0.91 | 2 |
| 1 | G1073 | GI-7489086 | T10741 extensin-like protein PRP5 - Persia... | 2 | 66 | 0.91 | 2 |
| 1 | G1073 | GI-4105119 | dehydrin 10 | -1 | 81 | 0.91 | 1 |
| 1 | G1073 | GI-6017946 | AF181460_1 dehydrin; DHN10 | -1 | 81 | 0.91 | 1 |
| 1 | G1073 | GI-7489009 | S14982 glycine-rich protein (clone w10-1) ... | -1 | 63 | 0.92 | 2 |
| 1 | G1073 | GI-397517 | hydroxyproline-rich glycoprotein | -2 | 51 | 0.93 | 2 |
| 1 | G1073 | GI-13786451 | AC025296_11 putative transcription factor ... | -1 | 86 | 0.93 | 1 |
| 1 | G1073 | GI-81871 | B29356 hydroxyproline-rich glycoprotein pr... | 2 | 61 | 0.93 | 3 |
| 1 | G1073 | GI-169347 | hydroxyproline-rich glycoprotein | 2 | 61 | 0.93 | 3 |
| 1 | G1073 | GI-1469223 | glycine-rich protein | -1 | 73 | 0.93 | 2 |
| 1 | G1073 | GI-7488554 | T14441 glycine-rich protein - wild cabbage... | -1 | 73 | 0.93 | 2 |
| 1 | G1073 | GI-19919 | Pistil extensin like protein, partial CDS... | -2 | 66 | 0.93 | 2 |
| 1 | G1073 | GI-322755 | PQ0475 pistil extensin-like protein (clone... | -2 | 66 | 0.93 | 2 |
| 1 | G1073 | GI-7024449 | glycine-rich protein | -1 | 73 | 0.94 | 1 |
| 1 | G1073 | GI-7024451 | glycine-rich RNA-binding protein | -1 | 76 | 0.95 | 1 |
| 1 | G1073 | GI-15623846 | putative receptor protein kinase [Oryza sa... | -1 | 84 | 0.95 | 1 |
| 1 | G1073 | GI-1345537 | extensin (class I) | 2 | 63 | 0.96 | 3 |
| 1 | G1073 | GI-7488998 | S14974 extensin class I (clone uG-18) - to... | 2 | 63 | 0.96 | 3 |
| 1 | G1073 | GI-7339703 | hypothetical protein [Oryza sativa] | 2 | 61 | 0.96 | 2 |
| 1 | G1073 | GI-688080 | lectin=chitin-binding protein [Solanum tub... | 2 | 59 | 0.96 | 1 |
| 1 | G1073 | GI-18844948 | hypothetical protein [Oryza sativa gaponi ... | 3 | 59 | 0.96 | 1 |
| 1 | G1073 | GI-10716605 | AC026815_7 putative RNA binding protein [O... | -1 | 72 | 0.97 | 2 |
| 1 | G1073 | GI-1229138 | low temperature-responsive RNA-binding pro... | -1 | 75 | 0.97 | 1 |
| 1 | G1073 | GI-7439979 | S71453 glycine-rich RNA-binding protein, I... | -1 | 75 | 0.97 | 1 |
| 1 | G1073 | GI-4033606 | Extensin | 2 | 61 | 0.97 | 3 |
| 1 | G1073 | GI-469072 | RNA-binding gricine-rich protein-1 (RGP-1c) | -1 | 75 | 0.98 | 1 |
| 1 | G1073 | GI-2119043 | S41773 glycine-rich RNA-binding protein RG... | -1 | 75 | 0.98 | 1 |
| 1 | G1073 | GI-7439982 | S59529 RNA-binding glycine-rich protein-1 ... | -1 | 75 | 0.98 | 1 |
| 1 | G1073 | GI-15021756 | AF397033_1 nodule extensin [Pisum sativum] | 2 | 53 | 0.98 | 2 |
| 1 | G1073 | GI-1165322 | extensin | - 2 | 68 | 0.98 | 2 |
| 1 | G1073 | GI-7441831 | T06782 extensin - soybean | 2 | 68 | 0.98 | 2 |
| 1 | G1073 | GI-607771 | arabinogalactan-like protein | 2 | 75 | 0.98 | 1 |
| 1 | G1073 | GI-1076237 | S52994 arabinogalactan-like protein - lobl... | 2 | 75 | 0.98 | 1 |
| 1 | G1073 | GI-22550 | 27kDa storage protein, zein | 2 | 56 | 0.98 | 2 |
| 1 | G1073 | GI-100925 | S22990 zein, 27K - maize (fragment) | 2 | 56 | 0.98 | 2 |
| 1 | G1073 | GI-6063539 | hypothetical protein | -1 | 52 | 0.98 | 2 |
| 1 | G1073 | GI-18542888 | AC091724_3 Unknown protein [Oryza sativa] | 2 | 56 | 0.98 | 2 |
| 1 | G1073 | GI-2852377 | hairy root 4 | -1 | 76 | 0.99 | 1 |
| 1 | G1073 | GI-7489211 | T01982 tumor related protein HR4 - common ... | -1 | 76 | 0.99 | 1 |
| 1 | G1073 | GI-469071 | RNA-binding glycine-rich protein-1 (RGP-1b) | -1 | 67 | 0.99 | 2 |
| 1 | G1073 | GI-2119044 | S41772 glycine-rich RNA-binding protein RG... | -1 | 67 | 0.99 | 2 |
| 1 | G1073 | GI-17385704 | contains EST C73460(E4160)-unknown protein... | -1 | 79 | 0.99 | 1 |
| 1 | G1073 | GI-310925 | cysteine-rich extensin-like protein-2 | -2 | 66 | 0.99 | 2 |
| 1 | G1073 | GI-539030 | B48232 cysteine-rich extensin-like protein... | -2 | 66 | 0.99 | 2 |
| 1 | G1073 | GI-322757 | PQ0476 pistil extensin-like protein (clone... | 2 | 60 | 0.991 | 2 |
| 1 | G1073 | GI-1076501 | S52985 cell wall protein - alfalfa | 2 | 61 | 0.991 | 3 |
| 1 | G1073 | GI-3818416 | proline-rich cell wall protein | 2 | 61 | 0.991 | 3 |
| 1 | G1073 | GI-100214 | S14971 extensin class I (done wY) - tomato | 2 | 57 | 0.992 | 2 |
| 1 | G1073 | GI-1345535 | extensin (class I) | 2 | 57 | 0.992 | 2 |
| 1 | G1073 | GI-18461235 | putative nuclear RNA binding protein A [Or... | -1 | 65 | 0.992 | 2 |
| 1 | G1073 | GI-5091598 | AC007858_1 10A19I.1 | 2 | 75 | 0.992 | 1 |
| 1 | G1073 | GI-6911142 | putative glycine-rich RNA binding protein 1 | -1 | 72 | 0.993 | 1 |
| 1 | G1073 | GI-14164468 | hypothetical protein [Oryza sativa] | 2 | 82 | 0.993 | 1 |
| 1 | G1073 | GI-13569981 | AC079890_1 hypothetical protein [Oryza sat... | 2 | 79 | 0.993 | 1 |
| 1 | G1073 | GI-18873840 | AC079874_9 unknown protein [Oryza sativa] | 2 | 79 | 0.993 | 1 |
| 1 | G1073 | GI-1173628 | glycine-rich protein | -1 | 75 | 0.995 | 1 |
| 1 | G1073 | GI-15021742 | AF397026_1 nodule extensin [Pisum sativum] | 2 | 56 | 0.995 | 3 |
| 1 | G1073 | GI-18103931 | glycine rich RNA binding protein [Oryza sa... | -1 | 75 | 0.996 | 1 |
| 1 | G1073 | GI-1345532 | glycine-rich protein | -1 | 57 | 0.996 | 1 |
| 1 | G1073 | GI-7489007 | S14979 glycine-rich protein (clone uE-7) -... | -1 | 57 | 0.996 | 1 |
| 1 | G1073 | GI-310923 | cysteine-rich extensin-like protein-1 | -2 | 66 | 0.996 | 2 |
| 1 | G1073 | GI-539029 | A48232 cysteine-rich extensin-like protein... | -2 | 66 | 0.996 | 2 |
| 1 | G1073 | GI-1657851 | cold acclimation protein WCOR518 | 2 | 67 | 0.997 | 2 |
| 1 | G1073 | GI-7446487 | T06806 proline rich protein homolog WCOR51... | 2 | 67 | 0.997 | 2 |
| 1 | G1073 | GI-7959089 | glycine-rich protein [Nicotiana tabacum] | -1 | 73 | 0.997 | 1 |
| 1 | G1073 | GI-8099125 | rice EST C27893 corresponds to a region of... | -1 | 53 | 0.997 | 3 |
| 1 | G1073 | GI-18844823 | hypothetical protein [Oryza sativa (japoni... | -1 | 60 | 0.997 | 3 |
| 1 | G1073 | GI-6651027 | AF127919_1 high mobility group protein I/Y | 2 | 61 | 0.998 | 2 |
| 1 | G1073 | GI-727264 | hydroxyproline-rich glycoprotein precursor | 2 | 61 | 0.998 | 3 |
| 1 | G1073 | GI-7488765 | T10863 extensin precursor - kidney bean | 2 | 61 | 0.998 | 3 |
| 1 | G1073 | GI-168695 | gamma zein | 2 | 56 | 0.998 | 2 |
| 1 | G1073 | GI-225315 | 1211356A zein gamma [Zea mays] | 2 | 56 | 0.998 | 2 |
| 1 | G1073 | GI-3126963 | acidic chitinase | -1 | 78 | 0.998 | 1 |
| 1 | G1073 | GI-258215 | NaPRP3=putative proline-rich protein [Nico... | 2 | 72 | 0.999 | 1 |
| 1 | G1073 | GI-322749 | JQ1686 extensin-like protein precursor - P... | 2 | 72 | 0.999 | 1 |
| 1 | G1073 | GI-1247390 | PRP3 | 2 | 72 | 0.999 | 1 |
| 1 | G1073 | GI-15290188 | hypothetical protein [Oryza sativa] | -1 | 57 | 0.999 | 2 |
| 1 | G1073 | GI-15623924 | hypothetical protein [Oryza sativa] | -1 | 57 | 0.999 | 2 |
| 1 | G1073 | GI-100209 | S25298 extensin (clone Tom J-10) - tomato | 2 | 58 | 0.999 | 3 |
| 1 | G1073 | GI-170442 | extensin (class I) | 2 | 58 | 0.999 | 3 |
| 1 | G1073 | GI-283044 | JQ1663 hybrid proline-rich protein - maize | 2 | 73 | 0.999 | 2 |
| 1 | G1073 | GI-433707 | prolin rich protein | 2 | 73 | 0.999 | 2 |
| 1 | G1073 | GI-22313 | ABA-inducible gene protein | -1 | 63 | 0.999 | 2 |
| 1 | G1073 | GI-82685 | S04536 embryonic abundant protein, glycine... | -1 | 63 | 0.999 | 2 |
| 1 | G1073 | GI-112994 | GRPA_MAIZE GLYCINE-RICH RNA-BINDING, ABACI... | -1 | 63 | 0.999 | 2 |
| 1 | G1073 | GI-226091 | 1410284A abscisic acid inducible gene [Zea... | -1 | 63 | 0.999 | 2 |
| 1 | G1073 | GI-2598593 | MtN12 | 2 | 53 | 0.9991 | 2 |
| 1 | G1073 | GI-72327 | ZMZM5 glutelin 5 - maize | 2 | 56 | 0.9992 | 1 |
| 1 | G1073 | GI-15822703 | RNA-binding protein precursor [Nicotiana t... | -1 | 63 | 0.9992 | 2 |
| 1 | G1073 | GI-100673 | A37288 DNA-binding protein GT-2 - rice (fr... | -1 | 64 | 0.9993 | 2 |
| 1 | G1073 | GI-6016872 | EST AU058127(S5254) corresponds to a regio... | 2 | 70 | 0.9994 | 1 |
| 1 | G1073 | GI-2196542 | glycine-rich protein | -1 | 74 | 0.9994 | 1 |
| 1 | G1073 | GI-7439984 | T03442 glycine-rich protein - rice | -1 | 74 | 0.9994 | 1 |
| 1 | G1073 | GI-22289 | glutelin-2 precursor | 2 | 56 | 0.9995 | 2 |
| 1 | G1073 | GI-22517 | zein Zc2 | 2 | 56 | 0.9995 | 2 |
| 1 | G1073 | GI-72326 | ZMZM19 glutelin 2 precursor (clone pME119)... | 2 | 56 | 0.9995 | 2 |
| 1 | G1073 | GI-121472 | GLU2_MAIZE GLUTELIN 2 PRECURSOR (ZEIN-GAMM... | 2 | 56 | 0.9995 | 2 |
| 1 | G1073 | GI-168485 | glutelin-2 | 2 | 56 | 0.9995 | 2 |
| 1 | G1073 | GI-16305109 | 27kD gamma zein [Zea mays] | 2 | 56 | 0.9995 | 2 |
| 1 | G1073 | GI-2331133 | glycine-rich protein | -1 | 72 | 0.9997 | 1 |
| 1 | G1073 | GI-11034632 | hypothetical protein [Oryza sativa] | -1 | 71 | 0.9998 | 2 |
| 1 | G1073 | GI-100210 | S25299 extensin precursor (clone Tom L-4) ... | 2 | 58 | 0.9998 | 3 |
| 1 | G1073 | GI-170444 | extensin (class II) | 2 | 58 | 0.9998 | 3 |
| 1 | G1073 | GI-12060518 | hypothetical protein [Oryza sativa] | -1 | 63 | 0.9998 | 2 |
| 1 | G1073 | GI-1486265 | extensin | 2 | 74 | 0.9999 | 1 |
| 1 | G1073 | GI-7442095 | T09965 extensin CYC17 precursor- Madagasc... | 2 | 74 | 0.9999 | 1 |
| 1 | G1073 | GI-15528745 | contains ESTs AU093876(E1018),AU093877(E10... | -1 | 74 | 0.9999 | 2 |
| 1 | G1073 | GI-100215 | S14970 extensin class I (clone w17-1) - to... | 2 | 53 | 0.9999 | 2 |
| 1 | G1073 | GI-1345531 | extensin (class I) | 2 | 53 | 0.9999 | 2 |
| 1 | G1073 | GI-15289856 | hypothetical protein [Oryza sativa] | 2 | 55 | 0.9999 | 1 |
| 1 | G1073 | GI-166376 | environmental stress-induced protein | -1 | 55 | 0.9999 | 1 |
| 1 | G1073 | GI-7488739 | T09610 environmental stress-induced protei... | -1 | 55 | 0.9999 | 1 |
| 1 | G1073 | GI-18813 | anther-specific protein SF18 | 2 | 64 | 0.9999 | 2 |
| 1 | G1073 | GI-100521 | S12246 anther-specific protein SF18 precur... | 2 | 64 | 0.9999 | 2 |
| 1 | G1073 | GI-114248 | ASF1_HELAN ANTHER-SPECIFIC PROTEIN SF18 PR... | 2 | 64 | 0.9999 | 2 |
| 1 | G1073 | GI-479021 | glycine-rich cell wall protein precursor | -1 | 64 | 0.99992 | 2 |
| 1 | G1073 | GI-1362083 | S56703 glycine-rich cell wall protein prec... | -1 | 64 | 0.99992 | 2 |
| 1 | G1073 | GI-469070 | RNA-binding glycine-rich protein-1 (RGP-1a) | -1 | 71 | 0.99994 | 1 |
| 1 | G1073 | GI-2119042 | S41771 glycine-rich RNA-binding protein RG... | -1 | 71 | 0.99994 | 1 |
| 1 | G1073 | GI-1247388 | PRP3 | 2 | 68 | 0.99994 | 1 |
| 1 | G1073 | GI-13486635 | putative protein kinase [Oryza sativa] | 2 | 65 | 0.99994 | 3 |

## Claims

1. A method for producing a transgenic plant having increased seed yield compared to a wild-type plant of the same species, the method steps comprising:
(a) introducing into a plant an expression vector comprising (i) a sequence having at least 85% amino acid identity to the polypeptide of SEQ ID NO:2 or (ii) a recombinant polynucleotide that encodes an AT-hook family polypeptide comprising a conserved domain that is at least 65% identical to amino acid residues 33 through 50 of SEQ ID NO: 2; and
(b) selecting a transgenic plant having increased seed yield so produced by comparing said transgenic plant with a wild-type plant of the same species.

2. A method according to claim 1, wherein said AT hook family polypeptide comprises a conserved domain that is at least 80% identical to amino acid residues 33 through 50 of SEQ ID NO: 2.

3. A method according to claim 1, wherein said AT-hook family polypeptide is SEQ ID NO: 2.

4. A method according to claim 3, wherein said seed yield is at least 50% greater than the seed yield of the wild-type plant.

5. A method to produce a transgenic seed, the method comprising:
(a) introducing into a plant an expression vector comprising (i) a sequence having at least 85% amino acid identity to the polypeptide of SEQ ID NO:2 or (ii) a recombinant polynucleotide that encodes an AT-hook family polypeptide comprising a conserved domain that is at least 65% identical to amino acid residues 33 through 50 of SEQ ID NO: 2;
(b) selecting a transgenic plant having increased seed yield so produced by comparing said transgenic plant with a wild-type plant of the same species; and
(c) growing the transgenic plant to produce a transgenic seed, wherein the transgenic seed comprises the recombinant polynucleotide.

6. A method according to claim 5, wherein a progeny plant grown from the transgenic seed has greater expression levels of the polypeptide than the wild-type plant.

7. A method according to any one of claims 1 to 6, wherein said transgenic plant is selected from the group consisting of *Cruciferae* including broccoli, rapeseed and cabbage; wheat; alfalfa; soybean; rice; corn; and *Arabidopsis.*

8. A method for increasing seed yield of a plant relative to a wild-type plant of the same species, comprising introducing into a plant an expression vector comprising a recombinant polynucleotide that encodes (i) a sequence having at least 85% amino acid identity to the polypeptide of SEQ ID NO:2 or (ii) an AT-hook family polypeptide comprising a conserved domain that is at least 65% identical to amino acid residues 33 through 50 of SEQ ID NO: 2.

9. A method according to claim 8, wherein said seed yield is at least 50% greater than a seed yield of the wild-type plant.

10. A method according to claim 8, wherein said AT hook family polypeptide_comprises a conserved domain that is at least 80% identical to amino acid residues 33 through 50 of SEQ ID NO: 2.

## Patentansprüche

1. Verfahren zur Herstellung einer transgenen Pflanze mit erhöhter Samenausbeute im Vergleich zu einer Wildtyp-Pflanze derselben Spezies, wobei das Verfahren folgende Schritte umfasst:
(a) Einführen eines Expressionsvektors in eine Pflanze, der (i) eine Sequenz mit zumindest 85 % Aminosäureidentität mit dem Polypeptid der Seq.-ID Nr. 2 oder (ii) ein rekombinantes Polynucleotid umfasst, das für ein Polypeptid der AT-Hook-Familie kodiert, das eine konservierte Domäne mit zumindest 65 % Identität mit den Aminosäureresten 33 bis 50 der Seq.-ID Nr. 2 umfasst; und
(b) Selektieren einer so hergestellten transgenen Pflanze mit erhöhter Samenausbeute durch Vergleichen der transgenen Pflanze mit einer Wildtyp-Pflanze derselben Spezies.

2. Verfahren nach Anspruch 1, worin das Polypeptid der AT-Hook-Familie eine konservierte Domäne umfasst, die zu zumindest 80 % mit den Aminosäureresten 33 bis 50 der Seq.-ID Nr. 2 identisch ist.

3. Verfahren nach Anspruch 1, worin das Polypeptid der AT-Hook-Familie Seq.-ID Nr. 2 ist.

4. Verfahren nach Anspruch 3, worin die Samenausbeute zumindest 50 % höher ist als die Samenausbeute der Wildtyp-Pflanze.

5. Verfahren zur Herstellung eines transgenen Samens, wobei das Verfahren Folgendes umfasst:
(a) Einführen eines Expressionsvektors in eine Pflanze, der (i) eine Sequenz mit zumindest 85 % Aminosäureidentität mit dem Polypeptid der Seq.-ID Nr. 2 oder (ii) ein rekombinantes Polynucleotid umfasst, das für ein Polypeptid der AT-Hook-Familie kodiert, das eine konservierte Domäne mit zumindest 65 % Identität mit den Aminosäureresten 33 bis 50 der Seq.-ID Nr. 2 umfasst;
(b) Selektieren einer so hergestellten transgenen Pflanze mit erhöhter Samenausbeute durch Vergleichen der transgenen Pflanze mit einer Wildtyp-Pflanze derselben Spezies; und
(c) Züchten der transgenen Pflanze, um einen transgenen Samen herzustellen, wobei der transgene Samen das rekombinante Polynucleotid umfasst.

6. Verfahren nach Anspruch 5, worin eine aus dem transgenen Samen gezüchtete Nachkommenspflanze ein höheres Expressionsniveau des Polypeptids aufweist als die Wildtyp-Pflanze.

7. Verfahren nach einem der Ansprüche 1 bis 6, worin die transgene Pflanze aus der aus *Cruciferae,* einschließlich Brokkoli, Raps und Kohl; Weizen; Luzerne; Sojabohnen; Reis; Mais; und *Arabidopsis* bestehenden Gruppe ausgewählt ist.

8. Verfahren zur Steigerung der Samenausbeute einer Pflanze in Bezug auf eine Wildtyp-Pflanze derselben Spezies, welches das Einführen eines Expressionsvektors in eine Pflanze umfasst, der ein rekombinantes Polynucleotid umfasst, das für (i) eine Sequenz mit zumindest 85 % Aminosäureidentität mit dem Polypeptid der Seq.-ID Nr. 2 oder (ii) ein Polypeptid der AT-Hook-Familie, das eine konservierte Domäne mit zumindest 65 % Identität mit den Aminosäureresten 33 bis 50 der Seq.-ID Nr. 2 umfasst, kodiert.

9. Verfahren nach Anspruch 8, worin die Samenausbeute zumindest 50 % höher ist als die Samenausbeute der Wildtyp-Pflanze.

10. Verfahren nach Anspruch 8, worin das Polypeptid der AT-Hook-Familie eine konservierte Domäne umfasst, die zu zumindest 80 % mit den Aminosäureresten 33 bis 50 der Seq.-ID Nr. 2 identisch ist.

## Revendications

1. Procédé de production d'une plante transgénique d'un plus grand rendement de semences en comparaison avec une plante de type sauvage de la même espèce, les étapes de procédé comprenant:
(a) introduire dans une plante un vecteur d'expression comprenant (i) une séquence ayant au moins 85% d'identité d'acide aminé avec le polypeptide de la SEQ ID NO: 2 ou bien (ii) un polynucléotide recombinant qui code pour un polypeptide de la famille de crochets AT comprenant un domaine conservé qui est au moins à 65% identique aux résidus d'acide aminé 33 à 50 de la SEQ ID NO: 2; et
(b) sélectionner une plante transgénique ayant un plus grand rendement de semences ainsi produite en comparant ladite plante transgénique à une plante de type sauvage de la même espèce.

2. Procédé selon la revendication 1, dans lequel ledit polypeptide de la famille des crochets AT comprend un domaine conservé qui est au moins à 80% identique aux résidus d'acide aminé 33 à 50 de la SEQ ID NO: 2.

3. Procédé selon la revendication 1, dans lequel ledit polypeptide de la famille de crochets AT est la SEQ ID NO: 2.

4. Procédé selon la revendication 3, dans lequel ledit rendement de semences est au moins 50% plus grand que le rendement de semences de la plante de type sauvage.

5. Procédé de production d'une semence transgénique, le procédé comprenant:
(a) introduire dans une plante un vecteur d'expression comprenant (i) une séquence ayant au moins 85% d'identité d'acide aminé avec le polypeptide de la SEQ ID NO: 2 ou bien (ii) un polynucléotide recombinant qui code pour un polypeptide de la famille des crochets AT comprenant un domaine conservé qui est au moins à 65% identique aux résidus d'acide aminé 33 à 50 de la SEQ ID NO: 2;
(b) sélectionner une plante transgénique d'un plus grand rendement de semences ainsi produite en comparant ladite plante transgénique avec une plante de type sauvage de la même espèce; et
(c) faire pousser la plante transgénique pour produire une semence transgénique, où la semence transgénique comprend le polynucléotide recombinant.

6. Procédé selon la revendication 5, dans lequel une plante de descendance poussée à partir de la semence transgénique possède des niveaux d'expression plus grands du polypeptide que la plante de type sauvage.

7. Procédé selon l'une quelconque des revendications 1 à 6, dans lequel ladite plante transgénique est sélectionnée dans le groupe consistant en *Cruciferae* incluant le broccoli, la graine de colza et le choux; le blé; la luzerne; le soja; le riz; le maïs et *Arabidopsis.*

8. Procédé pour augmenter le rendement de semences d'une plante relativement à une plante de type sauvage de la même espèce, comprenant l'introduction dans une plante d'un vecteur d'expression comprenant un polynucléotide recombinant qui code pour (i) une séquence ayant au moins 85% d'identité d'acide aminé avec le polypeptide de la SEQ ID NO: 2 ou bien (ii) un polypeptide de la famille des crochets AT comprenant un domaine conservé qui est au moins à 65% identique aux résidus d'acide aminé 33 à 50 de la SEQ ID NO: 2.

9. Procédé selon la revendication 8, dans lequel ledit rendement de semence est au moins 50% plus grand qu'un rendement de semence de la plante de type sauvage.

10. Procédé selon la revendication 8, dans lequel ledit polypeptide de la famille des crochets AT comprend un domaine conservé qui est au moins à 80% identique aux résidus d'acide aminé 33 à 50 de la SEQ ID NO: 2.


### SEQUENCE LISTING

<110> MENDEL BIOTECHNOLOGY, INC.
<120> Method for Modifying Plant Biomass
<130> AHB/FP6184998
<140> EP 02736510.5
   <141> 2002-03-26
<150> PCT/US02/09139
   <151> 2002-03-26
<150> US 09/823,676
   <151> 2001-03-30
<160> 8
<170> PatentIn version 3.0
<210> 1
   <211> 974
   <212> DNA
   <213> Arabidopsis thaliana
<220>
   <221> CDS
   <222> (62)..(874)
   <223> G1073
<400> 1
   ccccccgacc tgcctctaca gagacctgaa gattccagaa ccccacctga tcaaaaataa      60
   c atg gaa ctt aac aga tct gaa gca gac gaa gca aag gcc gag acc act    109
     Met Glu Leu Asn Arg Ser Glu Ala Asp Glu Ala Lys Ala Glu Thr Thr
     1               5                   10                  15
   ccc acc ggt gga gcc acc agc tca gcc aca gcc tct ggc tct tcc tcc      157
   Pro Thr Gly Gly Ala Thr Ser Ser Ala Thr Ala Ser Gly Ser Ser Ser
               20                  25                  30
   gga cgt cgt cca cgt ggt cgt cct gca ggt tcc aaa aac aaa ccc aaa      205
   Gly Arg Arg Pro Arg Gly Arg Pro Ala Gly Ser Lys Asn Lys Pro Lys
           35                  40                  45
   cct ccg acg att ata act aga gat agt cct aac gtc ctt aga tca cac      253
   Pro Pro Thr Ile Ile Thr Arg Asp Ser Pro Asn Val Leu Arg Ser His
       50                  55                  60
   gtt ctt gaa gtc acc tcc ggt tcg gac ata tcc gag gca gtc tcc acc      301
   Val Leu Glu Val Thr Ser Gly Ser Asp Ile Ser Glu Ala Val Ser Thr
   65                  70                  75                  80
   tac gcc act cgt cgc ggc tgc ggc gtt tgc att ata agc ggc acg ggt      349
   Tyr Ala Thr Arg Arg Gly Cys Gly Val Cys Ile Ile Ser Gly Thr Gly
                   85                  90                  95
   gcg gtc act aac gtc acg ata cgg caa cct gcg gct ccg gct ggt gga      397
   Ala Val Thr Asn Val Thr Ile Arg Gln Pro Ala Ala Pro Ala Gly Gly
               100                 105                 110
   ggt gtg att acc ctg cat ggt cgg ttt gac att ttg tct ttg acc ggt      445
   Gly Val Ile Thr Leu His Gly Arg Phe Asp Ile Leu Ser Leu Thr Gly
           115                 120                 125
   act gcg ctt cca ccg cct gca cca ccg gga gca gga ggt ttg acg gtg      493
   Thr Ala Leu Pro Pro Pro Ala Pro Pro Gly Ala Gly Gly Leu Thr Val
       130                 135                 140
   tat cta gcc gga ggt caa gga caa gtt gta gga ggg aat gtg gct ggt      541
   Tyr Leu Ala Gly Gly Gln Gly Gln Val Val Gly Gly Asn Val Ala Gly
   145                 150                 155                 160
   tcg tta att gct tcg gga ccg gta gtg ttg atg gct gct tct ttt gca      589
   Ser Leu Ile Ala Ser Gly Pro Val Val Leu Met Ala Ala Ser Phe Ala
                   165                 170                 175
   aac gca gtt tat gat agg tta ccg att gaa gag gaa gaa acc cca ccg      637
   Asn Ala Val Tyr Asp Arg Leu Pro Ile Glu Glu Glu Glu Thr Pro Pro
               180                 185                 190
   ccg aga acc acc ggg gtg cag cag cag cag ccg gag gcg tct cag tcg      685
   Pro Arg Thr Thr Gly Val Gln Gln Gln Gln Pro Glu Ala Ser Gln Ser
           195                 200                 205
   tcg gag gtt acg ggg agt ggg gcc cag gcg tgt gag tca aac ctc caa      733
   Ser Glu Val Thr Gly Ser Gly Ala Gln Ala Cys Glu Ser Asn Leu Gln
       210                 215                 220
   ggt gga aat ggt gga gga ggt gtt gct ttc tac aat ctt gga atg aat      781
   Gly Gly Asn Gly Gly Gly Gly Val Ala Phe Tyr Asn Leu Gly Met Asn
   225                 230                 235                 240
   atg aac aat ttt caa ttc tcc ggg gga gat att tac ggt atg agc ggc      829
   Met Asn Asn Phe Gln Phe Ser Gly Gly Asp Ile Tyr Gly Met Ser Gly
                   245                 250                 255
   ggt agc gga gga ggt ggt ggc ggt gcg act aga ccc gcg ttt tag          874
   Gly Ser Gly Gly Gly Gly Gly Gly Ala Thr Arg Pro Ala Phe
               260                 265                 270
   agttttagcg ttttggtgac accttttgtt gcgtttgcgt gtttgacctc aaactactag    934
   gctactagct atagcggttg cgaaatgcga atattaggtt                          974
<210> 2
   <211> 270
   <212> PRT
   <213> Arabidopsis thaliana
<400> 2
   Met Glu Leu Asn Arg Ser Glu Ala Asp Glu Ala Lys Ala Glu Thr Thr
   1               5                   10                  15
   Pro Thr Gly Gly Ala Thr Ser Ser Ala Thr Ala Ser Gly Ser Ser Ser
   20 25 30
   Gly Arg Arg Pro Arg Gly Arg Pro Ala Gly Ser Lys Asn Lys Pro Lys
   35 40 45
   Pro Pro Thr Ile Ile Thr Arg Asp Ser Pro Asn Val Leu Arg Ser His
   50 55 60
   Val Leu Glu Val Thr Ser Gly Ser Asp Ile Ser Glu Ala Val Ser Thr
   65 70 75 80
   Tyr Ala Thr Arg Arg Gly Cys Gly Val Cys Ile Ile Ser Gly Thr Gly
   85 90 95
   Ala Val Thr Asn Val Thr Ile Arg Gln Pro Ala Ala Pro Ala Gly Gly
   100 105 110
   Gly Val Ile Thr Leu His Gly Arg Phe Asp Ile Leu Ser Leu Thr Gly
   115 120 125
   Thr Ala Leu Pro Pro Pro Ala Pro Pro Gly Ala Gly Gly Leu Thr Val
   130 135 140
   Tyr Leu Ala Gly Gly Gln Gly Gln Val Val Gly Gly Asn Val Ala Gly
   145 150 155 160
   Ser Leu Ile Ala Ser Gly Pro Val Val Leu Met Ala Ala Ser Phe Ala
   165 170 175
   Asn Ala Val Tyr Asp Arg Leu Pro Ile Glu Glu Glu Glu Thr Pro Pro
   180 185 190
   Pro Arg Thr Thr Gly Val Gln Gln Gln Gln Pro Glu Ala Ser Gln Ser
   195 200 205
   Ser Glu Val Thr Gly Ser Gly Ala Gln Ala Cys Glu Ser Asn Leu Gln
   210 215 220
   Gly Gly Asn Gly Gly Gly Gly Val Ala Phe Tyr Asn Leu Gly Met Asn
   225 230 235 240
   Met Asn Asn Phe Gln Phe Ser Gly Gly Asp Ile Tyr Gly Met Ser Gly
   245 250 255
   Gly Ser Gly Gly Gly Gly Gly Gly Ala Thr Arg Pro Ala Phe
               260                 265                 270
<210> 3
   <211> 1040
   <212> DNA
   <213> Arabidopsis thaliana
<220>
   <221> CDS
   <222> (82)..(879)
   <223> G2789
<400> 3
   ctttagggac accaaatcta ttcaacctaa aagccttctt ttcccctata ttgaccaact      60
   ttttagcgaa tcagaagagg a atg gat gag gta tct cgt tct cat aca ccg       111
                           Met Asp Glu Val Ser Arg Ser His Thr Pro
                           1               5                   10
   caa ttt cta tca agt gat cat cag cac tat cac cat caa aac gct gga       159
   Gln Phe Leu Ser Ser Asp His Gln His Tyr His His Gln Asn Ala Gly
                   15                  20                  25
   cga caa aaa cgc ggc aga gaa gaa gaa gga gtt gaa ccc aac aat ata       207
   Arg Gln Lys Arg Gly Arg Glu Glu Glu Gly Val Glu Pro Asn Asn Ile
               30                  35                  40
   ggg gaa gac cta gcc acc ttt cct tcc gga gaa gag aat atc aag aag       255
   Gly Glu Asp Leu Ala Thr Phe Pro Ser Gly Glu Glu Asn Ile Lys Lys
           45                  50                  55
   aga agg cca cgt ggc aga cct gct ggt tcc aag aac aaa ccc aaa gca       303
   Arg Arg Pro Arg Gly Arg Pro Ala Gly Ser Lys Asn Lys Pro Lys Ala
       60                  65                  70
   cca atc ata gtc act cgc gac tcc gcg aac gcc ttc aga tgt cac gtc       351
   Pro Ile Ile Val Thr Arg Asp Ser Ala Asn Ala Phe Arg Cys His Val
   75                  80                  85                  90
   atg gag ata acc aac gcc tgc gat gta atg gaa agc cta gcc gtc ttc       399
   Met Glu Ile Thr Asn Ala Cys Asp Val Met Glu Ser Leu Ala Val Phe
                   95                  100                 105
   gct aga cgc cgt cag cgt ggc gtt tgc gtc ttg acc gga aac ggg gcc       447
   Ala Arg Arg Arg Gln Arg Gly Val Cys Val Leu Thr Gly Asn Gly Ala
               110                 115                 120
   gtt aca aac gtc acc gtt aga caa cct ggc gga ggc gtc gtc agt tta       495
   Val Thr Asn Val Thr Val Arg Gln Pro Gly Gly Gly Val Val Ser Leu
           125                 130                 135
   cac gga cgg ttt gag att ctt tct ctc tcg ggt tcg ttt ctt cct cca       543
   His Gly Arg Phe Glu Ile Leu Ser Leu Ser Gly Ser Phe Leu Pro Pro
       140                 145                 150
   ccg gca cca cca gct gcg tct ggt tta aag gtt tac tta gcc ggt ggt       591
   Pro Ala Pro Pro Ala Ala Ser Gly Leu Lys Val Tyr Leu Ala Gly Gly
   155                 160                 165                 170
   caa ggt caa gtg atc gga ggc agt gtg gtg gga ccg ctt acg gca tca      639
   Gln Gly Gln Val Ile Gly Gly Ser Val Val Gly Pro Leu Thr Ala Ser
                   175                 180                 185
   agt ccg gtg gtc gtt atg gca gct tca ttt gga aac gca tct tac gag      687
   Ser Pro Val Val Val Met Ala Ala Ser Phe Gly Asn Ala Ser Tyr Glu
               190                 195                 200
   agg ctg cca cta gag gag gag gag gaa act gaa aga gaa ata gat gga      735
   Arg Leu Pro Leu Glu Glu Glu Glu Glu Thr Glu Arg Glu Ile Asp Gly
           205                 210                 215
   aac gcg gct agg gcg att gga acg caa acg cag aaa cag tta atg caa      783
   Asn Ala Ala Arg Ala Ile Gly Thr Gln Thr Gln Lys Gln Leu Met Gln
       220                 225                 230
   gat gcg aca tcg ttt att ggg tcg ccg tcg aat tta att aac tct gtt      831
   Asp Ala Thr Ser Phe Ile Gly Ser Pro Ser Asn Leu Ile Asn Ser Val
   235                 240                 245                 250
   tcg ttg cca ggt gaa gct tat tgg gga acg caa cga ccg tct ttc taa      879
   Ser Leu Pro Gly Glu Ala Tyr Trp Gly Thr Gln Arg Pro Ser Phe
                   255                 260                 265
   gataatatca ttgataatat aagtttcgtc ttcttattct ttttcacttt ttaccttttt    939
   cactttctta ggttttgttt taacgtttga ttaatacctg aaggtttttg gaaaattttc    999
   gatcggataa aaggatttat gttgcgagcc gaaacgcggc c                       1040
<210> 4
   <211> 265
   <212> PRT
   <213> Arabidopsis thaliana
<400> 4
   Met Asp Glu Val Ser Arg Ser His Thr Pro Gln Phe Leu Ser Ser Asp
   1               5                   10                  15
   His Gln His Tyr His His Gln Asn Ala Gly Arg Gln Lys Arg Gly Arg
               20                  25                  30
   Glu Glu Glu Gly Val Glu Pro Asn Asn Ile Gly Glu Asp Leu Ala Thr
           35                  40                  45
   Phe Pro Ser Gly Glu Glu Asn Ile Lys Lys Arg Arg Pro Arg Gly Arg
       50                  55                  60
   Pro Ala Gly Ser Lys Asn Lys Pro Lys Ala Pro Ile Ile Val Thr Arg
   65                  70                  75                  80
   Asp Ser Ala Asn Ala Phe Arg Cys His Val Met Glu Ile Thr Asn Ala
                   85                  90                  95
   Cys Asp Val Met Glu Ser Leu Ala Val Phe Ala Arg Arg Arg Gln Arg
               100                 105                 110
   Gly Val Cys Val Leu Thr Gly Asn Gly Ala Val Thr Asn Val Thr Val
           115                 120                 125
   Arg Gln Pro Gly Gly Gly Val Val Ser Leu His Gly Arg Phe Glu Ile
       130                 135                 140
   Leu Ser Leu Ser Gly Ser Phe Leu Pro Pro Pro Ala Pro Pro Ala Ala
   145                 150                 155                 160
   Ser Gly Leu Lys Val Tyr Leu Ala Gly Gly Gln Gly Gln Val Ile Gly
                   165                 170                 175
   Gly Ser Val Val Gly Pro Leu Thr Ala Ser Ser Pro Val Val Val Met
               180                 185                 190
   Ala Ala Ser Phe Gly Asn Ala Ser Tyr Glu Arg Leu Pro Leu Glu Glu
           195                 200                 205
   Glu Glu Glu Thr Glu Arg Glu Ile Asp Gly Asn Ala Ala Arg Ala Ile
       210                 215                 220
   Gly Thr Gln Thr Gln Lys Gln Leu Met Gln Asp Ala Thr Ser Phe Ile
   225                 230                 235                 240
   Gly Ser Pro Ser Asn Leu Ile Asn Ser Val Ser Leu Pro Gly Glu Ala
                   245                 250                 255
   Tyr Trp Gly Thr Gln Arg Pro Ser Phe
               260                 265
<210> 5
   <211> 1130
   <212> DNA
   <213> Arabidopsis thaliana
<220>
   <221> CDS
   <222> (189)..(1019)
   <223> G1945
<400> 5
   atttcccaaa gggatttacg aaaagtccct ctcctctatc atctctttat tcaccccata     60
   ccaacaacct ctacatcttc ttcttcttct tcctcctctt ttattttctt tttaaatcat    120
   ttacacaaaa atccaaagac aaatctgaaa tctctaataa acaaatccat aaaataagaa    180
   aaacaaag atg aaa ggt gaa tac aga gag caa aag agt aac gaa atg ttt     230
            Met Lys Gly Glu Tyr Arg Glu Gln Lys Ser Asn Glu Met Phe
            1               5                   10
   tcc aag ctt cct cat cat caa caa caa cag caa caa caa caa caa caa      278
   Ser Lys Leu Pro His His Gln Gln Gln Gln Gln Gln Gln Gln Gln Gln
   15                  20                  25                  30
   cac tct ctt acc tct cac ttc cac ctc tcc tcc acc gta acc ccc acc      326
   His Ser Leu Thr Ser His Phe His Leu Ser Ser Thr Val Thr Pro Thr
                   35                  40                  45
   gtc gat gac tcc tcc atc gaa gtg gtc cga cgt cca cgt ggc aga cca      374
   Val Asp Asp Ser Ser Ile Glu Val Val Arg Arg Pro Arg Gly Arg Pro
               50                  55                  60
   cca ggt tcc aaa aac aaa cct aaa cca ccc gtc ttc gtc aca cgt gac      422
   Pro Gly Ser Lys Asn Lys Pro Lys Pro Pro Val Phe Val Thr Arg Asp
           65                  70                  75
   acc gac cct cct atg agt cct tac atc ctc gaa gtt cct tca gga aac      470
   Thr Asp Pro Pro Met Ser Pro Tyr Ile Leu Glu Val Pro Ser Gly Asn
       80                  85                  90
   gac gtc gtc gaa gcc atc aac cgt ttc tgc cgc cgt aaa tcc atc gga      518
   Asp Val Val Glu Ala Ile Asn Arg Phe Cys Arg Arg Lys Ser Ile Gly
   95                  100                 105                 110
   gtc tgc gtc ctt agt ggc tct ggc tct gta gct aac gtc act tta cgt      566
   Val Cys Val Leu Ser Gly Ser Gly Ser Val Ala Asn Val Thr Leu Arg
                   115                 120                 125
   cag cca tca ccg gca gct ctt ggc tct acc ata act ttc cat gga aag      614
   Gln Pro Ser Pro Ala Ala Leu Gly Ser Thr Ile Thr Phe His Gly Lys
               130                 135                 140
   ttt gat ctc ctc tcc gtc tcc gca acg ttt ctc cct cct ccg cct cgt      662
   Phe Asp Leu Leu Ser Val Ser Ala Thr Phe Leu Pro Pro Pro Pro Arg
           145                 150                 155
   act tcc ttg tct cct ccc gtt tct aac ttc ttc acc gtc tct ctc gct      710
   Thr Ser Leu Ser Pro Pro Val Ser Asn Phe Phe Thr Val Ser Leu Ala
       160                 165                 170
   gga cct caa gga caa atc atc gga ggg ttc gtc gct ggt cca ctt att      758
   Gly Pro Gln Gly Gln Ile Ile Gly Gly Phe Val Ala Gly Pro Leu Ile
   175                 180                 185                 190
   tcg gca gga aca gtt tac gtc atc gcc gca agt ttc aac aac cct tct      806
   Ser Ala Gly Thr Val Tyr Val Ile Ala Ala Ser Phe Asn Asn Pro Ser
                   195                 200                 205
   tat cac cgg tta ccg gcg gaa gaa gag caa aaa cac tcg gcg ggg aca      854
   Tyr His Arg Leu Pro Ala Glu Glu Glu Gln Lys His Ser Ala Gly Thr
               210                 215                 220
   ggg gaa aga gag gga caa tct ccg ccg gtc tct ggt ggc ggt gaa gag      902
   Gly Glu Arg Glu Gly Gln Ser Pro Pro Val Ser Gly Gly Gly Glu Glu
           225                 230                 235
   tca gga cag atg gcg gga agt gga gga gag tcg tgt ggg gta tca atg      950
   Ser Gly Gln Met Ala Gly Ser Gly Gly Glu Ser Cys Gly Val Ser Met
       240                 245                 250
   tac agt tgc cac atg ggt ggc tct gat gtt att tgg gcc cct aca gcc      998
   Tyr Ser Cys His Met Gly Gly Ser Asp Val Ile Trp Ala Pro Thr Ala
   255                 260                 265                 270
   aga gct cca ccg cca tac taa ccaatccttc tttcacaaat ctctttcttt        1049
   Arg Ala Pro Pro Pro Tyr
                   275
   ctttttttgt ttttttttgt tttgggttag gatgaatcaa gaaactaggg tttttttttt  1109
   ttttttttaa aaaaaaaaaa a                                              1130
<210> 6
   <211> 276
   <212> PRT
   <213> Arabidopsis thaliana
<400> 6
   Met Lys Gly Glu Tyr Arg Glu Gln Lys Ser Asn Glu Met Phe Ser Lys
   1               5                   10                  15
   Leu Pro His His Gln Gln Gln Gln Gln Gln Gln Gln Gln Gln His Ser
               20                  25                  30
   Leu Thr Ser His Phe His Leu Ser Ser Thr Val Thr Pro Thr Val Asp
           35                  40                  45
   Asp Ser Ser Ile Glu Val Val Arg Arg Pro Arg Gly Arg Pro Pro Gly
       50                  55                  60
   Ser Lys Asn Lys Pro Lys Pro Pro Val Phe Val Thr Arg Asp Thr Asp
   65                  70                  75                  80
   Pro Pro Met Ser Pro Tyr Ile Leu Glu Val Pro Ser Gly Asn Asp Val
                   85                  90                  95
   Val Glu Ala Ile Asn Arg Phe Cys Arg Arg Lys Ser Ile Gly Val Cys
               100                 105                 110
   Val Leu Ser Gly Ser Gly Ser Val Ala Asn Val Thr Leu Arg Gln Pro
           115                 120                 125
   Ser Pro Ala Ala Leu Gly Ser Thr Ile Thr Phe His Gly Lys Phe Asp
       130                 135                 140
   Leu Leu Ser Val Ser Ala Thr Phe Leu Pro Pro Pro Pro Arg Thr Ser
   145                 150                 155                 160
   Leu Ser Pro Pro Val Ser Asn Phe Phe Thr Val Ser Leu Ala Gly Pro
                   165                 170                 175
   Gln Gly Gln Ile Ile Gly Gly Phe Val Ala Gly Pro Leu Ile Ser Ala
               180                 185                 190
   Gly Thr Val Tyr Val Ile Ala Ala Ser Phe Asn Asn Pro Ser Tyr His
           195                 200                 205
   Arg Leu Pro Ala Glu Glu Glu Gln Lys His Ser Ala Gly Thr Gly Glu
       210                 215                 220
   Arg Glu Gly Gln Ser Pro Pro Val Ser Gly Gly Gly Glu Glu Ser Gly
   225                 230                 235                 240
   Gln Met Ala Gly Ser Gly Gly Glu Ser Cys Gly Val Ser Met Tyr Ser
                   245                 250                 255
   Cys His Met Gly Gly Ser Asp Val Ile Trp Ala Pro Thr Ala Arg Ala
               260                 265                 270
   Pro Pro Pro Tyr
           275
<210> 7
   <211> 1050
   <212> DNA
   <213> Arabidopsis thaliana
<220>
   <221> CDS
   <222> (63)..(740)
   <223> G2155
<400> 7
   ctcatatata ccaaccaaac ctctctctgc atctttatta acacaaaatt ccaaaagatt      60
   aa atg ttg tcg aag ctc cct aca cag cga cac ttg cac ctc tct ccc        107
       Met Leu Ser Lys Leu Pro Thr Gln Arg His Leu His Leu Ser Pro
       1               5                   10                  15
   tcc tct ccc tcc atg gaa acc gtc ggg cgt cca cgt ggc aga cct cga      155
   Ser Ser Pro Ser Met Glu Thr Val Gly Arg Pro Arg Gly Arg Pro Arg
                   20                  25                  30
   ggt tcc aaa aac aaa cct aaa gct cca atc ttt gtc acc att gac cct      203
   Gly Ser Lys Asn Lys Pro Lys Ala Pro Ile Phe Val Thr Ile Asp Pro
               35                  40                  45
   cct atg agt cct tac atc ctc gaa gtg cca tcc gga aac gat gtc gtt      251
   Pro Met Ser Pro Tyr Ile Leu Glu Val Pro Ser Gly Asn Asp Val Val
           50                  55                  60
   gaa gcc cta aac cgt ttc tgc cgc ggt aaa gcc atc ggc ttt tgc gtc      299
   Glu Ala Leu Asn Arg Phe Cys Arg Gly Lys Ala Ile Gly Phe Cys Val
       65                  70                  75
   ctc agt ggc tca ggc tcc gtt gct gat gtc act ttg cgt cag cct tct      347
   Leu Ser Gly Ser Gly Ser Val Ala Asp Val Thr Leu Arg Gln Pro Ser
   80                  85                  90                  95
   ccg gca gct cct ggc tca acc att act ttc cac gga aag ttc gat ctt      395
   Pro Ala Ala Pro Gly Ser Thr Ile Thr Phe His Gly Lys Phe Asp Leu
                   100                 105                 110
   ctc tct gtc tcc gcc act ttc ctc cct cct cta cct cct acc tcc ttg      443
   Leu Ser Val Ser Ala Thr Phe Leu Pro Pro Leu Pro Pro Thr Ser Leu
               115                 120                 125
   tcc cct ccc gtc tcc aat ttc ttc acc gtc tct ctc gcc gga cct cag      491
   Ser Pro Pro Val Ser Asn Phe Phe Thr Val Ser Leu Ala Gly Pro Gln
           130                 135                 140
   ggg aaa gtc atc ggt gga ttc gtc gct ggt cct ctc gtt gcc gcc gga      539
   Gly Lys Val Ile Gly Gly Phe Val Ala Gly Pro Leu Val Ala Ala Gly
       145                 150                 155
   act gtt tac ttc gtc gcc act agt ttc aag aac cct tcc tat cac cgg      587
   Thr Val Tyr Phe Val Ala Thr Ser Phe Lys Asn Pro Ser Tyr His Arg
   160                 165                 170                 175
   tta cct gct acg gag gaa gag caa aga aac tcg gcg gaa ggg gaa gag      635
   Leu Pro Ala Thr Glu Glu Glu Gln Arg Asn Ser Ala Glu Gly Glu Glu
                   180                 185                 190
   gag gga caa tcg ccg ccg gtc tct gga ggt ggt gga gag tcg atg tac      683
   Glu Gly Gln Ser Pro Pro Val Ser Gly Gly Gly Gly Glu Ser Met Tyr
               195                 200                 205
   gtg ggt ggc tct gat gtc att tgg gat ccc aac gcc aaa gct cca tcg      731
   Val Gly Gly Ser Asp Val Ile Trp Asp Pro Asn Ala Lys Ala Pro Ser
           210                 215                 220
   ccg tac tga ccacaaatcc atctcgttca aactagggtt tcttcttctt              780
   Pro Tyr
       225
   tagatcatca agaatcaaca aaaagattgc atttttagat tctttgtaat atcataattg    840
   actcactctt taatctctct atcacttctt ctttagcttt ttctgcagtg tcaaacttca    900
   catatttgta gtttgatttg actatcccca agttttgtat tttatcatac aaatttttgc    960
   ctgtctctaa tggttgtttt ttcgtttgta taatcttatg cattgtttat tggagctcca   1020
   gagattgaat gtataatata atggtttaat                                    1050
<210> 8
   <211> 225
   <212> PRT
   <213> Arabidopsis thaliana
<400> 8
   Met Leu Ser Lys Leu Pro Thr Gln Arg His Leu His Leu Ser Pro Ser
   1               5                   10                  15
   Ser Pro Ser Met Glu Thr Val Gly Arg Pro Arg Gly Arg Pro Arg Gly
               20                  25                  30
   Ser Lys Asn Lys Pro Lys Ala Pro Ile Phe Val Thr Ile Asp Pro Pro
           35                  40                  45
   Met Ser Pro Tyr Ile Leu Glu Val Pro Ser Gly Asn Asp Val Val Glu
       50                  55                  60
   Ala Leu Asn Arg Phe Cys Arg Gly Lys Ala Ile Gly Phe Cys Val Leu
   65                  70                  75                  80
   Ser Gly Ser Gly Ser Val Ala Asp Val Thr Leu Arg Gln Pro Ser Pro
                   85                  90                  95
   Ala Ala Pro Gly Ser Thr Ile Thr Phe His Gly Lys Phe Asp Leu Leu
               100                 105                 110
   Ser Val Ser Ala Thr Phe Leu Pro Pro Leu Pro Pro Thr Ser Leu Ser
           115                 120                 125
   Pro Pro Val Ser Asn Phe Phe Thr Val Ser Leu Ala Gly Pro Gln Gly
       130                 135                 140
   Lys Val Ile Gly Gly Phe Val Ala Gly Pro Leu Val Ala Ala Gly Thr
   145                 150                 155                 160
   Val Tyr Phe Val Ala Thr Ser Phe Lys Asn Pro Ser Tyr His Arg Leu
                   165                 170                 175
   Pro Ala Thr Glu Glu Glu Gln Arg Asn Ser Ala Glu Gly Glu Glu Glu
               180                 185                 190
   Gly Gln Ser Pro Pro Val Ser Gly Gly Gly Gly Glu Ser Met Tyr Val
           195                 200                 205
   Gly Gly Ser Asp Val Ile Trp Asp Pro Asn Ala Lys Ala Pro Ser Pro
       210                 215                 220
   Tyr
   225